(19) Europäisches Patentamt
European Patent Office
Office européen des brevets

(11) EP 2 125 811 B1

(12) **FASCICULE DE BREVET EUROPEEN**

(45) Date de publication et mention de la délivrance du brevet:
**29.05.2013 Bulletin 2013/22**

(21) Numéro de dépôt: **07872409.3**

(22) Date de dépôt: **20.12.2007**

(51) Int Cl.:
***C07D 471/04** (2006.01)*

(86) Numéro de dépôt international:
**PCT/FR2007/002122**

(87) Numéro de publication internationale:
**WO 2008/093024 (07.08.2008 Gazette 2008/32)**

(54) **DERIVES DE N-(AMINO-HETEROARYL)-1H-PYRROLOPYRIDINE-2-CARBOXAMIDES, LEUR PRÉPARATION ET LEUR APPLICATION EN THERAPEUTIQUE**

N-(AMINO-HETEROARYL)-1H-PYRROLOPYRIDIN-2-CARBOXAMIDDERIVATE, IHRE HERSTELLUNG UND THERAPEUTISCHE ANWENDUNG

N-(AMINO-HETEROARYL)-1H-PYRROLOPYRIDINE-2-CARBOXAMIDES DERIVATIVES, PREPARATION THEREOF AND THEIR USE IN THERAPY

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **26.12.2006 FR 0611353**

(43) Date de publication de la demande:
**02.12.2009 Bulletin 2009/49**

(73) Titulaire: **SANOFI**
**75008 Paris (FR)**

(72) Inventeurs:
- **DUBOIS, Laurent**
  **F-75013 Paris (FR)**
- **EVANNO, Yannick**
  **F-75013 Paris (FR)**
- **GILLE, Catherine**
  **F-75013 Paris (FR)**
- **MACHNIK, David**
  **F-75013 Paris (FR)**
- **MALANDA, André**
  **F-75013 Paris (FR)**

(74) Mandataire: **Le Coupanec, Pascale A.M.P.**
**Cabinet Nony**
**3, rue de Penthièvre**
**75008 Paris (FR)**

(56) Documents cités:
**EP-A1- 1 535 922** **WO-A-03/028719**
**WO-A-2004/056768** **US-A1- 2006 040 964**
**US-B1- 6 630 496**

- **BRANDS ET AL: "Novel, selective indole-based ECE inhibitors: Lead optimization via solid-phase and classical synthesis" BIOORGANIC & MEDICINAL CHEMISTRY LETTERS, OXFORD, GB, vol. 15, no. 19, 1 octobre 2005 (2005-10-01), pages 4201-4205, XP005038874 ISSN: 0960-894X**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention a pour objet des composés dérivés de *N*-(amino-hétéroaryl)-1*H*-pyrrolopyridine-2-carboxamides, qui présentent une activité antagoniste *in vitro* et *in vivo* pour les récepteurs de type TRPV1 (ou VR1).

Un premier objet de l'invention concerne des composés répondant à la formule générale (I) et définie en détail ci-apres.

Un autre objet de l'invention concerne des procédés de préparation de ces composés de formule générale (I).

Un autre objet de l'invention concerne l'utilisation de ces composés de formule générale (I) notamment dans des médicaments ou dans des compositions pharmaceutiques.

La présente invention décrit des composés réspondant à la formule générale (I) :

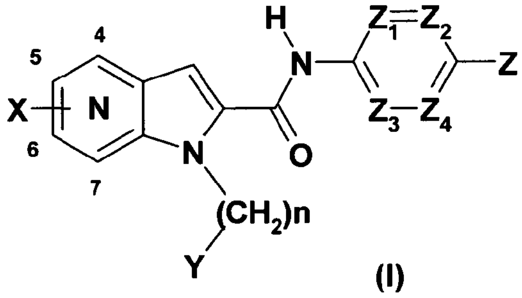

(I)

dans laquelle
le noyau pyrrolopyridine est un groupe pyrrolo[3,2-*b*]pyridine, un groupe pyrrolo[3,2-c]pyridine, un groupe pyrrolo[2,3-*c*]pyridine ou un groupe pyrrolo[2,3-*b*]pyridine ;
le noyau pyrrolopyridine étant éventuellement substitué en position carbonée 4, 5, 6 et / ou 7 par un ou plusieurs substituents X, identiques ou différents l'un de l'autre, choisis parmi un atome d'hydrogène ou d'halogène ou un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, cyano, $C_1$-$C_6$-thioalkyle, -S(O)-$C_1$-$C_6$-alkyle, -S(O)$_2$-$C_1$-$C_6$-alkyle, $SF_5$, C(O)$NR_1R_2$, $SO_2NR_1R_2$, nitro, $NR_1R_2$, OCO$NR_1R_2$, $NR_3COR_4$, $NR_3CONR_1R_2$, $NR_3SO_2R_5$, $NR_3SO_2NR_1R_2$, aryl-$C_1$-$C_6$-alkylène, hétéroaryl-$C_1$-$C_6$-alkylène, aryle ou hétéroaryle, l'aryle et l'hétéroaryle étant éventuellement substitués par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_1$-$C_6$-fluoroalcoxyle, nitro ou cyano ;
n est égal à 0, 1, 2 ou 3 ;
Y représente un aryle ou un hétéroaryle,
l'aryle ou l'hétéroaryle étant éventuellement substitué par un ou plusieurs groupes choisis parmi un atome d'halogène ou un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, hydroxyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, cyano, $C_1$-$C_6$-thioalkyle, thiol, -S(O)-$C_1$-$C_6$-alkyle, -S(O)$_2$-$C_1$-$C_6$-alkyle, C(O)$NR_1R_2$, $SO_2NR_1R_2$, $SF_5$, nitro, OCO$NR_1R_2$, $NR_3COR_4$, $NR_3CONR_1R_2$, $NO_1R_2$, $NR_3SO_2NR_1R_2$, $NR_3COR_4$, $NR_3SO_2R_5$, aryl-$C_1$-$C_6$-alkylène ou aryle, l'aryle et l'aryl-$C_1$-$C_6$-alkylène étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_1$-$C_6$-fluoroalcoxyle, nitro ou cyano ;
$Z_1$, $Z_2$, $Z_3$, $Z_4$ représentent, indépendamment l'un de l'autre, un atome d'azote ou un groupe C($R_6$), l'un au moins correspondant à un atome d'azote et l'un au moins correspondant à un groupe C($R_6$) ; l'atome d'azote ou un des atomes d'azote présent dans le cycle, défini comme azote de position 1, étant éventuellement substitué par $R_7$ lorsque l'atome de carbone en position 2 ou 4 par rapport à cet azote de référence est substitué par un groupe oxo ou thio ;
Z représente
soit une amine cyclique reliée par l'atome d'azote, de formule :

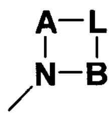

dans laquelle

A représente un groupe $C_1$-$C_7$-alkylène éventuellement substitué par un ou deux groupes $R_8$ ;

B représente un groupe $C_1$-$C_7$-alkylène éventuellement substitué par un ou deux groupes $R_9$ ;
L représente une liaison, un atome de soufre, d'oxygène ou d'azote ; l'atome d'azote étant éventuellement substitué par un groupe $R_{10}$ ou $R_{11}$ ;

les atomes de carbone de l'amine cyclique Z étant éventuellement substitués par un ou plusieurs groupes $R_{12}$ identiques ou différents l'un de l'autre ;
soit une amine acylique, reliée par l'atome d'azote, de formule NRaRb dans laquelle Ra et Rb représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, hydroxyle, $C_1$-$C_6$-alcoxyle, $C_3$-$C_7$-cycloalkyle-$C_1$-$C_6$-alkylène-O-, $C_1$-$C_6$-fluoroalcoxyle, $C_1$-$C_6$-alkyle-C(O)-, HO-C(O)-$C_1$-$C_6$-alkylène, $C_1$-$C_6$-alkyle-O-C(O)-$C_1$-$C_6$-alkylène, aryle ou hétéroaryle, Ra et Rb pouvant être éventuellement substitués par un ou plusieurs groupes Rc identiques ou différents l'un de l'autre ;
Rc représente un atome d'halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_3$-$C_7$-cycloalkyle-$C_1$-$C_6$-alkylène-O-, $C_1$-$C_6$-fluoroalcoxyle, $C_1$-$C_6$-thioalkyle, -S(O)-$C_1$-$C_6$-alkyle, -$S(O)_2$-$C_1$-$C_6$-alkyle, cyano, $C(O)NR_1R_2$, $NR_1R_2$, $SO_2NR_1R_2$, $NR_3COR_4$, $NR_3SO_2R_5$, $OC(O)NR_1R_2$, $NR_3COOR_5$, $NR_3CONR_1R_2$, $NR_3SO_2NR_1R_2$, hydroxyle, thiol, oxo, thio, aryl-$C_1$-$C_6$-alkylène, aryle ou hétéroaryle, l'aryle et l'hétéroaryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_1$-$C_6$-fluoroalcoxyle, nitro ou cyano ;
$R_1$ et $R_2$ représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_6$-alkylène, aryl-$C_1$-$C_6$-alkylène, aryle ou hétéroaryle, l'aryle et l'hétéroaryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_1$-$C_6$-fluoroalcoxyle, nitro ou cyano ; ou $R_1$ et $R_2$ forment ensemble, avec l'atome d'azote qui les porte, un groupe azétidinyle, pyrrolidinyle, pipéridinyle, azépinyle, morpholinyle, thiomorpholinyle, pipérazinyle, homopipérazinyle, ce groupe étant éventuellement substitué par un groupe $C_1$-$C_6$-alkyle, $C_3$-$C_7$-cycloalkyle, $C_3$-$C_7$-cycloalkyle-$C_1$-$C_6$-alkylène, aryl-$C_1$-$C_6$-alkylène, aryle ou hétéroaryle, l'aryle et l'hétéroaryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_1$-$C_6$-fluoroalcoxyle, nitro ou cyano ;
$R_3$ et $R_4$ représentent, indépendamment l'un de l'autre,
un atome d'hydrogène ou un groupe $C_1$-$C_6$-alkyle, $C_3$-$C_7$-cycloalkyle, $C_3$-$C_7$-cycloalkyle-$C_1$-$C_6$-alkylène, aryl-$C_1$-$C_6$-alkylène, aryle ou hétéroaryle, l'aryle et l'hétéroaryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_1$-$C_6$-fluoroalcoxyle, nitro ou cyano ;
ou $R_3$ et $R_4$ forment ensemble un groupe ($C_2$-$C_5$)alkylène ;
ou $R_1$ et $R_3$ forment ensemble un groupe ($C_2$-$C_5$)alkylène ;
$R_5$ représente un groupe $C_1$-$C_6$-alkyle, $C_3$-$C_7$-cycloalkyle, $C_3$-$C_7$-cycloalkyle-$C_1$-$C_6$-alkylène, aryl-$C_1$-$C_6$-alkylène, aryle ou hétéroaryle, l'aryle et l'hétéroaryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_1$-$C_6$-fluoroalcoxyle, nitro ou cyano ;
ou $R_3$ et $R_5$ forment ensemble un groupe ($C_2$-$C_5$)alkylène ;
$R_6$ représente un atome d'hydrogène ou d'halogène ou un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, $C_1$-$C_6$-thioalkyle,- S(O)-$C_1$-$C_6$-alkyle, -$S(O)_2$-$C_1$-$C_6$-alkyle, hydroxyle, thiol, oxo, thio, aryle, aryl-$C_1$-$C_6$-alkylène, hétéroaryle, l'aryle et l'hétéroaryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_1$-$C_6$-fluoroalcoxyle, nitro ou cyano ;
$R_7$ représente un atome d'hydrogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, aryle, aryl-$C_1$-$C_6$-alkylène ou hétéroaryle, l'aryle et l'hétéroaryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_1$-$C_6$-fluoroalcoxyle, nitro ou cyano ;
$R_8$, $R_9$ et $R_{10}$ sont définis tels que :
deux groupes $R_8$ peuvent former ensemble une liaison ou un groupe $C_1$-$C_6$-alkylène ;
deux groupes $R_9$ peuvent former ensemble une liaison ou un groupe $C_1$-$C_6$-alkylène ;
$R_8$ et $R_9$ peuvent former ensemble une liaison ou un groupe $C_1$-$C_6$-alkylène ;
$R_8$ et $R_{10}$ peuvent former ensemble une liaison ou un groupe $C_1$-$C_6$-alkylène ;
$R_9$ et $R_{10}$ peuvent former ensemble une liaison ou un groupe $C_1$-$C_6$-alkylène ;
$R_{11}$ représente un atome d'hydrogène, un groupe $C_1$-$C_6$-alkyle, $C_3$-$C_7$-cycloalkyle, $C_3$-$C_7$-cycloalkyle-$C_1$-$C_6$-alkylène,

$C_1$-$C_6$-fluoroalkyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, hydroxyle, $C_1$-$C_6$-alkyl-CO-, $COOR_5$, C(O)$NR_1R_2$, aryl-$C_1$-$C_6$-alkylène, aryle ou hétéroaryle, l'aryle et l'hétéroaryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_3$-$C_7$-cycloalcoxyle, $C_1$-$C_6$-fluoroalcoxyle, nitro ou cyano ;

$R_{12}$ représente un atome de fluor, un groupe $C_1$-$C_6$-alkyle éventuellement substitué par un groupe $R_{13}$, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-cycloalk-1,1-diyle, $C_3$-$C_7$-hétérocycloalk-1,1-diyle éventuellement substitué sur un atome d'azote par un groupe $R_{11}$ , $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, $CO_2H$, C(O)O-$C_1$-$C_6$-alkyle, C(O)$NR_1R_2$, $NR_1R_2$, $NR_3COR_4$, OC(O)$NR_1R_2$, $NR_3COOR_5$, $NR_3CONR_1R_2$, hydroxyle, thiol, oxo, thio, aryl-$C_1$-$C_6$-alkylène, aryle, l'aryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_3$-$C_7$-cycloalcoxyle, $C_1$-$C_6$-fluoroalcoxyle, nitro ou cyano ;

$R_{13}$ représente un groupe $C_1$-$C_6$-alcoxyle, $C_3$-$C_7$-cycloalcoxyle, $C_3$-$C_7$-cycloalkyle-$C_1$-$C_6$-alkylène-O-, C(O)$NR_1R_2$, $NR_1R_2$, $NR_3COR_4$, OC(O)$NR_1R_2$, $NR_3COOR_5$, hydroxyle.

Dans les composés de formule générale (I), le ou les atomes d'azote peuvent être sous forme oxydée (N-oxyde).

Parmi les composés de formule générale (I) décrits dans l'invention, un premier sous-groupe de composés est constitué par les composés pour lesquels le noyau pyrrolopyridine est un groupe pyrrolo[3,2-*b*]pyridine, un groupe pyrrolo [2,3-*c*]pyridine ou un groupe pyrrolo[2,3-*b*]pyridine.

Parmi les composés de formule générale (I) décrits dans l'invention, un second sous-groupe de composés est constitué par les composés pour lesquels, si n est égal à 0, alors le ou les substituents X, identiques ou différents l'un de l'autre, sont choisis parmi un atome d'hydrogène ou d'halogène ou un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, cyano, $C_1$-$C_6$-thioalkyle, -S(O)-$C_1$-$C_6$-alkyle, -$S(O)_2$-$C_1$-$C_6$-alkyle, $SF_5$, C(O) $NR_1R_2$, $SO_2NR_1R_2$, nitro, $NR_1R_2$, $OCONR_1R_2$, $NR_3COR_4$, $NR_3CONR_1R_2$, $NR_3SO_2R_5$, $NR_3SO_2NR_1R_2$.

Parmi les composés de formule générale (I) décrits dans l'invention, un troisième sous-groupe de composés est constitué par les composés pour lesquels quand n = 0 ou 1 alors Y est différent d'un phényle nu.

Parmi les composés de formule générale (I) décrits dans l'invention, un quatrième sous-groupe de composés est constitué par les composés pour lesquels le ou les substituents X, identiques ou différents l'un de l'autre, sont choisis parmi un atome d'hydrogène ou d'halogène, plus particulièrement de fluor, ou un groupe $C_1$-$C_6$-fluoroalkyle, plus particulièrement trifluorométhyle.

Parmi les composés de formule générale (I) décrits dans l'invention, un cinquième sous-groupe de composés est constitué par les composés pour lesquels n est égal à 1.

Parmi les composés de formule générale (I) décrits dans l'invention, un sixième sous-groupe de composés est constitué par les composés pour lesquels Y représente un aryle, plus particulièrement un phényle, ou un hétéroaryle, plus particulièrement un pyridinyle, l'aryle ou l'hétéroaryle étant éventuellement substitué par un ou plusieurs, plus particulièrement par un ou deux, groupes choisis parmi un atome d'halogène, plus particulièrement de fluor ou de chlore, ou un groupe $C_1$-$C_6$-alkyle, plus particulièrement méthyle, $C_1$-$C_6$-fluoroalkyle, plus particulièrement trifluorométhyle, $C_1$-$C_6$-alcoxyle, plus particulièrement méthoxy, $C_1$-$C_6$-fluoroalcoxyle, plus particulièrement trifluorométhoxy.

Parmi les composés de formule générale (I) décrits dans l'invention, un septième sous-groupe de composés est constitué par les composés pour lesquels $Z_1$, $Z_2$, $Z_3$, $Z_4$ représentent, indépendamment l'un de l'autre, un atome d'azote ou un groupe C($R_6$), l'un au moins correspondant à un atome d'azote et l'un au moins correspondant à un groupe C($R_6$) ;

$R_6$ représente un atome d'hydrogène ou d'halogène, plus particulièrement de fluor, ou un groupe $C_1$-$C_6$-alkyle, plus particulièrement un méthyle, $C_1$-$C_6$-fluoroalkyle, plus particulièrement un trifluorométhyle ou $C_1$-$C_6$-alcoxyle, plus particulièrement méthoxy.

Parmi les composés de formule générale (I) décrits dans l'invention, un huitième sous-groupe de composés est constitué par les composés pour lesquels Z représente soit une amine cyclique reliée par l'atome d'azote, de formule :

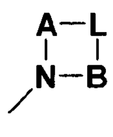

dans laquelle

A représente un groupe $C_1$-$C_7$-alkylène éventuellement substitué par un ou deux groupes $R_8$ ;
B représente un groupe $C_1$-$C_7$-alkylène éventuellement substitué par un ou deux groupes $R_9$ ;
L représente une liaison ;

les atomes de carbone de l'amine cyclique Z étant éventuellement substitués par un ou plusieurs groupes $R_{12}$ identiques ou différents l'un de l'autre ; $R_{12}$ représente un atome de fluor, un groupe $C_1$-$C_6$-alkyle, $C_1$-$C_6$-alcoxyle, $C_3$-$C_7$-cycloalkyle-$C_1$-$C_6$-alkylène-O-, $CO_2H$, C(O)O-$C_1$-$C_6$-alkyle ou hydroxyle ;
$R_8$ et $R_9$ sont définis tels que :
deux groupes $R_8$ peuvent former ensemble une liaison ou un groupe $C_1$-$C_6$-alkylène ;
deux groupes $R_9$ peuvent former ensemble une liaison ou un groupe $C_1$-$C_6$-alkylène ;
$R_8$ et $R_9$ peuvent former ensemble une liaison ou un groupe $C_1$-$C_6$-alkylène ;
soit une amine acylique, reliée par l'atome d'azote, de formule NRaRb dans laquelle Ra et Rb représentent, indépendamment l'un de l'autre, un atome d'hydrogène ou un groupe $C_1$-$C_6$-alkyle, $C_1$-$C_6$-alkyle-C(O)-, HO-C(O)-$C_1$-$C_6$-alkylène ou $C_1$-$C_6$-alkyle-O-C(O)-$C_1$-$C_6$-alkylène, Ra et Rb pouvant être éventuellement substitués par un groupe Rc où Rc représente un hydroxyle.

Parmi les composés de formule générale (I) décrits dans l'invention, un neuvième sous-groupe de composés est constitué par les composés pour lesquels Z représente soit une amine cyclique reliée par l'atome d'azote, de formule :

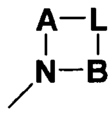

dans laquelle

- A représente un groupe $C_1$-$C_7$-alkylène éventuellement substitué par un groupe $R_8$ ;
- B représente un groupe $C_1$-$C_7$-alkylène éventuellement substitué par un groupe $R_9$ ;
- L représente une liaison ;

les atomes de carbone de l'amine cyclique Z étant éventuellement substitués par un ou plusieurs groupes $R_{12}$ identiques ou différents l'un de l'autre ; $R_{12}$ représente un atome de fluor, un groupe $C_1$-$C_6$-alkyle, $C_1$-$C_6$-alcoxyle, $C_3$-$C_7$-cycloalkyle-$C_1$-$C_6$-alkylène-O-, $CO_2H$, C(O)O-$C_1$-$C_6$-alkyle ou hydroxyle ;
$R_8$ et $R_9$ sont définis tels que :
$R_8$ et $R_9$ peuvent former ensemble une liaison ;
soit une amine acylique, reliée par l'atome d'azote, de formule NRaRb dans laquelle Ra et Rb représentent, indépendamment l'un de l'autre, un atome d'hydrogène ou un groupe $C_1$-$C_6$-alkyle, $C_1$-$C_6$-alkyle-C(O)-, HO-C(O)-$C_1$-$C_6$-alkylène ou $C_1$-$C_6$-alkyle-O-C(O)-$C_1$-$C_6$-alkylène, Ra et Rb pouvant être éventuellement substitués par un groupe Rc où Rc représente un hydroxyle.

Parmi les composés de formule générale (I) décrits dans l'invention, un dixième sous-groupe de composés est constitué par les composés pour lesquels Z représente
soit une amine cyclique reliée par l'atome d'azote et choisie parmi les groupes pyrrolidinyle, azétidinyle, azabicyclo[3.2.0]heptyle ou azabicyclo[3.1.0]hexyle, les atomes de carbone de l'amine cyclique étant éventuellement substitués par un ou plusieurs groupes $R_{12}$ identiques ou différents l'un de l'autre ; $R_{12}$ représente un atome de fluor, un groupe $C_1$-$C_6$-alkyle, plus particulièrement méthyle, $C_1$-$C_6$-alcoxyle, plus particulièrement méthoxy, $C_3$-$C_7$-cycloalkyle-$C_1$-$C_6$-alkylène-O-, plus particulièrement cyclopropylméthyloxy, $CO_2H$, C(O)O-$C_1$-$C_6$-alkyle, plus particulièrement C(O)O-*ter*-butyle, ou hydroxyle ;
soit une amine acylique, reliée par l'atome d'azote, de formule NRaRb dans laquelle Ra et Rb représentent, indépendamment l'un de l'autre, un atome d'hydrogène ou un groupe $C_1$-$C_6$-alkyle, plus particulièrement méthyle, propyle, isopropyle , $C_1$-$C_6$-alkyle-C(O)-, plus particulièrement $CH_3$-C(O)-, HO-C(O)-$C_1$-$C_6$-alkylène, plus particulièrement HO-C(O)-$(CH_2)_2$-, ou $C_1$-$C_6$-alkyle-O-C(O)-$C_1$-$C_6$-alkylène, plus particulièrement $CH_3CH_2$-COO-$(CH_2)_2$-, Ra et Rb pouvant être éventuellement substitués par un groupe Rc où Rc représente un hydroxyle.

Parmi les composés de l'invention, un onzième sous-groupe de composés est constitué par les composés de formule générale (I) dans laquelle à la fois le noyau pyrrolopyridine et/ou X et/ou n et/ou Y et/ou $Z_1$, $Z_2$, $Z_3$, $Z_4$ et/ou Z sont tels que définis dans les groupes ci-dessus.

Ainsi, la présente invention concerne des composés de formule (I)

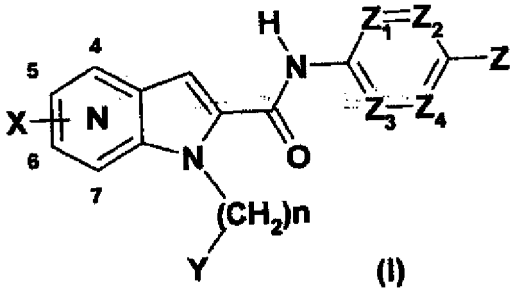

(I)

dans laquelle

le noyau pyrrolopyridine est un groupe pyrrolo[3,2-*b*]pyridine, un groupe pyrrolo[2,3-c]pyridine ou un groupe pyrrolo [2,3-*b*]pyridine ; le noyau pyridine étant éventuellement substitué en position 4,5,6 et/ou 7 par un ou plusieurs substituants le ou les substituents X, identiques ou différents l'un de l'autre, sont choisis parmi un atome d'hydrogène ou d'halogène, plus particulièrement de fluor, ou un groupe $C_1$-$C_6$-fluoroalkyle, plus particulièrement trifluorométhyle ;

n est égal à 1 ;

Y représente un phényle ou un pyridinyle, le phenyle ou le pyridinyle étant éventuellement substitué par un ou plusieurs, plus particulièrement par un ou deux, groupes choisis parmi un atome d'halogène, plus particulièrement de fluor ou de chlore, ou un groupe $C_1$-$C_6$-alkyle, plus particulièrement méthyle, $C_1$-$C_6$-fluoroalkyle, plus particulièrement trifluorométhyle, $C_1$-$C_6$-alcoxyle, plus particulièrement méthoxy, $C_1$-$C_6$-fluoroalcoxyle, plus particulièrement trifluorométhoxy ;

$Z_1$, $Z_2$, $Z_3$, $Z_4$ représentent, indépendamment l'un de l'autre, un atome d'azote ou un groupe $C(R_6)$, l'un au moins correspondant à un atome d'azote et l'un au moins correspondant à un groupe $C(R_6)$ ;

$R_5$ représente un atome d'hydrogène ou d'halogène, plus particulièrement de fluor, ou un groupe $C_1$-$C_6$-alkyle, plus particulièrement un méthyle, $C_1$-$C_6$-fluoroalkyle, plus particulièrement un trifluorométhyle ou $C_1$-$C_6$-alcoxyle, plus particulièrement méthoxy ;

Z représente soit une amine cyclique reliée par l'atome d'azote, de formule :

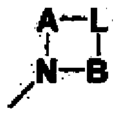

dans laquelle

A représente un groupe $C_1$-$C_7$-alkylène éventuellement substitué par un ou deux groupes $R_8$ ;

B représente un groupe $C_1$-$C_7$-alkylène éventuellement substitué par un ou deux groups $R_9$ :

L représente une liaison ;

les atomes de carbone de l'amine cyclique Z étant éventuellement substitués par un ou plusieurs groupes $R_{12}$ identiques ou différents l'un de l'autre; $R_{12}$ représente un atome de fluor, un groupe $C_1$-$C_6$-alkyle, $C_1$-$C_6$-alcoxyle, $C_3$-$C_7$-cycloalkyle-$C_1$-$C_6$-alkylène-O-, $CO_2H$, C(O)O-$C_1$-$C_6$-alkyle ou hydroxyle ;

$R_8$ et $R_9$ sont définis tels que :

deux groupes $R_8$ peuvent former ensemble une liaison ou un groupe $C_1$-$C_6$-alkylène ;

deux groupes $R_9$ peuvent former ensemble une liaison ou un groupe $C_1$-$C_6$-alkylène;

$R_8$ et $R_9$ peuvent former ensemble une liaison ou un groupe $C_1$-$C_8$-alkylène ;

soit une amine acylique, reliée par l'atome d'azote, de formule NRaRb dans laquelle Ra et Rb représentent, indépendamment l'un de l'autre, un atome d'hydrogène ou un groupe $C_1$-$C_6$-alkyle, $C_1$-$C_8$-alkyle-C(O)-, HO-C(O)-$C_1$-$C_6$-alkylène ou $C_1$-$C_6$-alkyle-O-C(O)-$C_1$-$C_6$-alkylène, Ra et Rb pouvant être éventuellement substitués par un groupe Rc où Rc représente un hydroxyle;

à l'état de base ou de sel d'addition à un acide, ainsi qu'à l'état d'hydrate ou de solvat.

Parmi les composés de formule générale (I) objets de l'invention, un premier sous-groupe de composés est constitué par les composés pour lesquels Z représente soit une amine cyclique reliée par l'atome d'azote, de formule :

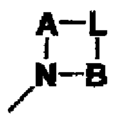

dans laquelle

A représente un groupe $C_1$-$C_7$-alkylène éventuellement substitué par un groupe $R_8$ ;
B représente un groupe $C_1$-$C_7$-alkylène éventuellement substitué par un groupe $R_9$ ;
L représente une liaison ;

les atomes de carbone de l'amine cyclique Z étant éventuellement substitués par un ou plusieurs groupes $R_{12}$ identiques ou différents l'un de l'autre ; $R_{12}$ représente un atome de fluor, un groupe $C_1$-$C_6$-alkyle, $C_1$-$C_6$-alcoxyle, $C_3$-$C_7$-cycloalkyle-$C_1$-$C_6$-alkylène-O-, $CO_2H$, C(O)O-$C_1$-$C_6$-alkyle ou hydroxyle ;
$R_8$ et $R_9$ sont définis tels que :
$R_8$ et $R_9$ peuvent former ensemble une liaison ;
soit une amine acylique, reliée par l'atome d'azote, de formule NRaRb dans laquelle Ra et Rb représentent, indépendamment l'un de l'autre, un atome d'hydrogène ou un groupe $C_1$-$C_6$-alkyle, $C_1$-$C_6$-alkyle-C(O)-, HO-C(O)-$C_1$-$C_6$-alkylène ou $C_1$-$C_6$-alkyle-O-C(O)-$C_1$-$C_6$-alkylène, Ra et Rb pouvant être éventuellement substitués par un groupe Rc où Rc représente un hydroxyle;
à l'état de base ou de sel d'addition à un acide, ainsi qu'à l'état d'hydrate ou de solvat.

Parmi les composés de formule générale (I) objets de l'invention, un deuxième sous-groupe de composés est constitué par les composés pour lesquels Z représente
soit une amine cyclique reliée par l'atome d'azote et choisie parmi les groupes pyrrolidinyle, azétidinyle, azabicyclo[3.2.0]heptyle ou azabicyclo[3.1.0]hexyle, les atomes de carbone de l'amine cyclique étant éventuellement substitués par un ou plusieurs groupes $R_{12}$ identiques ou différents l'un de l'autre ; $R_{12}$ représente un atome de fluor, un groupe $C_1$-$C_6$-alkyle, plus particulièrement méthyle, $C_1$-$C_6$-alcoxyle, plus particulièrement méthoxy, $C_3$-$C_7$-cycloalkyle-$C_1$-$C_6$-alkylène-O-, plus particulièrement cyclopropylméthyloxy, $CO_2H$, C(O)O-$C_1$-$C_6$-alkyle, plus particulièrement C(O)O-*ter*-butyle, ou hydroxyle ;
soit une amine acylique, reliée par l'atome d'azote, de formule NRaRb dans laquelle Ra et Rb représentent, indépendamment l'un de l'autre, un atome d'hydrogène ou un groupe $C_1$-$C_6$-alkyle, plus particulièrement méthyle, propyle, isopropyle, $C_1$-$C_6$-alkyle-C(O)-, plus particulièrement $CH_3$-C(O)-, HO-C(O)-$C_1$-$C_6$-alkylène, plus particulièrement HO-C(O)-$(CH_2)_2$-, ou $C_1$-$C_6$-alkyle-O-C(O)-$C_1$-$C_6$-alkylène, plus particulièrement $CH_3CH_2COO$-$(CH_2)_2$-, Ra et Rb pouvant être éventuellement substitués par un groupe Rc où Rc représente un hydroxyle ;
à l'état de base ou de sel d'addition à un acide, ainsi qu'à l'état d'hydrate ou de solvat.

Parmi les composés de formule générale (I), une sous famille de composés est représentée par la formule générale (Ia) :

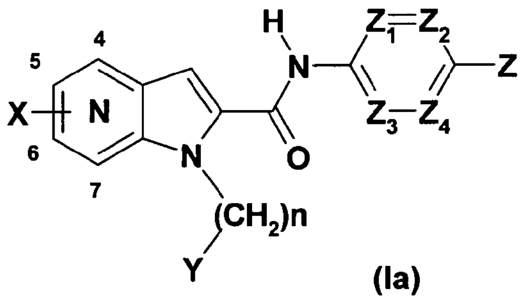

(Ia)

dans laquelle
le noyau pyrrolopyridine est un groupe pyrrolo[3,2-*b*]pyridine, un groupe pyrrolo[3,2-*c*]pyridine, un groupe pyrrolo[2,3-*c*]pyridine ou un groupe pyrrolo[2,3-*b*]pyridine ;
le noyau pyrrolopyridine étant éventuellement substitué en position carbonée 4, 5, 6 et / ou 7 par un ou plusieurs substituents X, identiques ou différents l'un de l'autre, choisis parmi un atome d'hydrogène ou d'halogène ou un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, cyano, $C_1$-$C_6$-thioalkyle, -S(O)-$C_1$-$C_6$-alkyle, -S(O)$_2$-$C_1$-$C_6$-alkyle, $SF_5$, C(O)$NR_1R_2$, $SO_2NR_1R_2$, nitro, $NR_1R_2$, $OCONR_1R_2$, $NR_3COR_4$, $NR_3CONR_1R_2$, $NR_3SO_2R_5$, $NR_3SO_2NR_1R_2$, aryl-$C_1$-$C_6$-alkylène, hétéroaryl-$C_1$-$C_6$-alkylène, aryle ou hétéroaryle, l'aryle et l'hétéroaryle étant éventuellement substitués par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_1$-$C_6$-fluoroalcoxyle, nitro ou cyano ;
n est égal à 0, 1, 2 ou 3 ;
Y représente un aryle ou un hétéroaryle,
l'aryle ou l'hétéroaryle étant éventuellement substitué par un ou plusieurs groupes choisis parmi un atome d'halogène

ou un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, hydroxyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, cyano, $C_1$-$C_6$-thioalkyle, thiol, -S(O)-$C_1$-$C_6$-alkyle, -$S(O)_2$-$C_1$-$C_6$-alkyle, $C(O)NR_1R_2$, $SO_2NR_1R_2$, $SF_5$, nitro, $OCONR_1R_2$, $NR_3COR_4$, $NR_3CONR_1R_2$, $NR_1R_2$, $NR_3SO_2NR_1R_2$, $NR_3COR_4$, $NR_3SO_2R_5$, aryl-$C_1$-$C_6$-alkylène ou aryle, l'aryle et l'aryl-$C_1$-$C_6$-alkylène étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_1$-$C_6$-fluoroalcoxyle, nitro ou cyano ;

$Z_1$, $Z_2$, $Z_3$, $Z_4$ représentent, indépendamment l'un de l'autre, un atome d'azote ou un groupe $C(R_6)$, l'un au moins correspondant à un atome d'azote et l'un au moins correspondant à un groupe $C(R_6)$ ; l'atome d'azote ou un des atomes d'azote présent dans le cycle, défini comme azote de position 1, étant éventuellement substitué par $R_7$ lorsque l'atome de carbone en position 2 ou 4 par rapport à cet azote de référence est substitué par un groupe oxo ou thio ;

Z représente

soit une amine cyclique reliée par l'atome d'azote, de formule :

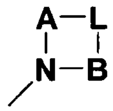

dans laquelle

A représente un groupe $C_1$-$C_7$-alkylène éventuellement substitué par un ou deux groupes $R_8$ ;
B représente un groupe $C_1$-$C_7$-alkylène éventuellement substitué par un ou deux groupes $R_9$ ;
L représente une liaison, un atome de soufre, d'oxygène ou d'azote ; l'atome d'azote étant éventuellement substitué par un groupe $R_{10}$ ou $R_{11}$ ;

les atomes de carbone de l'amine cyclique Z étant éventuellement substitués par un ou plusieurs groupes $R_{12}$ identiques ou différents l'un de l'autre ;

soit une amine acylique, reliée par l'atome d'azote, de formule NRaRb dans laquelle Ra et Rb représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, hydroxyle, $C_1$-$C_6$-alcoxyle, $C_3$-$C_7$-cycloalkyle-$C_1$-$C_6$-alkylène-O-, $C_1$-$C_6$-fluoroalcoxyle, aryle ou hétéroaryle, Ra et Rb pouvant être éventuellement substitués par un ou plusieurs groupes Rc identiques ou différents l'un de l'autre ;

Rc représente un atome d'halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_3$-$C_7$-cycloalkyle-$C_1$-$C_6$-alkylène-O-, $C_1$-$C_6$-fluoroalcoxyle, $C_1$-$C_6$-thioalkyle, -S(O)-$C_1$-$C_6$-alkyle, -$S(O)_2$-$C_1$-$C_6$-alkyle, cyano, $C(O)NR_1R_2$, $NR_1R_2$, $SO_2NR_1R_2$, $NR_3COR_4$, $NR_3SO_2R_5$, $OC(O)NR_1R_2$, $NR_3COOR_5$, $NR_3CONR_1R_2$, $NR_3SO_2NR_1R_2$, hydroxyle, thiol, oxo, thio, aryl-$C_1$-$C_6$-alkylène, aryle ou hétéroaryle, l'aryle et l'hétéroaryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_1$-$C_6$-fluoroalcoxyle, nitro ou cyano ;

$R_1$ et $R_2$ représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_6$-alkylène, aryl-$C_1$-$C_6$-alkylène, aryle ou hétéroaryle, l'aryle et l'hétéroaryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_1$-$C_6$-fluoroalcoxyle, nitro ou cyano ; ou $R_1$ et $R_2$ forment ensemble, avec l'atome d'azote qui les porte, un groupe azétidinyle, pyrrolidinyle, pipéridinyle, azépinyle, morpholinyle, thiomorpholinyle, pipérazinyle, homopipérazinyle, ce groupe étant éventuellement substitué par un groupe $C_1$-$C_6$-alkyle, $C_3$-$C_7$-cycloalkyle, $C_3$-$C_7$-cycloalkyle-$C_1$-$C_6$-alkylène, aryl-$C_1$-$C_6$-alkylène, aryle ou hétéroaryle, l'aryle et l'hétéroaryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_1$-$C_6$-fluoroalcoxyle, nitro ou cyano ;

$R_3$ et $R_4$ représentent, indépendamment l'un de l'autre,
un atome d'hydrogène ou un groupe $C_1$-$C_6$-alkyle, $C_3$-$C_7$-cycloalkyle, $C_3$-$C_7$-cycloalkyle-$C_1$-$C_6$-alkylène, aryl-$C_1$-$C_6$-alkylène, aryle ou hétéroaryle, l'aryle et l'hétéroaryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_1$-$C_6$-fluoroalcoxyle, nitro ou cyano ;

ou $R_3$ et $R_4$ forment ensemble un groupe $(C_2$-$C_5)$alkylène ;

ou $R_1$ et $R_3$ forment ensemble un groupe $(C_2$-$C_5)$alkylène ;

$R_5$ représente un groupe $C_1$-$C_6$-alkyle, $C_3$-$C_7$-cycloalkyle, $C_3$-$C_7$-cycloalkyle-$C_1$-$C_6$-alkylène, aryl-$C_1$-$C_6$-alkylène, aryle

ou hétéroaryle, l'aryle et l'hétéroaryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_1$-$C_6$-fluoroalcoxyle, nitro ou cyano ;

ou $R_3$ et $R_5$ forment ensemble un groupe ($C_2$-$C_5$)alkylène ;

$R_6$ représente un atome d'hydrogène ou d'halogène ou un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, $C_1$-$C_6$-thioalkyle, -S(O)-$C_1$-$C_6$-alkyle, -$S(O)_2$-$C_1$-$C_6$-alkyle, hydroxyle, thiol, oxo, thio, aryle, aryl-$C_1$-$C_6$-alkylène, hétéroaryle, l'aryle et l'hétéroaryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_1$-$C_6$-fluoroalcoxyle, nitro ou cyano ;

$R_7$ représente un atome d'hydrogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, aryle, aryl-$C_1$-$C_6$-alkylène ou hétéroaryle, l'aryle et l'hétéroaryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_1$-$C_6$-fluoroalcoxyle, nitro ou cyano ;

$R_8$, $R_9$ et $R_{10}$ sont définis tels que :

deux groupes $R_8$ peuvent former ensemble une liaison ou un groupe $C_1$-$C_6$-alkylène ;

deux groupes $R_9$ peuvent former ensemble une liaison ou un groupe $C_1$-$C_6$-alkylène ;

$R_8$ et $R_9$ peuvent former ensemble une liaison ou un groupe $C_1$-$C_6$-alkylène ;

$R_8$ et $R_{10}$ peuvent former ensemble une liaison ou un groupe $C_1$-$C_6$-alkylène ;

$R_9$ et $R_{10}$ peuvent former ensemble une liaison ou un groupe $C_1$-$C_6$-alkylène ;

$R_{11}$ représente un atome d'hydrogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, hydroxyle, $C_1$-$C_6$-alkyl-CO-, $COOR_5$, $C(O)NR_1R_2$, aryl-$C_1$-$C_6$-alkylène, aryle ou hétéroaryle, l'aryle et l'hétéroaryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_3$-$C_7$-cycloalcoxyle, $C_1$-$C_6$-fluoroalcoxyle, nitro ou cyano ;

$R_{12}$ représente un atome de fluor, un groupe $C_1$-$C_6$-alkyle éventuellement substitué par un groupe $R_{13}$, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-cycloalk-1,1-diyle, $C_3$-$C_7$-hétérocycloalk-1,1-diyle éventuellement substitué sur un atome d'azote par un groupe $R_{11}$ , $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, $CO_2H$, C(O)O-$C_1$-$C_6$-alkyle, $C(O)NR_1R_2$, $NR_1R_2$, $NR_3COR_4$, $OC(O)NR_1R_2$, $NR_3COOR_5$, $NR_3CONR_1R_2$, hydroxyle, thiol, oxo, thio, aryl-$C_1$-$C_6$-alkylène, aryle, l'aryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_3$-$C_7$-cycloalcoxyle, $C_1$-$C_6$-fluoroalcoxyle, nitro ou cyano ;

$R_{13}$ représente un groupe $C_1$-$C_6$-alcoxyle, $C_3$-$C_7$-cycloalcoxyle, $C_3$-$C_7$-cycloalkyle-$C_1$-$C_6$-alkylène-O-, $C(O)NR_1R_2$, $NR_1R_2$, $NR_3COR_4$, $OC(O)NR_1R_2$, $NR_3COOR_5$, hydroxyle.

Dans les composés de formule générale (Ia), le ou les atomes d'azote peuvent être sous forme oxydée (N-oxyde).

Parmi les composés de formule générale (Ia), un premier sous-groupe de composés est constitué par les composés pour lesquels le noyau pyrrolopyridine est un groupe pyrrolo[3,2-*b*]pyridine ou un groupe pyrrolo[2,3-*b*]pyridine.

Parmi les composés de formule générale (Ia), un second sous-groupe de composés est constitué par les composés pour lesquels, si n est égal à 0, alors le ou les substituents X, identiques ou différents l'un de l'autre, sont choisis parmi un atome d'hydrogène ou d'halogène ou un groupe $C_1$-$C_6$-alkyle, $C_3$-$C_7$-cycloalkyle, $C_3$-$C_7$-cycloalkyle-$C_1$-$C_6$-alkylène, $C_1$-$C_6$-fluoroalkyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, cyano, $C_1$-$C_6$-thioalkyle, -S(O)-$C_1$-$C_6$-alkyle, -$S(O)_2$-$C_1$-$C_6$-alkyle, $SF_5$, $C(O)NR_1R_2$, $SO_2NR_1R_2$, nitro, $NR_1R_2$, $OCONR_1R_2$, $NR_3COR_4$, $NR_3CONR_1R_2$, $NR_3SO_2R_5$, $NR_3SO_2NR_1R_2$.

Parmi les composés de formule générale (Ia), un troisième sous-groupe de composés est constitué par les composés pour lesquels le ou les substituents X, identiques ou différents l'un de l'autre, sont choisis parmi un atome d'halogène, plus particulièrement de fluor, ou un groupe $C_1$-$C_6$-fluoroalkyle, plus particulièrement trifluorométhyle.

Parmi les composés de formule générale (Ia), un quatrième sous-groupe de composés est constitué par les composés pour lesquels n est égal à 1.

Parmi les composés de formule générale (Ia), un cinquième sous-groupe de composés est constitué par les composés pour lesquels Y représente un aryle, plus particulièrement un phényle, ou un hétéroaryle, plus particulièrement un pyridinyle, l'aryle ou l'hétéroaryle étant éventuellement substitué par un ou plusieurs, plus particulièrement par un, atomes d'halogène, plus particulièrement de fluor.

Parmi les composés de formule générale (Ia), un sixième sous-groupe de composés est constitué par les composés pour lesquels $Z_1$, $Z_2$, $Z_3$, $Z_4$ représentent, indépendamment l'un de l'autre, un atome d'azote ou un groupe

$C(R_6)$, l'un au moins correspondant à un atome d'azote et l'un au moins correspondant à un groupe $C(R_6)$ ;
$R_6$ représente un atome d'hydrogène ou un groupe $C_1$-$C_6$-alkyle, plus particulièrement un méthyle, ou $C_1$-$C_6$-fluoroalkyle, plus particulièrement un trifluorométhyle.

Parmi les composés de formule générale (Ia), un septième sous-groupe de composés est constitué par les composés pour lesquels Z représente
soit une amine cyclique reliée par l'atome d'azote, de formule :

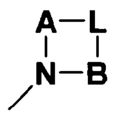

dans laquelle

A représente un groupe $C_1$-$C_7$-alkylène ;
B représente un groupe $C_1$-$C_7$-alkylène ;
L représente une liaison ;

les atomes de carbone de l'amine cyclique Z étant éventuellement substitués par un ou plusieurs groupes $R_{12}$ identiques ou différents l'un de l'autre ; $R_{12}$ représente un atome de fluor, un groupe $C_1$-$C_6$-alkyle, hydroxyle ;
soit une amine acylique, reliée par l'atome d'azote, de formule NRaRb dans laquelle Ra et Rb représentent, indépendamment l'un de l'autre, un atome d'hydrogène ou un groupe $C_1$-$C_6$-alkyle.

Parmi les composés de formule générale (Ia), un huitième sous-groupe de composés est constitué par les composés pour lesquels Z représente
soit un groupe pyrrolidinyle ou azétidinyle, relié par l'atome d'azote, les atomes de carbone du groupe pyrrolidinyle étant éventuellement substitués par un ou plusieurs groupes $R_{12}$ identiques ou différents l'un de l'autre ; $R_{12}$ représente un atome de fluor, un groupe $C_1$-$C_6$-alkyle, plus particulièrement méthyle, ou hydroxyle ;
soit une amine acylique, reliée par l'atome d'azote, de formule NRaRb dans laquelle Ra et Rb représentent, indépendamment l'un de l'autre, un atome d'hydrogène ou un groupe $C_1$-$C_6$-alkyle, plus particulièrement méthyle, isopropyle.

Parmi les composés de formule générale (Ia), un neuvième sous-groupe de composés est constitué par les composés de formule générale (Ia) dans laquelle à la fois le noyau pyrrolopyridine et/ou X et/ou n et/ou Y et/ou $Z_1$, $Z_2$, $Z_3$, $Z_4$ et/ou Z sont tels que définis dans les sous-groupes ci-dessus.

A titre d'exemples non limitatifs d'amines Z substituées ou non substituées , on peut citer la méthylamine, l'éthylamine, la 2-méthoxyéthylamine, la 2-hydroxyéthylamine, la cyclopropylamine, l'hydroxylamine, la 2-(*N,N*-diméthylamino)éthylamine, la diméthylamine, l'isopropylamine, la *N*-éthyl-méthylamine, la 2,5-diméthylpyrrolidine, la 3-hydroxypyrrolidine, la 3-éthoxypyrrolidine, la 3,3-difluoropyrrolidine, la 3,3-difluoroazétidine, la 3-hydroxyazétidine, la proline, l'aziridine, l'azétidine, la pyrrolidine, la pipéridine, l'azépine, la morpholine, la thiomorpholine, la pipérazine, l'homopipérazine, les azabicyclo[3.1.0]hexanes, les azabicyclo[3.2.0]heptanes , les azabicyclo[3.3.0]octanes, les octahydrofuropyrroles, les octahydropyrrolopyrroles, l'octahydroindole, l'octahydroisoindole, les octahydropyrrolopyridines, la décahydroquinoléine, la décahydroisoquinoléine, les décahydronaphthyridines, l'octahydropyridopyrazine, les azabicyclo[3.1.1]heptanes, les diazabicylo[2.2.1]heptanes, les azabicylo[3.2.1]octanes, les diazabicylo[3.2.1]octanes, les azabicyclo[3.3.1]nonanes.

Dans le cadre de la présente invention on entend par :

- $C_t$-$C_z$ où t et z peuvent prendre les valeurs de 1 à 7, une chaîne carbonée pouvant avoir de t à z atomes de carbone, par exemple $C_1$-$C_3$ une chaîne carbonée qui peut avoir de 1 à 3 atomes de carbone ;
- un alkyle : un groupe aliphatique saturé linéaire ou ramifié. A titre d'exemples, on peut citer les groupes méthyle, éthyle, propyle, isopropyle, butyle, isobutyle, tertiobutyle, pentyle, etc ;
- un alkylène : un groupe alkyle divalent saturé, linéaire ou ramifié, par exemple un groupe $C_{1-3}$-alkylène représente une chaîne carbonée divalente de 1 à 3 atomes de carbone, linéaire ou ramifiée, par exemple un méthylène, éthylène, 1-méthyléthylène, propylène ;
- un cycloalkyle : un groupe carboné cyclique. A titre d'exemples, on peut citer les groupes cyclopropyle, cyclobutyle, cyclopentyle, cyclohexyle, etc ;
- un hétérocycloalkyle : un groupe cyclique de 3 à 7 chaînons contenant de 1 à 2 hétéroatomes choisis parmi O, S ou N ;
- un cycloalk-1,1-diyle ou un hétérocycloalk-1,1-diyle : un groupe du type

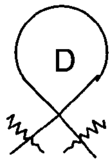

où D est un groupe cycloalkyle ou hétérocycloalkyle ;

- un fluoroalkyle : un groupe alkyle dont un ou plusieurs atomes d'hydrogène ont été substitués par un atome de fluor ;
- un alcoxyle : un radical -O-alkyle où le groupe alkyle est tel que précédemment défini ;
- un cycloalcoxyle : un radical -O-cycloalkyle où le groupe cylcoalkyle est tel que précédemment défini ;
- un fluoroalcoxyle : un groupe alcoxyle dont un ou plusieurs atomes d'hydrogène ont été substitués par un atome de fluor ;
- un thioalkyle : un radical -S-alkyle où le groupe alkyle est tel que précédemment défini ;
- un aryle : un groupe aromatique cyclique comprenant entre 6 et 10 atomes de carbones. A titre d'exemples de groupes aryles, on peut citer les groupes phényle ou naphthyle ;
- un hétéroaryle : un groupe mono-, bi- ou tricyclique aromatique de 5 à 14 chaînons contenant de 1 à 8 hétéroatomes choisis parmi O, S ou N.

A titre d'exemples d'hétéroaryle monocyclique, on peut citer les groupes imidazolyle, pyrazolyle thiazolyle, oxazolyle, isothiazolyle, isoxazolyle, furanyle, thiophényle, oxadiazolyle, thiadiazolyle, triazolyle, tétrazolyle, pyridinyle, pyrazinyle, pyrimidinyle, pyridazinyle, triazinyle ;
à titre d'exemples d'hétéroaryle bicyclique, on peut citer les groupes indolyle, isoindolyle benzofuranyle, benzothiophényle, benzoxazolyle, benzimidazolyle, indazolyle, benzothiazolyle, isobenzofuranyle, isobenzothiazolyle, pyrrolo[2,3-*c*]pyridinyle, pyrrolo[2,3-*b*]pyridinyle, pyrrolo[3,2-*b*]pyridinyle, pyrrolo[3,2-*c*]pyridinyle, quinoléinyle, isoquinoléinyle, cinnolinyle, quinazolinyle ou quinoxalinyle ;
à titre d'exemples d'hétéroaryle tricyclique, on peut citer les groupes pyrido[1,2-*a*]benzimidazolyle, thiazolo[1,2-*a*]benzimidazolyle, imidazo[1,2-*a*]benzimidazolyle , pyrimido[1,2-*a*]benzimidazolyle ou pyrazino[1,2-*a*]benzimidazolyle ;

- un atome d'halogène : un fluor, un chlore, un brome ou un iode ;
- « oxo » signifie « =O » ;
- « thio » signifie « =S ».

Les composés de formule (I) selon l'invention peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.
Les composés de formule (I) selon l'invention peuvent exister à l'état de bases ou de sels d'addition à des acides. De tels sels d'addition font partie de l'invention.
Ces sels sont avantageusement préparés avec des acides pharmaceutiquement acceptables, mais les sels d'autres acides utiles, par exemple, pour la purification ou l'isolement des composés de formule (I) font également partie de l'invention.
Les composés de formule générale (I) selon l'invention peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.
Parmi les composés de formule générale (I) objets de l'invention, on peut notamment citer les composés suivants :

1. *N*-[(6-Méthylamino)pyridin-3-yl]-5-trifluorométhyl-1-(3-fluorobenzyl)-1*H*-pyrrolo[2,3-b]pyridine-2-carboxamide
2. *N*-(6-*N,N*-diméthylamino-5-méthylpyridin-3-yl)-5-trifluorométhyl-1-(3-fluorobenzyl)-1*H*-pyrrolo[2,3-*b*]pyridine-2-carboxamide
3. *N*-(6-*N,N*-diméthylamino-4-méthylpyridin-3-yl)-5-trifluorométhyl-1-(3-fluorobenzyl)-1*H*-pyrrolo[2,3-*b*]pyridine-2-carboxamide
4. *N*-(6-*N,N*-diméthylamino-5-trifluorométhyl-pyridin-3-yl)-5-trifluorométhyl-1-(3-fluorobenzyl)-1 *H*-pyrrolo[2,3-*b*]pyridine-2-carboxamide
5. *N*-[6-(Pyrrolidin-1-yl)pyridin-3-yl]-5-fluoro-1-(3-fluorobenzyl)-1*H*-pyrrolo[2,3-*b*]pyridine-2-carboxamide
6. *N*-[6-(Pyrrolidin-1-yl)pyridin-3-yl]-5-trifluorométhyl-1-(3-fluorobenzyl)-1*H*-pyrrolo[2,3-*b*]pyridine-2-carboxamide
7. *N*-[5-(Pyrrolidin-1-yl)pyrazin-2-yl]-5-trifluorométhyl-1-(3-fluorobenzyl)-1*H*-pyrrolo[2,3-*b*]pyridine-2-carboxamide
8. *N*-[6-(Diméthylamino)pyridazin-3-yl]-5-trifluorométhyl-1-(3-fluorobenzyl)-1*H*-pyrrolo[2,3-*b*]pyridine-2-carboxamide

9. *N*-[6-(pyrrolidin-1-yl)pyridin-3-yl]-5-trifluorométhyl-1-[(4-pyridyl)methyl]-1*H*-pyrrolo[2,3-*b*]pyridine-2-carboxamide

10. *N*-[6-(Isopropylamino)pyridin-3-yl]-5-trifluorométhyl-1-(3-fluorobenzyl)-1*H*-pyrrolo[2,3-*b*]pyridine-2-carboxamide

11. *N*-[6-(3-hydroxypyrrolidin-1-yl)pyridin-3-yl]-5-trifluorométhyl-1-(3-fluorobenzyl)-1*H*-pyrrolo[2,3-*b*]pyridine-2-carboxamide

12. N-[6-(3,3-difluoropyrrolidin-1-yl)pyridin-3-yl]-5-trifluorométhyl-1-(3-fluorobenzyl)-1H-pyrrolo[2,3-*b*]pyridine-2-carboxamide

13. *N*-[6-(diméthylamino)pyridin-3-yl]-5-trifluorométhyl-1-(3-fluorobenzyl)-1*H*-pyrrolo[2,3-*b*]pyridine-2-carboxamide

14. *N*-[6-(cis-2,5-diméthylpyrrolidin-1-yl)pyridin-3-yl]-5-trifluorométhyl-1-(3-fluorobenzyl)-1*H*-pyrrolo[2,3-*b*]pyridine-2-carboxamide

15 *N*-[6-(azétidin-1-yl)pyridin-3-yl]-5-trifluorométhyl-1-(3-fluorobenzyl)-1*H*-pyrrolo[2,3-*b*]pyridine-2-carboxamide

16 *N*-[6-(Pyrrolidin-1-yl)pyridin-3-yl]-5-trifluorométhyl-1-(3-fluorobenzyl)-1*H*-pyrrolo[3,2-*b*]pyridine-2-carboxamide

17 *N*-[6-(2-(S)-carboxypyrrolidin-1-yl)pyridin-3-yl]-5-trifluorométhyl-1-(3-fluorobenzyl)-1*H*-pyrrolo[2,3-*b*]pyridine-2-carboxamide

18 *N*-[6-(2-(S)-tertbutyloxycarbonylpyrrolidin-1-yl)pyridin-3-yl]-5-trifluorométhyl-1-(3-fluorobenzyl)-1*H*-pyrrolo[2,3-*b*]pyridine-2-carboxamide

19 *N*-[6-(3,3-difluoroazétidin-1-yl)pyridin-3-yl]-5-trifluorométhyl-1-(3-fluorobenzyl)-1*H*-pyrrolo[2,3-*b*]pyridine-2-carboxamide

20 *N*-[6-(3-hydroxyazétidin-1-yl)pyridin-3-yl]-5-trifluorométhyl-1-(3-fluorobenzyl)-1*H*-pyrrolo[2,3-*b*]pyridine-2-carboxamide

21 *N*-[4-méthyl-6-diméthylaminopyridin-3-yl]-5-trifluorométhyl-1-(3-fluorobenzyl)-1*H*-pyrrolo[2,3-*b*]pyridine-2-carboxamide

22 *N*-[4-méthyl-6-(pyrrolidin-1-yl)pyridin-3-yl]-6-trifluorométhyl-1-(3-fluorobenzyl)-1*H*-pyrrolo[2,3-*b*]pyridine-2-carboxamide

23 *N*-[6-(3-méthoxypyrrolidin-1-yl)pyridin-3-yl]-5-trifluorométhyl-1-(3-fluorobenzyl)-1*H*-pyrrolo[2,3-*b*]pyridine-2-carboxamide

24 *N*-[4-méthyl-6-diméthylaminopyridin-3-yl]-5-trifluorométhyl-1-[(pyridin-4-yl)méthyl]-1*H*-pyrrolo[2,3-*b*]pyridine-2-carboxamide

25 *N*-[4-méthyl-6-(pyrrolidin-1-yl)pyridin-3-yl]-5-trifluorométhyl-1-(3-fluorobenzyl)-1*H*-pyrrolo[2,3-*b*]pyridine-2-carboxamide

26 *N*-[4-méthyl-6-(pyrrolidin-1-yl)pyridin-3-yl]-6-trifluorométhyl-1-[(pyridin-4-yl)méthyl]-1*H*-pyrrolo[2,3-*b*]pyridine-2-carboxamide

27 *N*-[4-méthyl-6-(pyrrolidin-1-yl)pyridin-3-yl]-5-trifluorométhyl-1-[(pyridin-4-yl)méthyl]-1*H*-pyrrolo[2,3-*b*]pyridine-2-carboxamide

28 *N*-[4-méthyl-6-(méthylamino)pyridin-3-yl]-5-trifluorométhyl-1-[(pyridin-4-yl)méthyl]-1*H*-pyrrolo[2,3-*b*]pyridine-2-carboxamide

29 *N*-[4-méthoxy-6-(pyrrolidin-1-yl)pyridin-3-yl]-5-trifluorométhyl-1-[(pyridin-4-yl)méthyl]-1*H*-pyrrolo[2,3-*b*]pyridine-2-carboxamide

30 *N*-[6-(azétidin-1-yl)pyridin-3-yl]-5-trifluorométhyl-1-[(pyridin-4-yl)méthyl]-1*H*-pyrrolo[2,3-*b*]pyridine-2-carboxamide

31 *N*-[6-(acétylamino)pyridin-3-yl]-5-trifluorométhyl-1-(3-fluorobenzyl)-1*H*-pyrrolo[2,3-*b*]pyridine-2-carboxamide

32 *N*-[6-aminopyridin-3-yl]-5-trifluorométhyl-1-(3-fluorobenzyl)-1*H*-pyrrolo[2,3-*b*]pyridine-2-carboxamide

33 *N*-[6-(pyrrolidin-1-yl)pyridin-3-yl]-6-trifluorométhyl-1-(3-fluorobenzyl)-1*H*-pyrrolo[2,3-*b*]pyridine-2-carboxamide

34 *N*-[6-(pyrrolidin-1-yl)-4-(trifluorométhyl)pyridin-3-yl]-5-trifluorométhyl-1-(3-fluorobenzyl)-1*H*-pyrrolo[2,3-*b*]pyridine-2-carboxamide

35 *N*-[5-fluoro-6-(diméthylamino)pyridin-3-yl]-5-trifluorométhyl-1-(3-fluorobenzyl)-1*H*-pyrrolo[2,3-*b*]pyridine-2-carboxamide

36 *N*-[2-(diméthylamino)pyrimidin-5-yl]-5-trifluorométhyl-1-(3-fluorobenzyl)-1*H*-pyrrolo[2,3-*b*]pyridine-2-carboxamide

37 *N*-[6-(3-azabicyclo[3.2.0]hept-3-yl)pyridin-3-yl]-5-trifluorométhyl-1-(3-fluorobenzyl)-1*H*-pyrrolo[2,3-*b*]pyridine-2-carboxamide

38 *N*-[6-(3-azabicyclo[3.1.0]hex-3-yl)pyridin-3-yl]-5-trifluorométhyl-1-(3-fluorobenzyl)-1*H*-pyrrolo[2,3-*b*]pyridine-2-carboxamide

39 *N*-[6-(3-azabicyclo[3.1.0]hex-3-yl)pyridin-3-yl]-5-fluoro-1-(3-fluorobenzyl)-1*H*-pyrrolo[2,3-*b*]pyridine-2-carboxamide

40 Acide 3-[[5-[[[1-(3-fluorobenzyl)-5-trifluorométhyl-1*H*-pyrrolo[2,3-b]pyridin-2-yl]carbonyl]amino]pyridin-2-yl]amino]propionique

41 *N*-[6-(3-hydroxypropylamino)pyridin-3-yl]-5-trifluorométhyl-1-(3-fluorobenzyl)-1*H*-pyrrolo[2,3-*b*]pyridine-2-carboxamide

42 *N*-[6-(azétidin-1-yl)pyridin-3-yl]-5-trifluorométhyl-1-benzyl-1*H*-pyrrolo[2,3-*b*]pyridine-2-carboxamide

43 *N*-[6-(azétidin-1-yl)pyridin-3-yl]-5-trifluorométhyl-1-(2-fluorobenzyl)-1*H*-pyrrolo[2,3-*b*]pyridine-2-carboxamide

44 *N*-[6-(azétidin-1-yl)pyridin-3-yl]-5-trifluorométhyl-1-(4-fluorobenzyl)-1*H*-pyrrolo[2,3-*b*]pyridine-2-carboxamide

45 *N*-[6-(azétidin-1-yl)pyridin-3-yl]-5-trifluorométhyl-1-(3-trifluorométhyl)benzyl-1*H*-pyrrolo[2,3-*b*]pyridine-2-carboxamide

46 *N*-[6-(azétidin-1-yl)pyridin-3-yl]-5-trifluorométhyl-1-(4-méthylbenzyl)-1*H*-pyrrolo[2,3-*b*]pyridine-2-carboxamide

47 *N*-[6-(azétidin-1-yl)pyridin-3-yl]-5-trifluorométhyl-1-(3-méthylbenzyl)-1*H*-pyrrolo[2,3-*b*]pyridine-2-carboxamide

48 *N*-[6-(azétidin-1-yl)pyridin-3-yl]-5-trifluorométhyl-1-(3-méthoxybenzyl)-1*H*-pyrrolo[2,3-*b*]pyridine-2-carboxamide

49 *N*-[6-(azétidin-1-yl)pyridin-3-yl]-5-trifluorométhyl-1-(3-chlorobenzyl)-1*H*-pyrrolo[2,3-*b*]pyridine-2-carboxamide

50 *N*-[6-(azétidin-1-yl)pyridin-3-yl]-5-trifluorométhyl-1-(3-trifluorométhyloxy)benzyl-1*H*-pyrrolo[2,3-*b*]pyridine-2-carboxamide

51 *N*-[6-(azétidin-1-yl)pyridin-3-yl]-5-trifluorométhyl-1-(4-méthoxybenzyl)-1*H*-pyrrolo[2,3-*b*]pyridine-2-carboxamide

52 *N*-[6-(azétidin-1-yl)pyridin-3-yl]-5-trifluorométhyl-1-[(3-chloro-5-(trifluorométhyl)]benzyl-1*H*-pyrrolo[2,3-*b*]pyridine-2-carboxamide

53 *N*-[6-(azétidin-1-yl)pyridin-3-yl]-5-fluoro-1-benzyl-1*H*-pyrrolo[2,3-*b*]pyridine-2-carboxamide

54 *N*-[6-(azétidin-1-yl)pyridin-3-yl]-5-fluoro-1-(2-fluorobenzyl)-1*H*-pyrrolo[2,3-*b*]pyridine-2-carboxamide

55 *N*-[6-(azétidin-1-yl)pyridin-3-yl]-5-fluoro-1-(4-fluorobenzyl)-1*H*-pyrrolo[2,3-*b*]pyridine-2-carboxamide

56 *N*-[6-(azétidin-1-yl)pyridin-3-yl]-5-fluoro-1-(3-trifluorométhyl)benzyl-1*H*-pyrrolo[2,3-*b*]pyridine-2-carboxamide

57 *N*-[6-(azétidin-1-yl)pyridin-3-yl]-5-fluoro-1-(4-méthylbenzyl)-1*H*-pyrrolo[2,3-*b*]pyridine-2-carboxamide

58 *N*-[6-(azétidin-1-yl)pyridin-3-yl]-5-fuoro-1-(3-méthylbenzyl)-1*H*-pyrrolo[2,3-*b*]pyridine-2-carboxamide

59 *N*-[6-(azétidin-1-yl)pyridin-3-yl]-5-fluoro-1-(3-méthoxybenzyl)-1*H*-pyrrolo[2,3-*b*]pyridine-2-carboxamide

60 *N*-[6-(azétidin-1-yl)pyridin-3-yl]-5-fluoro-1-(3-chlorobenzyl)-1*H*-pyrrolo[2,3-*b*]pyridine-2-carboxamide

61 *N*-[6-(azétidin-1-yl)pyridin-3-yl]-5-fluoro-1-(3-trifluorométhyloxy)benzyl-1*H*-pyrrolo[2,3-*b*]pyridine-2-carboxamide

62 *N*-[6-(azétidin-1-yl)pyridin-3-yl]-5-fluoro-1-(4-méthoxybenzyl)-1*H*-pyrrolo[2,3-*b*]pyridine-2-carboxamide

63 *N*-[6-(azétidin-1-yl)pyridin-3-yl]-5-fluoro-1-[(3-chloro-5-(trifluorométhyl)]benzyl-1*H*-pyrrolo[2,3-*b*]pyridine-2-carboxamide

64 *N*-[6-(azétidin-1-yl)pyridin-3-yl]-6-trifluorométhyl-1-benzyl-1*H*-pyrrolo[2,3-*b*]pyridine-2-carboxamide

65 *N*-[6-(azétidin-1-yl)pyridin-3-yl]-6-trifluorométhyl-1-(2-fluorobenzyl)-1*H*-pyrrolo[2,3-*b*]pyridine-2-carboxamide

66 *N*-[6-(azétidin-1-yl)pyridin-3-yl]-6-trifluorométhyl-1-(4-fluorobenzyl)-1*H*-pyrrolo[2,3-*b*]pyridine-2-carboxamide

67 *N*-[6-(azétidin-1-yl)pyridin-3-yl]-6-trifluorométhyl-1-(3-trifluorométhyl)benzyl-1*H*-pyrrolo[2,3-*b*]pyridine-2-carboxamide

68 *N*-[6-(azétidin-1-yl)pyridin-3-yl]-6-trifluorométhyl-1-(4-méthylbenzyl)-1*H*-pyrrolo[2,3-*b*]pyridine-2-carboxamide

69 *N*-[6-(azétidin-1-yl)pyridin-3-yl]-6-trifluorométhyl-1-(3-méthylbenzyl)-1*H*-pyrrolo[2,3-*b*]pyridine-2-carboxamide

70 *N*-[6-(azétidin-1-yl)pyridin-3-yl]-6-trifluorométhyl-1-(3-méthoxybenzyl)-1*H*-pyrrolo[2,3-*b*]pyridine-2-carboxamide

71 *N*-[6-(azétidin-1-yl)pyridin-3-yl]-6-trifluorométhyl-1-(3-chlorobenzyl)-1*H*-pyrrolo[2,3-*b*]pyridine-2-carboxamide

72 *N*-[6-(azétidin-1-yl)pyridin-3-yl]-6-trifluorométhyl-1-(3-trifluorométhyloxy)benzyl-1*H*-pyrrolo[2,3-*b*]pyridine-2-carboxamide

73 *N*-[6-(azétidin-1-yl)pyridin-3-yl]-6-trifluorométhyl-1-(4-méthoxybenzyl)-1*H*-pyrrolo[2,3-*b*]pyridine-2-carboxamide

74 *N*-[6-(azétidin-1-yl)pyridin-3-yl]-6-trifluorométhyl-1-[(3-chloro-5-(trifluorométhyl)]benzyl-1*H*-pyrrolo[2,3-*b*]pyridine-2-carboxamide

75 *N*-[6-(azétidin-1-yl)pyridin-3-yl]-1-benzyl-1H-pyrrolo[2,3-*b*]pyridine-2-carboxamide

76 *N*-[6-(azétidin-1-yl)pyridin-3-yl]-1-(2-fluorobenzyl)-1*H*-pyrrolo[2,3-*b*]pyridine-2-carboxamide

77 *N*-[6-(azétidin-1-yl)pyridin-3-yl]-(4-fluorobenzyl)-1*H*-pyrrolo[2,3-*b*]pyridine-2-carboxamide

78 *N*-[6-(azétidin-1-yl)pyridin-3-yl]-1-(3-trifluorométhyl)benzyl-1*H*-pyrrolo[2,3-*b*]pyridine-2-carboxamide

79 *N*-[6-(azétidin-1-yl)pyridin-3-yl]-1-(4-méthylbenzyl)-1*H*-pyrrolo[2,3-*b*]pyridine-2-carboxamide

80 *N*-[6-(azétidin-1-yl)pyridin-3-yl]-1-(3-méthylbenzyl)-1*H*-pyrrolo[2,3-*b*]pyridine-2-carboxamide

81 *N*-[6-(azétidin-1-yl)pyridin-3-yl]-1-(3-méthoxybenzyl)-1*H*-pyrrolo[2,3-*b*]pyridine-2-carboxamide

82 *N*-[6-(azétidin-1-yl)pyridin-3-yl]-1-(3-chlorobenzyl)-1*H*-pyrrolo[2,3-*b*]pyridine-2-carboxamide

83 *N*-[6-(azétidin-1-yl)pyridin-3-yl]-1-(3-trifluorométhyloxy)benzyl-1*H*-pyrrolo[2,3-*b*]pyridine-2-carboxamide

84 *N*-[6-(azétidin-1-yl)pyridin-3-yl]-1-(4-méthoxybenzyl)-1*H*-pyrrolo[2,3-*b*]pyridine-2-carboxamide

85 *N*-[6-(azétidin-1-yl)pyridin-3-yl]-1-[(3-chloro-5-(trifluorométhyl)]benzyl]-1*H*-pyrrolo[2,3-*b*]pyridine-2-carboxamide

86 *N*-[6-(3-hydroxyazétidin-1-yl)pyridin-3-yl]-5-trifluorométhyl-1-(2-fluorobenzyl)-1*H*-pyrrolo[2,3-*b*]pyridine-2-carboxamide

87 *N*-[6-(3-hydroxyazétidin-1-yl)pyridin-3-yl]-5-trifluorométhyl-1-(4-fluorobenzyl)-1*H*-pyrrolo[2,3-*b*]pyridine-2-carboxamide

88 *N*-[6-(3-hydroxyazétidin-1-yl)pyridin-3-yl]-5-trifluorométhyl-1-(3-trifluorométhyl)benzyl-1*H*-pyrrolo[2,3-*b*]pyridine-2-carboxamide
89 *N*-[6-(3-hydroxyazétidin-1-yl)pyridin-3-yl]-5-trifluorométhyl-1-(4-méthylbenzyl)-1*H*-pyrrolo[2,3-*b*]pyridine-2-carboxamide
90 *N*-[6-(3-hydroxyazétidin-1-yl)pyridin-3-yl]-5-trifluorométhyl-1-(3-méthylbenzyl)-1*H*-pyrrolo[2,3-*b*]pyridine-2-carboxamide
91 *N*-[6-(3-hydroxyazétidin-1-yl)pyridin-3-yl]-5-trifluorométhyl-1-(3-méthoxybenzyl)-1*H*-pyrrolo[2,3-*b*]pyridine-2-carboxamide
92 *N*-[6-(3-hydroxyazétidin-1-yl)pyridin-3-yl]-5-trifluorométhyl-1-(3-chlorobenzyl)-1*H*-pyrrolo[2,3-*b*]pyridine-2-carboxamide
93 *N*-[6-(3-hydroxyazétidin-1-yl)pyridin-3-yl]-5-trifluorométhyl-1-(3-trifluorométhyloxy)benzyl-1*H*-pyrrolo[2,3-*b*]pyridine-2-carboxamide
94 *N*-[6-(3-hydroxyazétidin-1-yl)pyridin-3-yl]-5-trifluorométhyl-1-(4-méthoxybenzyl)-1 *H*-pyrrolo[2,3-*b*]pyridine-2-carboxamide
95 *N*-[6-(3-hydroxyazétidin-1-yl)pyridin-3-yl]-5-trifluorométhyl-1-[(3-chloro-5-(trifluorométhyl)]benzyl-1*H*-pyrrolo[2,3-*b*]pyridine-2-carboxamide
96 *N*-[6-(3-hydroxyazétidin-1-yl)pyridin-3-yl]-6-trifluorométhyl-1-benzyl-1*H*-pyrrolo[2,3-*b*]pyridine-2-carboxamide
97 *N*-[6-(3-hydroxyazétidin-1-yl)pyridin-3-yl]-6-trifluorométhyl-1-(2-fluorobenzyl)-1*H*-pyrrolo[2,3-*b*]pyridine-2-carboxamide
98 *N*-[6-(3-hydroxyazétidin-1-yl)pyridin-3-yl]-6-trifluorométhyl-1-(4-fluorobenzyl)-1*H*-pyrrolo[2,3-*b*]pyridine-2-carboxamide
99 *N*-[6-(3-hydroxyazétidin-1-yl)pyridin-3-yl]-6-trifluorométhyl-1-(3-trifluorométhyl)benzyl-1*H*-pyrrolo[2,3-*b*]pyridine-2-carboxamide
100 *N*-[6-(3-hydroxyazétidin-1-yl)pyridin-3-yl]-6-trifluorométhyl-1-(4-méthylbenzyl)-1*H*-pyrrolo[2,3-*b*]pyridine-2-carboxamide
101 *N*-[6-(3-hydroxyazétidin-1-yl)pyridin-3-yl]-6-trifluorométhyl-1-(3-méthylbenzyl)-1*H*-pyrrolo[2,3-*b*]pyridine-2-carboxamide
102 *N*-[6-(3-hydroxyazétidin-1-yl)pyridin-3-yl]-6-trifluorométhyl-1-(3-méthoxybenzyl)-1 *H*-pyrrolo[2,3-*b*]pyridine-2-carboxamide
103 *N*-[6-(3-hydroxyazétidin-1-yl)pyridin-3-yl]-6-trifluorométhyl-1-(3-chlorobenzyl)-1*H*-pyrrolo[2,3-*b*]pyridine-2-carboxamide
104 *N*-[6-(3-hydroxyazétidin-1-yl)pyridin-3-yl]-6-trifluorométhyl-1-(3-trifluorométhyloxy)benzyl-1*H*-pyrrolo[2,3-*b*]pyridine-2-carboxamide
105 *N*-[6-(3-hydroxyazétidin-1-yl)pyridin-3-yl]-6-trifluorométhyl-1-(4-méthoxybenzyl)-1*H*-pyrrolo[2,3-*b*]pyridine-2-carboxamide
106 *N*-[6-(3-hydroxyazétidin-1-yl)pyridin-3-yl]-6-trifluorométhyl-1-[(3-chloro-5-(trifluorométhyl)]benzyl-1*H*-pyrrolo[2,3-*b*]pyridine-2-carboxamide
107 *N*-[6-(3-hydroxyazétidin-1-yl)pyridin-3-yl]-5-fluoro-1-benzyl-1*H*-pyrrolo[2,3-*b*]pyridine-2-carboxamide
108 *N*-[6-(3-hydroxyazétidin-1-yl)pyridin-3-yl]-5-fluoro-1-(2-fluorobenzyl)-1*H*-pyrrolo[2,3-*b*]pyridine-2-carboxamide
109 *N*-[6-(3-hydroxyazétidin-1-yl)pyridin-3-yl]-5-fluoro-1-(4-fluorobenzyl)-1*H*-pyrrolo[2,3-*b*]pyridine-2-carboxamide
110 *N*-[6-(3-hydroxyazétidin-1-yl)pyridin-3-yl]-5-fluoro-1-(3-trifluorométhyl)benzyl-1*H*-pyrrolo[2,3-*b*]pyridine-2-carboxamide
*111* *N*-[6-(3-hydroxyazétidin-1-yl)pyridin-3-yl]-5-fluoro-1-(4-méthylbenzyl)-1*H*-pyrrolo[2,3-*b*]pyridine-2-carboxamide
112 *N*-[6-(3-hydroxyazétidin-1-yl)pyridin-3-yl]-5-fuoro-1-(3-méthylbenzyl)-1*H*-pyrrolo[2, 3-*b*]pyrid ine-2-carboxamide
113 *N*-[6-(3-hydroxyazétidin-1-yl)pyridin-3-yl]-5-fluoro-1-(3-méthoxybenzyl)-1*H*-pyrrolo[2,3-*b*]pyridine-2-carboxamide
114 *N*-[6-(3-hydroxyazétidin-1-yl)pyridin-3-yl]-5-fluoro-1-(3-chlorobenzyl)-1*H*-pyrrolo[2,3-*b*]pyridine-2-carboxamide
115 *N*-[6-(3-hydroxyazétidin-1-yl)pyridin-3-yl]-5-fluoro-1-(3-trifluorométhyloxy)benzyl-1*H*-pyrrolo[2,3-*b*]pyridine-2-carboxamide
116 *N*-[6-(3-hydroxyazétidin-1-yl)pyridin-3-yl]-5-fluoro-1-(4-méthoxybenzyl)-1*H*-pyrrolo[2, 3-*b*]pyrid ine-2-carboxamide
117 *N*-[6-(3-hydroxyazétidin-1-yl)pyridin-3-yl]-5-fluoro-1-[(3-chloro-5-(trifluorométhyl)]benzyl-1*H*-pyrrolo[2,3-*b*]pyridine-2-carboxamide
118 *N*-[6-(3-hydroxyazétidin-1-yl)pyridin-3-yl]-1-benzyl-1*H*-pyrrolo[2,3-*b*]pyridine-2-carboxamide
119 *N*-[6-(3-hydroxyazétidin-1-yl)pyridin-3-yl]-1-(2-fluorobenzyl)-1*H*-pyrrolo[2,3-*b*]pyridine-2-carboxamide
120 *N*-[6-(3-hydroxyazétidin-1-yl)pyridin-3-yl]-(4-fluorobenzyl)-1*H*-pyrrolo[2,3-*b*]pyridine-2-carboxamide

121 *N*-[6-(3-hydroxyazétidin-1-yl)pyridin-3-yl]-1-(3-trifluorométhyl)benzyl-1*H*-pyrrolo[2,3-*b*]pyridine-2-carboxamide
122 *N*-[6-(3-hydroxyazétidin-1-yl)pyridin-3-yl]-1-(4-méthylbenzyl)-1*H*-pyrrolo[2,3-*b*]pyridine-2-carboxamide
123 *N*-[6-(3-hydroxyazétidin-1-yl)pyridin-3-yl]-1-(3-méthylbenzyl)-1*H*-pyrrolo[2,3-*b*]pyridine-2-carboxamide
124 *N*-[6-(3-hydroxyazétidin-1-yl)pyridin-3-yl]-1-(3-méthoxybenzyl)-1*H*-pyrrolo[2,3-*b*]pyridine-2-carboxamide
125 *N*-[6-(3-hydroxyazétidin-1-yl)pyridin-3-yl]-1-(3-chlorobenzyl)-1*H*-pyrrolo[2,3-*b*]pyridine-2-carboxamide
126 *N*-[6-(3-hydroxyazétidin-1-yl)pyridin-3-yl]-1-(3-trifluorométhyloxy)benzyl-1*H*-pyrrolo[2,3-*b*]pyridine-2-carboxamide
127 *N*-[6-(3-hydroxyazétidin-1-yl)pyridin-3-yl]-1-(4-méthoxybenzyl)-1*H*-pyrrolo[2,3-*b*]pyridine-2-carboxamide
128 *N*-[6-(3-hydroxyazétidin-1-yl)pyridin-3-yl]-1-[(3-chloro-5-(trifluorométhyl)]benzyl-1*H*-pyrrolo[2,3-*b*]pyridine-2-carboxamide
129 *N*-[6-(azétidin-1-yl)pyridin-3-yl]-5-trifluorométhyl-1-benzyl-1*H*-pyrrolo[3,2-*b*]pyridine-2-carboxamide
130 *N*-[6-(azétidin-1-yl)pyridin-3-yl]-5-trifluorométhyl-1-(2-fluorobenzyl)-1*H*-pyrrolo[3,2-*b*]pyridine-2-carboxamide
131 *N*-[6-(azétidin-1-yl)pyridin-3-yl]-5-trifluorométhyl-1-(4-fluorobenzyl)-1*H*-pyrrolo[3,2-*b*]pyridine-2-carboxamide
132 *N*-[6-(azétidin-1-yl)pyridin-3-yl]-5-trifluorométhyl-1-(3-trifluorométhyl)benzyl-1*H*-pyrrolo[3,2-*b*]pyridine-2-carboxamide
133 *N*-[6-(azétidin-1-yl)pyridin-3-yl]-5-trifluorométhyl-1-(4-méthylbenzyl)-1*H*-pyrrolo[3,2-*b*]pyridine-2-carboxamide
134 *N*-[6-(azétidin-1-yl)pyridin-3-yl]-5-trifluorométhyl-1-(3-méthylbenzyl)-1*H*-pyrrolo[3,2-*b*]pyridine-2-carboxamide
135 *N*-[6-(azétidin-1-yl)pyridin-3-yl]-5-trifluorométhyl-1-(3-méthoxybenzyl)-1*H*-pyrrolo[3,2-*b*]pyridine-2-carboxamide
136 *N*-[6-(azétidin-1-yl)pyridin-3-yl]-5-trifluorométhyl-1-(3-chlorobenzyl)-1*H*-pyrrolo[3,2-*b*]pyridine-2-carboxamide
137 *N*-[6-(azétidin-1-yl)pyridin-3-yl]-5-trifluorométhyl-1-(3-trifluorométhyloxy)benzyl-1*H*-pyrrolo[3,2-*b*]pyridine-2-carboxamide
138 *N*-[6-(azétidin-1-yl)pyridin-3-yl]-5-trifluorométhyl-1-(4-méthoxybenzyl)-1*H*-pyrrolo[3,2-*b*]pyridine-2-carboxamide
139 *N*-[6-(azétidin-1-yl)pyridin-3-yl]-5-trifluorométhyl-1-[(3-chloro-5-(trifluorométhyl)]benzyl-1*H*-pyrrolo[3,2-*b*]pyridine-2-carboxamide
*140* *N*-[6-(3-hydroxyazétidin-1-yl)pyridin-3-yl]-5-trifluorométhyl-1-benzyl-1*H*-pyrrolo[3,2-*b*]pyridine-2-carboxamide
141 *N*-[6-(3-hydroxyazétidin-1-yl)pyridin-3-yl]-5-trifluorométhyl-1-(2-fluorobenzyl)-1*H*-pyrrolo[3,2-*b*]pyridine-2-carboxamide
142 *N*-[6-(3-hydroxyazétidin-1-yl)pyridin-3-yl]-5-trifluorométhyl-1-(4-fluorobenzyl)-1*H*-pyrrolo[3,2-*b*]pyridine-2-carboxamide
143 *N*-[6-(3-hydroxyazétidin-1-yl)pyridin-3-yl]-5-trifluorométhyl-1-(3-trifluorométhyl)benzyl-1*H*-pyrrolo[3,2-*b*]pyridine-2-carboxamide
144 *N*-[6-(3-hydroxyazétidin-1-yl)pyridin-3-yl]-5-trifluorométhyl-1-(4-méthylbenzyl)-1*H*-pyrrolo[3,2-*b*]pyridine-2-carboxamide
145 *N*-[6-(3-hydroxyazétidin-1-yl)pyridin-3-yl]-5-trifluorométhyl-1-(3-méthylbenzyl)-1*H*-pyrrolo[3,2-*b*]pyridine-2-carboxamide
146 *N*-[6-(3-hydroxyazétidin-1-yl)pyridin-3-yl]-5-trifluorométhyl-1-(3-méthoxybenzyl)-1*H*-pyrrolo[3,2-*b*]pyridine-2-carboxamide
147 *N*-[6-(3-hydroxyazétidin-1-yl)pyridin-3-yl]-5-trifluorométhyl-1-(3-chlorobenzyl)-1*H*-pyrrolo[3,2-*b*]pyridine-2-carboxamide
148 *N*-[6-(3-hydroxyazétidin-1-yl)pyridin-3-yl]-5-trifluorométhyl-1-(3-rifluorométhyloxy)benzyl-1*H*-pyrrolo[3,2-*b*]pyridine-2-carboxamide
149 *N*-[6-(azétidin-1-yl)pyridin-3-yl]-5-trifluorométhyl-1-(4-méthoxybenzyl)-1*H*-pyrrolo[3,2-*b*]pyridine-2-carboxamide
150 *N*-[6-(3-hydroxyazétidin-1-yl)pyridin-3-yl]-5-trifluorométhyl-1-[(3-chloro-5-(trifluorométhyl)]benzyl-1*H*-pyrrolo[3,2-*b*]pyridine-2-carboxamide
*151* *N*-[6-(azétidin-1-yl)pyridin-3-yl]-1-benzyl-1*H*-pyrrolo[2,3-*c*]pyridine-2-carboxamide 152 *N*-[6-(azétidin-1-yl)pyridin-3-yl]-1-(2-fluorobenzyl)-1*H*-pyrrolo[2,3-*c*]pyridine-2-carboxamide
153 *N*-[6-(azétidin-1-yl)pyridin-3-yl]-(4-fluorobenzyl)-1*H*-pyrrolo[2,3-*c*]pyridine-2-carboxamide
154 *N*-[6-(azétidin-1-yl)pyridin-3-yl]-1-(3-trifluorométhyl)benzyl-1*H*-pyrrolo[2,3-*c*]pyridine-2-carboxamide
155 *N*-[6-(azétidin-1-yl)pyridin-3-yl]-1-(4-méthylbenzyl)-1*H*-pyrrolo[2,3-*c*]pyridine-2-carboxamide
156 *N*-[6-(azétidin-1-yl)pyridin-3-yl]-1-(3-méthylbenzyl)-1*H*-pyrrolo[2,3-*c*]pyridine-2-carboxamide
157 *N*-[6-(azétidin-1-yl)pyridin-3-yl]-1-(3-méthoxybenzyl)-1H-pyrrolo[2,3-*c*]pyridine-2-carboxamide
158 *N*-[6-(azétidin-1-yl)pyridin-3-yl]-1-(3-chlorobenzyl)-1*H*-pyrrolo[2,3-*c*]pyridine-2-carboxamide
159 *N*-[6-(azétidin-1-yl)pyridin-3-yl]-1-(3-trifluorométhyloxy)benzyl-1*H*-pyrrolo[2,3-*c*]pyridine-2-carboxamide
160 *N*-[6-(azétidin-1-yl)pyridin-3-yl]-1-[(3-chloro-5-(trifluorométhyl)]benzyl-1*H*-pyrrolo[2,3-c]pyridine-2-carboxamide

161 *N*-[6-(3-hydroxyazétidin-1-yl)pyridin-3-yl]-1-benzyl-1*H*-pyrrolo[2,3-*c*]pyridine-2-carboxamide
162 *N*-[6-(3-hydroxyazétidin-1-yl)pyridin-3-yl]-1-(2-fluorobenzyl)-1*H*-pyrrolo[2,3-*c*]pyridine-2-carboxamide
163 *N*-[6-(3-hydroxyazétidin-1-yl)pyridin-3-yl]-(4-fluorobenzyl)-1*H*-pyrrolo[2,3-*c*]pyridine-2-carboxamide
164 *N*-[6-(3-hydroxyazétidin-1-yl)pyridin-3-yl]-1-(3-trifluorométhyl)benzyl-1*H*-pyrrolo[2,3-*c*]pyridine-2-carboxamide
165 *N*-[6-(3-hydroxyazétidin-1-yl)pyridin-3-yl]-1-(4-méthylbenzyl)-1*H*-pyrrolo[2,3-*c*]pyridine-2-carboxamide
166 *N*-[6-(3-hydroxyazétidin-1-yl)pyridin-3-yl]-1-(3-méthylbenzyl)-1*H*-pyrrolo[2,3-*c*]pyridine-2-carboxamide
167 *N*-[6-(3-hydroxyazétidin-1-yl)pyridin-3-yl]-1-(3-méthoxybenzyl)-1*H*-pyrrolo[2,3-*c*]pyridine-2-carboxamide
168 *N*-[6-(3-hydroxyazétidin-1-yl)pyridin-3-yl]-1-(3-chlorobenzyl)-1*H*-pyrrolo[2,3-*c*]pyridine-2-carboxamide
169 *N*-[6-(3-hydroxyazétidin-1-yl)pyridin-3-yl]-1-(3-trifluorométhyloxy)benzyl-1 *H*-pyrrolo[2,3-*c*]pyridine-2-carboxamide
170 *N*-[6-(3-hydroxyazétidin-1-yl)pyridin-3-yl]-1-[(3-chloro-5-(trifluorométhyl)]benzyl-1*H*-pyrrolo[2,3-c]pyridine-2-carboxamide
171 3-[[5-[[[1-(3-fluorobenzyl)-5-trifluorométhyl-1*H*-pyrrolo[2,3-*b*]pyridin-2-yl]carbonyl]amino]pyridin-2-yl]amino] propionate d'éthyle
172 *N*-[6-(3-méthoxyazétidin-1-yl)pyridin-3-yl]-5-trifluorométhyl-1-(3-fluorobenzyl)-1H-pyrrolo[2,3-*b*]pyridine-2-carboxamide
173 *N*-[6-[3-(cyclopropylméthyloxy)azétidin-1-yl]pyridin-3-yl]-5-trifluorométhyl-1-(3-fluorobenzyl)-1 *H*-pyrrolo[2,3-*b*] pyridine-2-carboxamide

Parmi les composés de formule générale (I) objets de l'invention, on peut également citer les composés suivants :

1. *N*-[(6-Méthylamino)pyridin-3-yl]-5-trifluorométhyl-1-(3-fluorobenzyl)-1*H*-pyrrolo[2,3-*b*]pyridine-2-carboxamide
2. *N*-(6-*N,N*-diméthylamino-5-méthylpyridin-3-yl)-5-trifluorométhyl-1-(3-fluorobenzyl)-1*H*-pyrrolo[2,3-*b*]pyridine-2-carboxamide
3. *N*-(6-*N,N*-diméthylamino-4-méthylpyridin-3-yl)-5-trifluorométhyl-1-(3-fluorobenzyl)-1*H*-pyrrolo[2,3-*b*]pyridine-2-carboxamide
4. *N*-(6-*N,N*-diméthylamino-5-trifluorométhyl-pyridin-3-yl)-5-trifluorométhyl-1-(3-fluorobenzyl)-1*H*-pyrrolo[2,3-*b*]pyridine-2-carboxamide
5. *N*-[6-(Pyrrolidin-1-yl)pyridin-3-yl]-5-fluoro-1-(3-fluorobenzyl)-1*H*-pyrrolo[2,3-*b*]pyridine-2-carboxamide
6. *N*-[6-(Pyrrolidin-1-yl)pyridin-3-yl]-5-trifluorométhyl-1-(3-fluorobenzyl)-1*H*-pyrrolo[2,3-*b*]pyridine-2-carboxamide
7. *N*-[5-(Pyrrolidin-1-yl)pyrazin-2-yl]-5-trifluorométhyl-1-(3-fluorobenzyl)-1*H*-pyrrolo[2,3-*b*]pyridine-2-carboxamide
8. *N*-[6-(Diméthylamino)pyridazin-3-yl]-5-trifluorométhyl-1-(3-fluorobenzyl)-1*H*-pyrrolo[2,3-*b*]pyridine-2-carboxamide
9. *N*-[6-(pyrrolidin-1-yl)pyridin-3-yl]-5-trifluorométhyl-1-[(4-pyridyl)methyl]-1*H*-pyrrolo[2,3-*b*]pyridine-2-carboxamide
10. *N*-[6-(Isopropylamino)pyridin-3-yl]-5-trifluorométhyl-1-(3-fluorobenzyl)-1*H*-pyrrolo[2,3-*b*]pyridine-2-carboxamide
11. *N*-[6-(3-hydroxypyrrolidin-1-yl)pyridin-3-yl]-5-trifluorométhyl-1-(3-fluorobenzyl)-1*H*-pyrrolo[2,3-*b*]pyridine-2-carboxamide
12. *N*-[6-(3,3-difluoropyrrolidin-1-yl)pyridin-3-yl]-5-trifluorométhyl-1-(3-fluorobenzyl)-1*H*-pyrrolo[2,3-*b*]pyridine-2-carboxamide
13. *N*-[6-(diméthylamino)pyridin-3-yl]-5-trifluorométhyl-1-(3-fluorobenzyl)-1*H*-pyrrolo[2,3-*b*]pyridine-2-carboxamide
14. *N*-[6-(cis-2,5-diméthylpyrrolidin-1-yl)pyridin-3-yl]-5-trifluorométhyl-1-(3-fluorobenzyl)-1*H*-pyrrolo[2,3-*b*]pyridine-2-carboxamide
15. *N*-[6-(azétidin-1-yl)pyridin-3-yl]-5-trifluorométhyl-1-(3-fluorobenzyl)-1*H*-pyrrolo[2,3-*b*]pyridine-2-carboxamide
16. *N*-[6-(Pyrrolidin-1-yl)pyridin-3-yl]-5-trifluorométhyl-1-(3-fluorobenzyl)-1*H*-pyrrolo[3,2-*b*]pyridine-2-carboxamide
17 *N*-[6-(2-(S)-carboxypyrrolidin-1-yl)pyridin-3-yl]-5-trifluorométhyl-1-(3-fluorobenzyl)-1*H*-pyrrolo[2,3-*b*]pyridine-2-carboxamide
18 *N*-[6-(2-(*S*)-tertbutyloxycarbonylpyrrolidin-1-yl)pyridin-3-yl]-5-trifluorométhyl-1-(3-fluorobenzyl)-1*H*-pyrrolo[2,3-*b*]pyridine-2-carboxamide
19 *N*-[6-(3,3-difluoroazétidin-1-yl)pyridin-3-yl]-5-trifluorométhyl-1-(3-fluorobenzyl)-1*H*-pyrrolo[2,3-*b*]pyridine-2-carboxamide
20 *N*-[6-(3-hydroxyazétidin-1-yl)pyridin-3-yl]-5-trifluorométhyl-1-(3-fluorobenzyl)-1*H*-pyrrolo[2,3-*b*]pyridine-2-carboxamide
21 *N*-[4-méthyl-6-diméthylaminopyridin-3-yl]-5-trifluorométhyl-1-(3-fluorobenzyl)-1*H*-pyrrolo[2,3-*b*]pyridine-2-carboxamide
22. *N*-[4-méthyl-6-(pyrrolidin-1-yl)pyridin-3-yl]-6-trifluorométhyl-1-(3-fluorobenzyl)-1*H*-pyrrolo[2,3-*b*]pyridine-2-carboxamide

23. *N*-[6-(3-méthoxypyrrolidin-1-yl)pyridin-3-yl]-5-trifluorométhyl-1-(3-fluorobenzyl)-1*H*-pyrrolo[2,3-*b*]pyridine-2-carboxamide
24. *N*-[4-méthyl-6-diméthylaminopyridin-3-yl]-5-trifluorométhyl-1-[(pyridin-4-yl)méthyl]-1*H*-pyrrolo[2,3-*b*]pyridine-2-carboxamide
25. *N*-[4-méthyl-6-(pyrrolidin-1-yl)pyridin-3-yl]-5-trifluorométhyl-1-(3-fluorobenzyl)-1*H*-pyrrolo[2,3-*b*]pyridine-2-carboxamide
26. *N*-[4-méthyl-6-(pyrrolidin-1-yl)pyridin-3-yl]-6-trifluorométhyl-1-[(pyridin-4-yl)méthyl]-1*H*-pyrrolo[2,3-*b*]pyridine-2-carboxamide
27. *N*-[4-méthyl-6-(pyrrolidin-1-yl)pyridin-3-yl]-5-trifluorométhyl-1-[(pyridin-4-yl)méthyl]-1*H*-pyrrolo[2,3-*b*]pyridine-2-carboxamide
28. *N*-[4-méthyl-6-(méthylamino)pyridin-3-yl]-5-trifluorométhyl-1-[(pyridin-4-yl)méthyl]-1*H*-pyrrolo[2,3-*b*]pyridine-2-carboxamide
29. *N*-[4-méthoxy-6-(pyrrolidin-1-yl)pyridin-3-yl]-5-trifluorométhyl-1-[(pyridin-4-yl)méthyl]-1*H*-pyrrolo[2,3-*b*]pyridine-2-carboxamide
30. *N*-[6-(azétidin-1-yl)pyridin-3-yl]-5-trifluorométhyl-1-[(pyridin-4-yl)méthyl]-1*H*-pyrrolo[2,3-*b*]pyridine-2-carboxamide
31. *N*-[6-(acétylamino)pyridin-3-yl]-5-trifluorométhyl-1-(3-fluorobenzyl)-1*H*-pyrrolo[2,3-*b*]pyridine-2-carboxamide
32. *N*-[6-aminopyridin-3-yl]-5-trifluorométhyl-1-(3-fluorobenzyl)-1*H*-pyrrolo[2,3-*b*]pyridine-2-carboxamide
33. *N*-[6-(pyrrolidin-1-yl)pyridin-3-yl]-6-trifluorométhyl-1-(3-fluorobenzyl)-1*H*-pyrrolo[2,3-*b*]pyridine-2-carboxamide
34. *N*-(6-(pyrrolidin-1-yl)-4-(trifluorométhyl)pyridin-3-yl]-5-trifluorométhyl-1-(3-fluorobenzyl)-1*H*-pyrrolo[2,3-*b*]pyridine-2-carboxamide
35. *N*-[5-fluoro-6-(diméthylamino)pyridin-3-yl]-5-trifluorométhyl-1-(3-fluorobenzyl)-1*H*-pyrrolo[2,3-*b*]pyridine-2-carboxamide
36. *N*-[2-(diméthylamino)pyrimidin-5-yl]-5-trifluorométhyl-1-(3-fluorobenzyl)-1*H*-pyrrolo[2,3-*b*]pyridine-2-carboxamide
37. *N*-[6-(3-azabicyclo[3.2.0]hept-3-yl)pyridin-3-yl]-5-trifluorométhyl-1-(3-fluorobenzyl)-1*H*-pyrrolo[2,3-*b*]pyridine-2-carboxamide
38. *N*-[6-(3-azabicyclo[3.1.0]hex-3-yl)pyridin-3-yl]-5-trifluorométhyl-1-(3-fluorobenzyl)-1*H*-pyrrolo[2,3-*b*]pyridine-2-carboxamide
39. *N*-[6-(3-azabicyclo[3.1.0]hex-3-yl)pyridin-3-yl]-5-fluoro-1-(3-fluorobenzyl)-1*H*-pyrrolo[2,3-*b*]pyridine-2-carboxamide
40. Acide 3-[[5-[[[1-(3-fluorobenzyl)-5-trifluorométhyl-1*H*-pyrrolo[2,3-*b*]pyridin-2-yl]carbonyl]amino]pyridin-2-yl]amino]propionique
41. *N*-[6-(3-hydroxypropylamino)pyridin-3-yl]-5-trifluorométhyl-1-(3-fluorobenzyl)-1*H*-pyrrolo[2,3-*b*]pyridine-2-carboxamide
43. *N*-[6-(azétidin-1-yl)pyridin-3-yl]-5-trifluorométhyl-1-(2-fluorobenzyl)-1*H*-pyrrolo[2,3-*b*]pyridine-2-carboxamide
44. *N*-[6-(azétidin-1-yl)pyridin-3-yl]-5-trifluorométhyl-1-(4-fluorobenzyl)-1*H*-pyrrolo[2,3-*b*]pyridine-2-carboxamide
45. *N*-[6-(azétidin-1-yl)pyridin-3-yl]-5-trifluorométhyl-1-(3-trifluorométhyl)benzyl-1*H*-pyrrolo[2,3-*b*]pyridine-2-carboxamide
46. *N*-[6-(azétidin-1-yl)pyridin-3-yl]-5-trifluorométhyl-1-(4-méthylbenzyl)-1*H*-pyrrolo[2,3-*b*]pyridine-2-carboxamide *b*]pyridine-2-carboxamide
48. *N*-[6-(azétidin-1-yl)pyridin-3-yl]-5-trifluorométhyl-1-(3-méthoxybenzyl)-1*H*-pyrrolo[2,3-*b*]pyridine-2-carboxamide
49. *N*-[6-(azétidin-1-yl)pyridin-3-yl]-5-trifluorométhyl-1-(3-chlorobenzyl)-1*H*-pyrrolo[2,3-*b*]pyridine-2-carboxamide
50. *N*-[6-(azétidin-1-yl)pyridin-3-yl]-5-trifluorométhyl-1-(3-trifluorométhyloxy)benzyl-1*H*-pyrrolo[2,3-*b*]pyridine-2-carboxamide
51. *N*-[6-(azétidin-1-yl)pyridin-3-yl]-5-trifluorométhyl-1-(4-méthoxybenzyl)-1*H*-pyrrolo(2,3-*b*]pyridine-2-carboxamide
62. *N*-[6-(azétidin-1-yl)pyridin-3-yl]-5-fluoro-1-(4-méthoxybenzyl)-1*H*-pyrrolo[2,3-*b*]pyridine-2-carboxamide
67. *N*-[6-(azétidin-1-yl)pyridin-3-yl]-6-trifluorométhyl-1-(3-trifluorométhyl)benzyl-1*H*-pyrrolo[2,3-*b*]pyridine-2-carboxamide
68. *N*-[6-(azétidin-1-yl)pyridin-3-yl]-6-trifluorométhyl-1-(4-méthylbenzyl)-1*H*-pyrrolo[2,3-*b*]pyridine-2-carboxamide
71. *N*-[6-(azétidin-1-yl)pyridin-3-yl]-6-trifluorométhyl-1-(3-chlorobenzyl)-1*H*-pyrrolo[2,3-*b*]pyridine-2-carboxamide
75. *N*-[6-(azétidin-1-yl)pyridin-3-yl]-1-benzyl-1*H*-pyrrolo[2,3-*b*]pyridine-2-carboxamide 78. *N*-[6-(azétidin-1-yl)pyridin-3-yl]-1-(3-trifluorométhyl)benzyl-1*H*-pyrrolo[2,3-*b*]pyridine-2-carboxamide
81. *N*-[6-(azétidin-1-yl)pyridin-3-yl]-1-(3-méthoxybenzyl)-1*H*-pyrrolo[2,3-*b*]pyridine-2-carboxamide
84. *N*-[6-(azétidin-1-yl)pyridin-3-yl]-1-(4-méthoxybenzyl)-1*H*-pyrrolo[2,3-*b*]pyridine-2-carboxamide
115. *N*-[6-(3-hydroxyazétidin-1-yl)pyridin-3-yl]-5-fluoro-1-(3-trifluorométhyloxy)benzyl-1*H*-pyrrolo[2,3-*b*]pyridine-2-carboxamide

*116.* *N*-[6-(3-hydroxyazétidin-1-yl)pyridin-3-yl]-5-fluoro-1-(4-méthoxybenzyl)-1*H*-pyrrolo[2,3-*b*]pyridine-2-carboxamide

*117.* *N*-[6-(3-hydroxyazétidin-1-yl)pyridin-3-yl]-5-fluoro-1-[(3-chloro-5-(trifluorométhyl)]benzyl-1*H*-pyrrolo[2,3-*b*]pyridine-2-carboxamide

124. *N*-[6-(3-hydroxyazétidin-1-yl)pyridin-3-yl]-1-(3-méthoxybenzyl)-1*H*-pyrrolo[2,3-*b*]pyridine-2-carboxamide

125. *N*-[6-(3-hydroxyazétidin-1-yl)pyridin-3-yl]-1-(3-chlorobenzyl)-1*H*-pyrrolo[2,3-*b*]pyridine-2-carboxamide

129. *N*-[6-(azétidin-1-yl)pyridin-3-yl]-5-trifluorométhyl-1-benzyl-1*H*-pyrrolo[3,2-*b*]pyridine-2-carboxamide

*130.* *N*-[6-(azétidin-1-yl)pyridin-3-yl]-5-trifluorométhyl-1-(2-fluorobenzyl)-1*H*-pyrrolo[3,2-*b*]pyridine-2-carboxamide

131. *N*-[6-(azétidin-1-yl)pyridin-3-yl]-5-trifluorométhyl-1-(4-fluorobenzyl)-1*H*-pyrrolo[3,2-*b*]pyridine-2-carboxamide

134. *N*-[6-(azétidin-1-yl)pyridin-3-yl]-5-trifluorométhyl-1-(3-méthylbenzyl)-1*H*-pyrrolo[3,2-*b*]pyridine-2-carboxamide

135. *N*-[6-(azétidin-1-yl)pyridin-3-yl]-5-trifluorométhyl-1-(3-méthoxybenzyl)-1*H*-pyrrolo[3,2-*b*]pyridine-2-carboxamide

138. *N*-[6-(azétidin-1-yl)pyridin-3-yl]-5-trifluorométhyl-1-(4-méthoxybenzyl)-1*H*-pyrrolo[3,2-*b*]pyridine-2-carboxamide

164. *N*-[6-(3-hydroxyazétidin-1-yl)pyridin-3-yl]-1-(3-trifluorométhyl)benzyl-1*H*-pyrrolo[2,3-*c*]pyridine-2-carboxamide

165. *N*-[6-(3-hydroxyazétidin-1-yl)pyridin-3-yl]-1-(4-méthylbenzyl)-1*H*-pyrrolo[2,3-*c*]pyridine-2-carboxamide

Dans ce qui suit, on entend par groupe partant, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxyle activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 5th Edition, Wiley Interscience, 2001.

On peut préparer les composés de formule générale (I) selon le procédé illustré par le schéma 1 qui suit.

Selon le schéma 1, les composés de formule générale (IV) peuvent être obtenus par réaction d'un composé de formule générale (II) dans laquelle X est tel que défini dans la formule générale (I) et B représente un groupe $C_1$-$C_6$-alcoxyle, avec un composé de formule générale (III), dans laquelle Y et n sont tels que définis dans la formule générale (I) et GP représente un groupe partant ou GP représente un groupe hydroxyle.

Dans le schéma 1, les composés de formule générale (II), quand leur mode de préparation n'est pas décrit, sont disponibles dans le commerce, décrits dans la littérature ou préparés par analogie à de nombreux procédés décrits dans la littérature (P. Roy et al Synthesis 2005, 16, 2751-2757 ; N. Lahance et al Synthesis 2005, 15, 2571-2577 ; C. Barberis et al Tetrahedron Left 2005, 46(51), 8877-8880 ; T. Lomberget Synlett 2005, 13, 2080-2082 ; 1909 ; M. Nazare et al Angew Chem Int Ed 2004, 43(34), 4526-4528 ; P.M. Fresneda et al Tetrahedron Lett 2000, 41(24), 4777-4780 ).

Lorsque le composé de formule générale (III), est défini tel que n est égal à 1, 2 ou 3 et GP représente un groupe partant tel qu'un atome de chlore, de brome ou d'iode, la réaction peut être réalisée en présence d'une base telle que l'hydrure de sodium ou le carbonate de potassium, dans un solvant polaire tel que le diméthylformamide, le diméthylsulfoxyde ou l'acétone (n = 1 : Kolasa T., Bioorg.Med.Chem. 1997, 5 (3) 507, n = 2 : Abramovitch R., Synth. Commun., 1995, 25 (1), 1).

Lorsque le composé de formule générale (III) est défini tel que n est égal à 1, 2 ou 3 et GP représente un groupe hydroxyle, les composés de formule générale (IV), peuvent être obtenus par réaction du composé de formule générale (II) avec un composé de formule générale (III) en présence d'une phosphine telle que, par exemple, la triphénylphosphine et d'un réactif tel que, par exemple, l'azodicarboxylate de diéthyle en solution dans un solvant tel que le dichlorométhane ou le tétrahydrofuranne (O. Mitsonobu, Synthesis, 1981, 1-28). De façon analogue, les composés de formule générale (IV), peuvent être obtenus par réaction du composé de formule générale (II) avec un composé de formule générale (III) en présence d'une phosphine supportée sur une résine et d'un réactif tel que, par exemple, l'azodicarboxylate de diisopropyle en solution dans un solvant tel que le dichlorométhane ou le tétrahydrofuranne.

Schéma 1

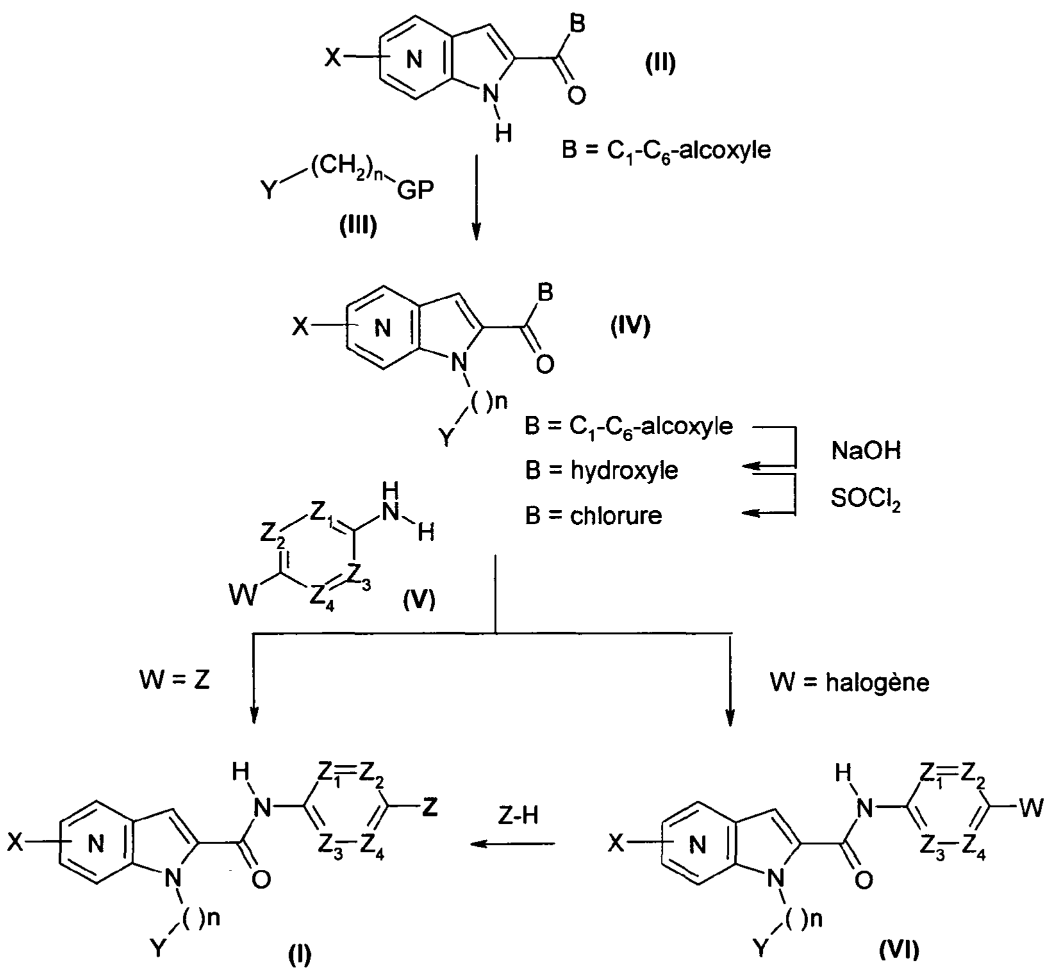


Lorsque le composé de formule générale (III) est défini tel que n est égal à 0 et GP représente un groupe partant tel qu'un atome de chlore, de brome ou d'iode, la réaction peut être réalisée par application et adaptation des méthodes décrites par S. L. Buchwald et al. (J. Am. Chem. Soc., 2001, 123, 7727 et 2002, 124, 11684), de préférence sous atmosphère inerte en milieu basique, par exemple en présence de triphosphate de potassium, en présence d'un sel de cuivre tel que l'iodure de cuivre, éventuellement en présence d'un additif tel que la *N,N'*-diméthylcyclohexane-1,2-diamine, le tout dans un solvant organique tel que le toluène.

Le composé de formule générale (IV), pour lequel B représente un groupe $C_1$-$C_6$-alcoxyle, peut être transformé en composé de formule générale (IV), où B représente un groupe hydroxyle, par l'action d'une base telle que la soude ou la potasse en solution dans un solvant tel que l'éthanol. Le composé de formule générale (IV), où B représente un groupe hydroxyle peut, par la suite, être transformé en composé de formule générale (IV), où B représente un atome de chlore, par l'action d'un agent chlorant tel que que le chlorure de thionyle dans un solvant tel que le dichlorométhane.

Le composé de formule générale (VI) peut ensuite être obtenu, par exemple, par réaction d'un composé de formule générale (IV) où B est un atome de chlore, tel qu'obtenu ci-dessus, avec une amine de formule générale (V), dans laquelle $Z_1$, $Z_2$, $Z_3$, $Z_4$ sont tels que définis dans la formule générale (I) et W correspond à un halogène tel qu'un atome de chlore, dans un solvant tel que le dichloroéthane, le toluène ou le tétrahydrofuranne.

Le composé de formule générale (VI) peut également être obtenu par réaction d'un composé de formule générale (IV) où B est un groupe hydroxyle, tel qu'obtenu ci-dessus, avec une amine de formule générale (V), dans laquelle $Z_1$, $Z_2$, $Z_3$, $Z_4$ sont tels que définis dans la formule générale (I) et W correspond à un atome d'halogène tel qu'un atome de chlore, en présence d'un agent de couplage tel que le cyanophosphonate de diéthyle ou le *N*-(3-diméthylaminopropyl)-*N'*-éthylcarbodiimide, éventuellement en présence d'une base telle que la triéthylamine, dans un solvant tel que le diméthylformamide.

Le composé de formule générale (I) peut être obtenu, par exemple, par réaction d'un composé de formule générale (IV) où B est un atome de chlore, tel qu'obtenu ci-dessus, avec une amine de formule générale (V), dans

laquelle W = Z et Z, $Z_1$, $Z_2$, $Z_3$, $Z_4$ sont tels que définis dans la formule générale (I), dans un solvant tel que le dichloroéthane, le toluène ou le tétrahydrofuranne.

Le composé de formule générale (I) peut également être obtenu par réaction d'un composé de formule générale (IV) où B est un groupe hydroxyle, tel qu'obtenu ci-dessus, avec une amine de formule générale (V), dans laquelle W = Z et Z, $Z_1$, $Z_2$, $Z_3$, $Z_4$ sont tels que définis dans la formule générale (I), en présence d'un agent de couplage tel que le diéthylcyanophosphonate ou le *N*-(3-diméthylaminopropyl)-*N'*-éthylcarbodiimide, et éventuellement en présence d'une base telle que la triéthylamine, dans un solvant tel que le diméthylformamide.

Le composé de formule générale (I) peut également être obtenu par réaction d'un composé de formule générale (VI) en présence d'une amine de formule Z-H, dans laquelle Z est tel que défini dans la formule générale (I), sans solvant ou dans un solvant tel que la N-méthylpyrrolidone, ou bien par application et adaptation des méthodes décrites par S. L. Buchwald et al. (J. Am. Chem. Soc., 2001, 123, 7727 et 2002, 124, 11684), de préférence sous atmosphère inerte en milieu basique, par exemple en présence de triphosphate de potassium, en présence d'un sel de cuivre tel que l'iodure de cuivre, éventuellement en présence d'un additif tel que la *N,N'*-diméthylcyclohexane-1,2-diamine, le tout dans un solvant organique tel que le toluène, ou bien par application et adaptation des méthodes décrites par Hartwig et al. (Angewandte Chemie, 2005, 44, 1371-1375), par exemple en présence d'une base et de quantités catalytiques d'un catalyseur à base de palladium, tel que le palladium diacétate, et d'une phosphine.

Les composés de formules générales (I), (II) et (IV), dans lesquelles X représente un groupe cyano ou un aryle, peuvent être obtenus par une réaction de couplage, catalysée par un métal tel que le palladium, réalisée sur les composés de formules générales (I), (II) et (IV) correspondants, dans lesquelles X représente un groupe partant, par exemple un brome, selon des méthodes qui sont décrites dans la littérature ou qui sont connues de l'homme de métier.

Les composés de formules générales (I), (II) et (IV), dans lesquelles X représente un groupe $C(O)NR_1R_2$, peuvent être obtenus à partir des composés de formules générales (I), (II) et (IV) correspondants, dans lesquelles X représente un groupe cyano, selon des méthodes qui sont décrites dans la littérature ou qui sont connues de l'homme du métier. Les composés de formules générales (I), (II), (IV) et (VI) dans lesquelles X représente un groupe -S(O)-alkyle ou $-S(O)_2$-alkyle, peuvent être obtenus par oxydation des composés de formules générales (I), (II), (IV) et (VI) correspondants, dans lesquelles X représente représentent un groupe $C_1$-$C_6$-thioalkyle, selon des méthodes qui sont décrites dans la littérature ou qui sont connues de l'homme du métier.

Les composés de formules générales (I), (II), (IV) et (VI) dans lesquelles X représente un groupe $NR_1R_2$, $NR_3COR_4$ ou $NR_3SO_2R_5$, peuvent être obtenus à partir des composés de formules générales (I), (II), (IV) et (VI) correspondants, dans lesquelles X représente un groupe nitro, par exemple par réduction, puis acylation ou sulfonylation, selon des méthodes qui sont décrites dans la littérature ou qui sont connues de l'homme du métier. Les composés de formules générales (I), (II) et (IV), dans lesquelles X représente un groupe $NR_1R_2$, $NR_3COR_4$ ou $NR_3SO_2R_5$, peuvent être obtenus à partir des composés de formules générales (I), (II) et (IV) correspondants, dans lesquelles X représente par exemple, un atome de brome par réaction de couplage respectivement avec une amine, un amide ou une sulfonamide en présence d'une base, d'une phosphine et d'un catalyseur à base de palladium, selon des méthodes qui sont décrites dans la littérature ou qui sont connues de l'homme du métier.

Les composés de formules générales (I), (II) et (IV), dans lesquelles X représente un groupe $SO_2NR_1R_2$ peuvent être obtenus par une méthode analogue à celle décrite dans Pharmazie 1990, 45, 346, ou selon des méthodes qui sont décrites dans la littérature ou qui sont connues de l'homme du métier.

Les composés de formule générale (I) dans lesquelles Z représente une amine acyclique NRaRb correspondant à un groupe $NH_2$ peuvent être obtenus, selon des conditions connues de l'homme de l'art et décrites dans la littérature (Greene, Wuts, Protective groups in organic synthesis, Wiley-Interscience) à partir de précurseurs de formule générale (I) où NRaRb = NH-GP, GP correspondant à un groupe protecteur tel qu'un groupe acétyle ou terbutoxycarbonyle.

Les composés de formule générale (I) comportant un groupe $CO_2H$ peuvent être obtenus, selon des conditions connues de l'homme de l'art et décrites dans la littérature (Greene, Wuts, Protective groups in organic synthesis, Wiley-Interscience) à partir de précurseurs de formule générale (I) comportant un groupe $CO_2GP$, GP correspondant à un groupe protecteur d'acide carboxylique tel qu'un groupe méthyle ou terbutyle.

Les composés de formule générale (I) comportant un groupe $CH_2OH$ peuvent être obtenus, selon des conditions connues de l'homme de l'art à partir de composés de formule générale (I) comportant un groupe $CO_2$alky, par exemple, par réaction en présence d'un agent réducteur tel que le borohydrure de sodium dans un solvant tel que le tétrahydrofuranne.

Les composés de formule générale (III) sont disponibles dans le commerce, décrits dans la littérature (Carling R.W. et al J.Med.Chem. 2004 (47), 1807 - 1822 ou Russel M.G.N. et al. J.Med.Chem. 2005 (48), 1367 - 1383) ou accessibles en utilisant des méthodes connues de l'homme du métier.

Les composés de formule générale (V) et les autres réactifs, quand leur mode de préparation n'est pas décrit, sont disponibles dans le commerce ou décrits dans la littérature (WO200502845, WO2002048152, WO2006040522 W02004052869, W02004062665, WO2005035526, W02004110986, Heterocycles 1977, 6(12), 1999 - 2004, par exemple).

L'invention vise également un procédé de préparation d'un composé de formule (I) selon l'invention caractérisé en ce que l'on fait réagir un composé de formule générale (IV)

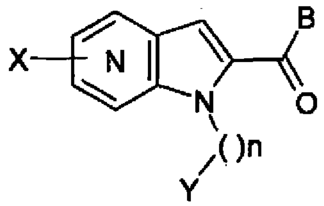

**(IV)**

dans laquelle X, Y et n sont tels que définis dans la formule générale (I) selon l'invention, et B représente un atome de chlore, avec une amine de formule générale (V)

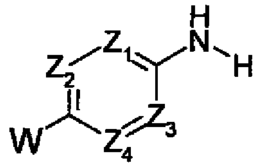

dans laquelle W=Z et Z, $Z_1$, $Z_2$, $Z_3$ et $Z_4$ sont tels que définis dans la formule générale (I) selon l'invention, dans un solvant.

L'invention vise également un procédé de préparation d'un composé de formule (I) selon l'invention caractérisé en ce que l'on fait réagir un composé de formule générale (IV)

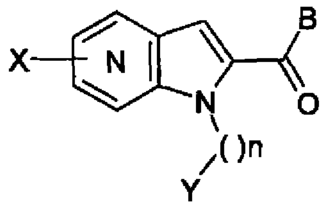

**(IV)**

dans laquelle X, Y et n sont tels que définis dans la formule générale (I) selon l'invention, et B représente un groupe hydroxyle, avec une amine de formule générale (V)

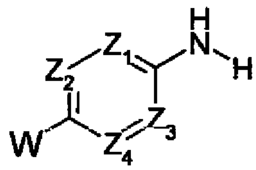

dans laquelle W=Z et Z, $Z_1$, $Z_2$, $Z_3$ et $Z_4$ sont tels que définis dans la formule générale (I) selon l'invention, en présence d'un agent de couplage et éventuellement d'une base, dans un solvant.

L'invention vise également un procédé de préparation d'un composé de formule (I) selon l'invention caractérisé en ce que l'on fait réagir un composé de formule générale (VI)

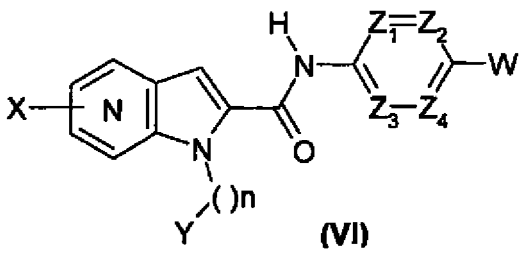

**(VI)**

dans laquelle X, Y, n, $Z_1$, $Z_2$, $Z_3$ et $Z_4$ sont tels que définis dans la formule générale (I) selon l'invention, et W représente

un atome d'halogène, avec une amine de formule Z-H dans laquelle Z est tel que défini dans la formule générale (I) selon l'invention.

L'invention, selon un autre de ses aspects, a également pour objet les composés de formules (Va), (Vb), (Vc), (Vd), (Ve), (Vf), (Vg) et (Vh). Ces composés sont utiles comme intermédiaires de synthèse pour la préparation des composés de formule (I).

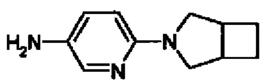
(Va)
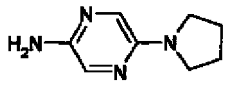
(Vb)
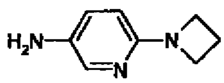
(Vc)
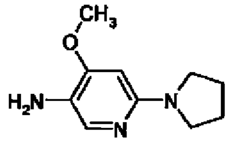
(Vd)

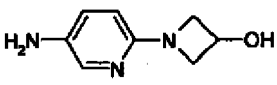
(Ve)
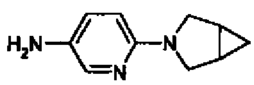
(Vf)
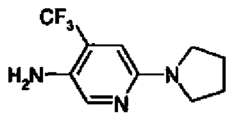
(Vg)
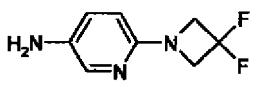
(Vh)

Les amines de formules (Va), (Vb), (Vc), (Vd), (Ve), (Vf), (Vg) et (Vh) peuvent être préparées, par exemple selon le procédé décrit dans l'exemple 12.1, par substitution nucléophile aromatique d'un précurseur 6-halogénopyridine, éventuellement substitué (Vd : par un groupe 4-méthoxy, Vg : par un groupe 4-trifluorométhyle), avec une amine, telle que la pyrrolidine, par exemple dans un solvant tel que l'éthanol.

L'amine (Vb) peut être préparée par exemple selon le procédé décrit dans l'exemple n°7 en chauffant une solution de 2-amino-5-bromopyrazine commerciale dans la pyrrolidine. L'accès aux amines Va-h peut aussi nécessiter la réduction d'un groupe nitro, par exemple, par hydrogénation catalytique en présence d'un catalyseur tel que le palladium sur charbon, ou par toutes autres méthodes connues de l'homme de l'art, de réduction d'un groupe nitro en amine. L'accès aux amines Va-h peut également nécessiter, comme dans le cas de l'amine (Vg) décrite à l'exemple 17, l'introduction d'un groupe amine par réarrangement d'un groupe acide carboxylique selon la méthode de curtius ou par toutes autres méthodes connues de l'homme de l'art.

Les amines de formules (Va), (Vb), (Vc), (Vd), (Ve), (Vf), (Vg) et (Vh) ont été préparées, à l'état de base ou de sel d'addition à un acide.

Le tableau 1 rassemble les données RMN $^1H$ de ces amines.

Tableau 1

| N° | R.M.N. $^1H$, $\delta$ (ppm): |
|---|---|
| Va | Dans le DMSO $D_6$, $\delta$ (ppm): 7,62 (d, 1H) ; 6,95 (d, 1H) ; 6,49 (d, 1H) ; 4,42 (pic élargi, 2H) ; 3,52 (d, 2H) ; 2,98 (m, 4H) ; 2,21 (m, 2H) ; 1,72 (m, 2H). |
| Vb | Dans le DMSO $D_6$, $\delta$ (ppm): 7,5 (d, 1H) ; 7,32 (d, 1H) ; 5,15 (s, 2H) ; 3,25 (m, 4H) ; 1,88 (m, 4H). |
| Vc | Dans le DMSO $D_6$, $\delta$ (ppm): 7,57 (d, 1H) ; 6,92 (dxd, 1H) ; 6,2 (d, 1H) ; 4,46 (pic élargi, 2H) ; 3,78 (m, 4H) ; 2,25 (quint., 2H). |
| Vd | Dans le DMSO $D_6$, $\delta$ (ppm): 7,16 (s, 1H) ; 6,61 (s, 1H) ; 4,52 (pic élargi, 2H) ; 3,71 (s, 3H) ; 3,29 (m, 4H) ; 1,81 (m, 4H). |
| Ve | Dans le DMSO $D_6$, $\delta$ (ppm): 7,58 (s, 1H) ; 6,91 (d, 1H) ; 6,22 (d, 1H) ; 5,48 (pic élargi, 1H) ; 4,48 (pic élargi, 3H) ; 3,98 (m, 2H) ; 3,49 (m, 2H) ; |
| Vf | Dans le DMSO $D_6$, $\delta$ (ppm): 7,55 (s, 1H) ; 6,99 (d, 1H) ; 6,25 (d, 1H) ; 4,32 (pic élargi, 2H) ; 3,51 (d, 2H) ; 3,11 (m, 2H) ; 1,6 (m, 2H) ; 0,66 (m, 1H) ; 0,2 (m, 1 H). |
| Vg (HCl 1:1) | Dans le DMSO $D_6$, $\delta$ (ppm): 7,92 (s, 1H) ; 7,49 (pic élargi, 2H) ; 7,1 (s, 1H) ; 3,52 (m, 4H) ; 2 (m, 4H). |
| Vh | Dans le DMSO $D_6$, $\delta$ (ppm): 7,6 (s, 1H) ; 6,96 (dxd, 1H) ; 6,4 (d, 1H) ; 4,67 (s, 2H) ; 4,2 (t, 4H). |

Les exemples suivants décrivent la préparation de certains composés conformes à l'invention. Ces exemples ne sont pas limitatifs et ne font qu'illustrer la présente invention. Les numéros des composés exemplifiés renvoient à

ceux donnés dans le tableau 1. Les microanalyses élémentaires, les analyses LC-MS (chromatographie liquide couplée à la spectrométrie de masse) les spectres I.R. ou les spectres R.M.N. confirment les structures des composés obtenus.

**<u>Exemple 1</u> (Composé N°1 du tableau 2)**

*N*-[(6-Méthylamino)pyridin-3-yl]-5-trifluorométhyl-1-(3-fluorobenzyl)-1*H*-pyrrolo[2,3-b]pyridine-2-carboxamide

1.1. 5-Trifluorométhyl-1*H*-pyrrolo[2,3-*b*]pyridine-2-carboxylate d'éthyle

Dans un ballon de 100 mL, muni d'un agitateur magnétique, est introduit 0,3 g (1,3 mmole) d'acide 5-trifluorométhyl-1*H*-pyrrolo[2,3-*b*]pyridine-2-carboxylique (Angew Chem Int Ed 2004, 43(34), 4526-4528) et 50 mL d'éthanol. A cette solution, on ajoute 0,5 mL d'acide sulfurique concentré. Le mélange réactionnel est ensuite porté à reflux pendant 18 heures. La solution refroidie est concentrée à sec sous pression réduite. Le résidu est repris au dichlorométhane (100 mL), on lave la phase organique successivement avec une solution aqueuse de soude normale (30 mL), avec de l'eau (20 mL) puis avec une solution aqueuse saturée en chlorure de sodium. On la séche sur sulfate de sodium puis on la concentre sous pression réduite. On isole 0,29 g (1,12 mmole) du produit attendu sous forme d'une poudre jaune.
R.M.N. $^1$H (DMSO $D_6$), δ (ppm) : 12,95 (s, NH) ; 8,8 (d, 1 H) ; 8,6 (d, 1 H) ; 7,3 (s, 1 H) ; 4,4 (q, 2H) ; 1,35 (t, 3H).

1.2 5-Trifluorométhyl-1-[(3-fluorophényl)méthyl]-1*H*-pyrrolo[2,3-*b*]pyridine-2-carboxylate d'éthyle

A une solution de 0,3 g (1,16 mmole) de produit obtenu à l'étape 1.1, dans 20 mL de tétrahydrofurane sec, maintenue sous atmosphère inerte, sont ajoutés successivement sous agitation, 0,23 g (1,74 mmole) d'alcool 3-fluorobenzylique puis 0,46 g (1,74 mmole) de triphénylphosphine. On ajoute ensuite, goutte à goutte, 0,31 g (1,74 mmole) d'azodicarboxylate de diéthyle. Le mélange réactionnel est alors agité pendant 20 h à température ambiante puis concentré sous pression réduite. On purifie l'huile résultante par chromatographie sur colonne de gel de silice en éluant avec un mélange d'heptane et d'acétate d'éthyle. On isole 0,34 g (0,93 mmole) du produit attendu.
R.M.N. $^1$H (DMSO $D_6$), δ (ppm) : 8,9 (d, 1 H) ; 8,7 (d, 1 H) ; 7,5 (s, 1 H) ; 7,4-6,95 (m, 2H) ; 6,85 (m, 2H) ; 5,9 (s, 2H) ; 4,3 (q, 2H) , 1,3 (t, 3H).

1.3 Acide 5-trifluorométhyl-1-[(3-fluorophényl)méthyl]-1*H*-pyrrolo[2,3-*b*]pyridine-2-carboxylique

Une solution de 3,15 g (8,6 mmoles) de 5-trifluorométhyl-1-[(3-fluorophényl)méthyl]-1iH-pyrrolo[2,3-*b*]pyridine-2-carboxylate d'éthyle obtenu à l'étape 1.2 dans 100 mL d'éthanol et de 26 mL de soude 2N est agitée quatre heures à reflux. Après ce temps, le mélange réactionnel est concentré sous pression réduite puis repris dans 40 mL d'eau. Le milieu est acidifié à pH 3 par ajouts successifs d'acide chlorhydrique 1 N. Le précipité est recueilli par filtration, lavé à l'eau puis séché sous pression réduite. On isole ainsi 2,9 g du produit attendu, sous la forme d'une poudre blanche, qui est utilisé tel quel dans l'étape suivante.
R.M.N. $^1$H (DMSO $D_6$), δ (ppm): 13,5 (pic élargi, 1H) ; 8,81 (d, 1H) ; 8,63 (d, 1H) ; 7,47 (s, 1H) ; 7,3 (m, 1H) ; 7,1-6,8 (m, 3H) ; 5,94 (s, 2H).

1.4 N-[(6-Méthylamino)pyridin-3-yl]-5-trifluorométhyl-1-(3-fluorobenzyl)-1*H*-pyrrolo[2,3-*b*]pyridine-2-carboxamide (Composé n°1 du tableau 2)

A une solution, agitée à 20°C, de 0,2 g (0,59 mmole) de composé préparé à l'étape 1.3, 113 mg (0,59 mmole) de chlorhydrate de *N*-(3-diméthylaminopropyl)-*N*'-éthylcarbodiimide (EDAC) et 79 mg (0,59 mmole) de 1-hydroxy-benzotriazole (HOBT) dans 50 mL de DMF, sont ajoutés 109 mg (0,89 mmole) de 2-(méthylamino)-5-aminopyridine (J.Med.Chem. 1994 (37) 18-25). Le mélange réactionnel est agité 14 heures à 20°C puis est versé dans 50 mL d'eau. Le mélange est ensuite extrait par 3 fois 30 mL d'acétate d'éthyle. Les phases organiques rassemblées sont lavées deux fois avec 20 mL d'eau, séchées sur sulfate de sodium puis concentrées sous pression réduite. Le produit obtenu est purifié par chromatographie sur colonne de silice en éluant avec un mélange de dichlorométhane et de méthanol. On isole ainsi 54 mg du produit attendu.
PF = 252 - 254 °C
R.M.N. $^1$H (DMSO $D_6$), δ (ppm) : 10,35 (s, 1 H) ; 8,79 (d, 1H) ; 8,7 (d, 1 H) ; 8,21 (d, 1H) ; 7,65 (dxd, 1 H) ; 7,49 (s, 1 H) ; 7,27 (m, 1 H) ; 7,1-6,85 (m, 3H) ; 6,4 (m, 2H) ; 5,93 (s, 2H) 2,77 (d, 3H).

**Exemple 2 (Composé N°2 du tableau 2)**

Chlorhydrate (1 :1) de *N*-(6-*N*,*N*-diméthylamino-5-méthyl-pyridin-3-yl)-5-trifluorométhyl-1-(3-fluorobenzyl)-1*H*-pyrrolo [2,3-*b*]pyridine-2-carboxamide

2.1 6-*N*,*N*-Diméthylamino-5-méthyl-3-nitropyridine

On introduit dans un tube de schlenk, équipé d'un barreau aimanté, 0,95 g (5,5 mmoles) de 6-chloro-5-méthyl-3-nitropyridine, 5 mL (39,8 mmoles) d'une solution aqueuse de diméthylamine à 40% et 2 mL d'éthanol. Le tube est fermé et le milieu réactionel est agité à 120°C durant 15 heures. Après ce temps, le tube est refroidi, le mélange réactionnel est concentré sous pression réduite puis repris dans 50 mL d'eau. Le mélange résultant est extrait deux fois avec 30 mL de dichlorométhane. Les phases organiques réunies sont lavées avec 50 mL d'une solution aqueuse saturée en chlorure de sodium, séchées sur sulfate de sodium et concentrées sous pression réduite. On obtient ainsi 0,95 g du produit attendu sous la forme d'un solide jaune qui sera utilisé tel quel dans la suite de la synthèse.
R.M.N. $^1H$ ($CDCl_3$), $\delta$ (ppm) : 8,84 (d, 1H) ; 7,98 (d, 1 H) ; 3,1 (s, 6H) ; 2,3 (s, 3H).

2.2 6-*N*,*N*-Diméthylamino-5-méthyl-3-aminopyridine

Une suspension de 0,94 g (5,2 mmoles) du produit obtenu à l'étape 2.1, 0,5 mL (10,4 mmoles) d'hydrazine monohydrate et 0,4 g de Nickel de Raney dans 40 mL d'éthanol est agitée trois heures à 20°C. On élimine l'insoluble par filtration sur un tampon de célite, le filtrat est concentrée sous pression réduite. On obtient ainsi 0,8 g du produit attendu qui sera utilisé tel quel dans la suite de la synthèse.
R.M.N. $^1H$ ($CDCl_3$), $\delta$ (ppm) : 7,61 (d, 1H) ; 6,78 (d, 1H) ; 3,4-3,1 (pic élargi, 2H) ; 2,65 (s, 6H) ; 2,19 (s, 3H).

2.3 Chlorhydrate (1 :1) de N-(6-N,N-diméthylamino-5-méthyl-pyridin-3-yl)-5-trifluorométhyl-1-(3-fluorobenzyl)-1*H*-pyrrolo[2,3-*b*]pyridine-2-carboxamide (compose n°2 du tableau 2)

Le composé N° 2 a été préparé selon un procédé similaire à celui décrit à l'étape 1.4 en faisant réagir 0,17 g (1,11 mmole) de l'amine obtenue à l'étape 2.2 avec 0,25 g (0,74 mmole) de l'acide obtenu à l'étape 1.3. Le produit obtenu est repris dans 5 mL d'acide chlorhydrique 0,1N dans l'isopropanol et 5 mL de dichlorométhane. La solution est concentrée sous pression réduite, on isole ainsi 0,23 g du produit attendu sous la forme d'un chlorhydrate.
PF = 252 - 257 °C ; Chlorhydrate (1 :1)
R.M.N. $^1H$ (DMSO $D_6$), $\delta$ (ppm) : 10,88 (s, 1H) ; 8,8 (s, 1H) ; 8,71 (s, 1H) ; 8,42 (d, 1H) ; 8,02 (d, 1 H) ; 7,63 (s, 1 H) ; 7,28 (m, 1 H) ; 7,1-6,85 (m, 3H) ; 5,93 (s, 2H) ; 2,97 (s, 6H) ; 2,32 (s, 3H).

**Exemple 3 (Composé N°3 du tableau 2)**

Chlorhydrate (1 :1) de *N*-(6-*N*,*N*-diméthylamino-4-méthyl-pyridin-3-yl)-5-trifluorométhyl-1-(3-fluorobenzyl)-1*H*-pyrrolo [2,3-*b*]pyridine-2-carboxamide

Le composé N° 3 a été préparé selon un procédé similaire à celui décrit à l'étape 1.4 en faisant réagir 0,17 g (1,11 mmole) de 6-*N*,*N*-diméthylamino-4-méthyl-3-aminopyridine (W02004062665) avec 0,25 g (0,74 mmole) de l'acide obtenu à l'étape 1.3. Le produit obtenu est repris dans 3,8 mL d'acide chlorhydrique 0,1N dans l'isopropanol et 2 mL de dichlorométhane. Après 15 heures à 20°C, on recueille par filtration le précipité que l'on rince avec 50 mL d'éther éthylique puis on le sèche sous pression réduite. On isole ainsi 0,15 g du produit attendu sous la forme d'un chlorhydrate.
PF = 242 - 247 °C ; Chlorhydrate (1 :1)
R.M.N. $^1H$ (DMSO $D_6$), $\delta$ (ppm): 10,31 (s, 1H) ; 8,8 (s, 1H) ; 8,73 (s, 1H) ; 7,91 (s, 1H) ; 7,6 (s, 1 H) ; 7,3 (m, 1 H) ; 7,1-6,8 (m, 4H) ; 5,91 (s, 2H) ; 3,16 (s, 6H) ; 2,18 (s, 3H).

**Exemple 4 (Composé N°4 du tableau 2)**

Chlorhydrate (1 :1) de *N*-(6-*N*,*N*-diméthylamino-5-trifluorométhyl-pyridin-3-yl)-5-trifluorométhyl-1-(3-fluorobenzyl)-1*H*-pyrrolo[2,3-*b*]pyridine-2-carboxamide Le composé N° 4 a été préparé, selon un procédé similaire à celui décrit à l'étape 1.4, en faisant réagir 0,15 g (0,74 mmole) de 6-*N*,*N*-diméthylamino-5-trifluorométhyl-3-aminopyridine (WO2004110986) avec 0,25 g (0,74 mmole) de l'acide obtenu à l'étape 1.3. Le produit obtenu est repris dans 3,8 mL d'acide chlorhydrique 0,1N dans l'isopropanol et 2 mL de dichlorométhane. La solution est concentrée sous pression réduite, on isole ainsi 0,28 g du produit attendu sous la forme d'un chlorhydrate.
PF = 213 - 218 °C ; Chlorhydrate (1 :1)

R.M.N. $^1H$ (DMSO $D_6$), δ (ppm): 10,81 (s, 1 H) ; 8,75 (m, 3H) ; 8,31 (d, 1 H) ; 7,61 (s, 1H) ; 7,3 (m, 1 H) ; 7,1-6,85 (m, 3H) ; 5,94 (s, 2H) ; 2,88 (s, 6H).

**<u>Exemple 5</u> (Composé N°5 du tableau 2)**

*N*-[6-(Pyrrolidin-1-yl)pyridin-3-yl]-5-fluoro-1-(3-fluorobenzyl)-1*H*-pyrrolo[2,3-b]pyridine-2-carboxamide

5.1 2-Amino-3-iodo-5-fluoropyridine

Dans un bicol de 500 mL, muni d'un agitateur magnétique, sont introduits 5 g (44,6 mmoles) de 2-amino-5-fluoropyridine, 13,9 g (44,6 mmoles) de sulfate d'argent et 400 mL d'éthanol. On ajoute ensuite, par petites portions, 11,31 g (44,6 mmoles) d'iode en poudre. L'agitation est poursuivie à température ambiante pendant 24 heures. On élimine par filtration l'insoluble que l'on lave à l'éthanol et le filtrat est concentré sous pression réduite. Le résidu ainsi obtenu est repris dans un mélange d'acétate d'éthyle (200 mL) et d'une solution de carbonate de sodium (200 mL). Après séparation, la phase organique est lavée successivement avec une solution aqueuse de thiosulfate de sodium à 25% puis avec une solution aqueuse saturée en chlorure de sodium puis séchée sur sulfate de sodium et concentrée sous pression réduite. Le solide résultant est purifié par chromatographie sur colonne de silice en éluant avec un mélange de n-heptane et d'acétate d'éthyle. On obtient 2,67 g (11,22 mmoles) du produit attendu.
R.M.N. $^1H$ (DMSO $D_6$), δ (ppm) : 7,95 (s, 1H) ; 7,85 (s, 1H) ; 5,9 (s, $NH_2$).

5.2 Acide 5-fluoro-1*H*-pyrrolo[2,3-*b*]pyridine-2-carboxylique

Dans un tube scellé de 25 mL, muni d'un agitateur magnétique et maintenu sous bullage d'argon, sont introduits, 0,5 g (2,10 mmoles) de 2-amino-3-iodo-5-fluoropyridine obtenu à l'étape 5.1, 0,55 g (6,3 mmoles) d'acide pyruvique, 0,71 g (6,3 mmoles) de 1,4-diazabicyclo[2.2.2]octane (DABCO) et 15 mL de diméthylformamide anhydre. Après quelques minutes, on ajoute 0,05 g (0,22 mmole) d'acétate de palladium. Le mélange réactionnel est maintenu sous agitation et sous bullage d'argon pendant 20 minutes puis rapidement scellé et porté à 100 °C pendant 2h30. La solution refroidie est concentrée sous pression réduite. Le résidu est ensuite repris par de l'acétate d'éthyle (100 mL) et de l'eau (75 mL). La phase organique est lavée à l'eau puis extraite par deux fois 50 mL d'une solution aqueuse de soude 2N. Les phases aqueuses basiques sont rassemblées, refroidies à 0°C puis acidifiées par ajout d'acide chlorhydrique (pH 3). On extrait le milieu par de l'acétate d'éthyle (4x50 mL), les phases organiques rassemblées sont séchées sur du sulfate de sodium puis concentrées sous pression réduite. On obtient 0,158 g (0,88 mmole) du produit attendu sous forme d'une poudre jaune.
R.M.N. $^1H$ (DMSO $D_6$), δ (ppm) : 13,2 (s, 1 H) ; 12,4 (s, 1 H) ; 8,4 (d, 1 H) ; 7,95 (dd, 1H) ; 7,1 (d, 1 H).

5.3 5-Fluoro-1*H*-pyrrolo[2,3-*b*]pyridine-2-carboxylate d'éthyle

Dans un ballon de 100 mL, muni d'un agitateur magnétique, sont introduits 0,2 g ( 1,11 mmole) d'acide obtenu à l'étape 5.2 et 10 mL d'éthanol. On ajoute 1 mL d'acide sulfurique concentré au mélange réactionnel que l'on porte ensuite à reflux pendant 18 heures. La solution refroidie est concentrée à sec sous pression réduite. Le résidu est repris par de l'acétate d'éthyle (50 mL) que l'on lave successivement avec une solution aqueuse de soude normale (2 x 10 mL), avec de l'eau (10 mL) puis avec une solution aqueuse saturée en chlorure de sodium. On séche la phase organique sur sulfate de sodium puis on la concentre sous pression réduite. On isole 0,21 g du produit attendu. R.M.N. $^1H$ (DMSO $D_6$), δ (ppm): 12,6 (s, NH) ; 8,4 (d, 1 H) ; 8,0 (dd, 1 H) ; 7,1 (d, 1 H) ; 4,35 (q, 2H) ; 1,35 (t, 3H).

5.4 5-Fluoro-1-[(3-fluorophényl)méthyl]-1H-pyrrolo[2,3-b]pyridine-2-carboxylate d'éthyle

A une solution de 0,2 g (0,96 mmole) de produit obtenu à l'étape 5.3, dans 15 mL de tétrahydrofurane sec, maintenue sous atmosphère inerte, sont ajoutés successivement sous agitation, 0,18 g (1,44 mmole) d'alcool 3-fluorobenzylique puis 0,39 g (1,44 mmole) de triphénylphosphine. On ajoute ensuite, goutte à goutte à 0°C, 0,26 g (1,44 mmole) d'azodicarboxylate de diéthyle. Le mélange réactionnel est alors agité pendant 20h à température ambiante puis concentré sous pression réduite. On purifie l'huile résultante par chromatographie sur colonne de gel de silice en éluant avec un mélange de dichlorométhane et de méthanol. On isole 0,26 g (0,82 mmole) du produit attendu.

5.5 Acide 5-fluoro-1-[(3-fluorophényl)méthyl]-1*H*-pyrrolo[2,3-*b*]pyridine-2-carboxylique

Un mélange de 1,3 g (4,11 mmoles) de 5-fluoro-1-[(3-fluorophényl)méthyl]-1*H*-pyrrolo[2,3-*b*]pyridine-2-carboxylate d'éthyle, obtenu à l'étape 5.4, dans 40 mL d'éthanol et de 0,7 g (12,33 mmoles) de potasse dans 2 mL d'eau

est chauffé deux heures à reflux. Après ce temps, le mélange est concentré à sec et repris dans 100 mL d'eau. Le pH de la solution résultante est acidifié par ajouts d'une solution d'acide chlorhydrique concentrée. On recueille par filtration le précipité que l'on rince à l'eau puis on le sèche sous pression réduite. On isole ainsi 1,2 g d'un solide beige qui sera utilisé dans la suite de la synthèse sans purification supplémentaire.
PF = 197 - 198 °C

5.6 *N*-[6-(Pyrrolidin-1-yl)pyridin-3-yl]-5-fluoro-1-(3-fluorobenzyl)-1*H*-pyrrolo[2,3-*b*]pyridine-2-carboxamide (composé n°5 du tableau 2)

Une solution de 0,4 g (1,39 mmole) de l'acide obtenu lors de l'étape 5.5 et de 0.25 g (1,53 mmole) de 1,1'-carbonyldiimidazole dans 22 mL de tétrahydrofurane sec est agitée 3h à 50°C. Après ce temps est ajouté une solution de 0,426 g (1,8 mmole) de chlorhydrate de 3-amino-6-(pyrrolidin-1-yl)pyridine (WO200248152) et de 0,3 mL (2,16 mmoles) de triéthylamine dans 6 mL de tétrahydrofurane. Le mélange réactionnel est agité 15 heures à 30°C puis versé sur 100 mL d'eau. Le mélange résultant est extrait avec trois fois 50 mL d'acétate d'éthyle. Les phases organiques sont réunies, lavées avec 50 mL d'eau, séchées sur sulfate de sodium puis concentrées sous pression réduite. Le solide obtenu est trituré dans 1,5 mL de méthanol puis recueilli par filtration et séché. On obtient ainsi 0,18 g d'un solide blanc.
PF = 239-240 °C

Chlorhydrate (2:3) de *N*-[6-(Pyrrolidin-1-yl)pyridin-3-yl]-5-fluoro-1-(3-fluorobenzyl)-1*H*-pyrrolo[2,3-*b*]pyridine-2-carboxamide

Le composé obtenu est repris dans 3,5 mL d'une solution d'acide chlorhydrique 0,1N dans l'isopropanol. Le solide résultant est filtré puis séché sous pression réduite. On isole ainsi 0,12 g du produit attendu sous la forme d'un chlorhydrate.
PF = 241 - 242 °C ; Chlorhydrate (2 :3)
R.M.N. $^1$H (DMSO $D_6$), $\delta$ (ppm): 10,79 (s, 1H) ; 8,49 (m, 2H) ; 8,15 (m, 2H) ; 7,52 (s, 1H) ; 7,29 (m, 1 H) ; 7,12-6,8 (m, 4H) ; 5,9 (s, 2H) ; 3,51 (m, 4H) ; 1,98 (m, 4H).

**<u>Exemple 6</u> (Composé N°6 du tableau 2)**

*N*-[6-(Pyrrolidin-1-yl)pyridin-3-yl]-5-trifluorométhyl-1-(3-fluorobenzyl)-1*H*-pyrrolo[2,3-*b*]pyridine-2-carboxamide

6.1 1 *N*-(6-Chloropyridin-3-yl)-5-trifluorométhyl-1-(3-fluorobenzyl)-1*H*-pyrrolo[2,3-*b*]pyridine-2-carboxamide

Selon un procédé similaire à celui décrit à l'étape 1.4, en faisant réagir 0,85 g (6,65 mmoles) de 6-chloro-3-aminopyridine avec 1,5 g (4,43 mmoles) de l'acide obtenu à l'étape 1.3, on obtient 1,23 g du produit attendu sous la forme d'une poudre blanche.
PF =212-213°C
R.M.N. $^1$H (DMSO $D_6$), $\delta$ (ppm): 10,9 (s, 1H) ; 8,75 (m, 3H) ; 8,19 (dxd, 1 H) ; 7,61 (s, 1H) ; 7,5 (d, 1 H) ; 7,29 (m, 1 H) ; 7,1-6,8 (m, 3H) ; 5,96 (s, 2H).

6.2 *N*-[6-(Pyrrolidin-1-yl)pyridin-3-yl]-5-trifluorométhyl-1-(3-fluorobenzyl)-1*H*-pyrrolo[2,3-*b*]pyridine-2-carboxamide (composé n°6 du tableau 2).

Une solution de 0,1 g (0,22 mmole) du produit obtenu à l'étape 6.1, de 0,19 mL (0,158 mmole) de pyrrolidine et de 0,5 mL de *N*-méthylpyrrolidinone, placée dans un tube scellé, est chauffée au four micro-onde, 20 mn à 200°C sous 300 W. On ajoute 100 mL d'eau au mélange réactionnel que l'on extrait trois fois avec 50 mL d'acétate d'éthyle. Les phases organiques sont réunies, lavées trois fois avec 20 mL d'eau, séchées sur sulfate de sodium puis concentrées sous pression réduite. Le produit obtenu est ensuite purifié par chromatographie sur colonne de silice en éluant avec un mélange de dichlorométhane et de méthanol puis recristallisé dans un mélange d'acétate d'éthyle et d'heptane. On obtient 60 mg du produit attendu sous la forme de cristaux blancs.
PF : 234 - 236 °C
R.M.N. $^1$H (DMSO $D_6$), $\delta$ (ppm): 10,32 (s, 1H) ; 8,75 (m, 1H) ; 8,66 (m, 1H) ; 8,29 (d, 1H) ; 7,74 (dxd, 1 H) ; 7,5 (s, 1 H) ; 7,29 (m, 1 H) ;7,1-6,8 (m, 3H) ; 6,4 (d, 1H) ; 5,91 (s, 2H) ; 3,32 (m, 4H) ; 1,91 (m, 4H).

**<u>Exemple 7</u> (Composé N°7 du tableau 2)**

*N*-[5-(Pyrrolidin-1-yl)pyrazin-2-yl]-5-trifluorométhyl-1-(3-fluorobenzyl)-1*H*-pyrrolo[2,3-*b*]pyridine-2-carboxamide

7.1 2-Amino-5-(pyrrolidin-1-yl)pyrazine (amine Vb)

Une solution de 0,6 g (3,45 mmoles) de 2-amino-5-bromopyrazine et de 3 mL (36 mmoles) de pyrrolidine, placée dans un tube scellé, est chauffée au four micro-onde, deux heures à 180°C sous 200 W. On ajoute 100 mL d'eau au mélange réactionnel que l'on extrait trois fois avec 50 mL d'acétate d'éthyle. Les phases organiques sont réunies, lavées trois fois avec 20 mL d'eau puis une fois avec 20 mL d'une solution aqueuse saturée en chlorure de sodium, séchées sur sulfate de sodium puis concentrées sous pression réduite. Le produit obtenu est ensuite purifié par chromatographie sur colonne de silice en éluant avec un mélange de dichlorométhane et de méthanol. On obtient 0,21 g du composé attendu sous la forme d'un solide marron.
R.M.N. $^1$H (DMSO $D_6$), $\delta$ (ppm): 7,5 (d, 1H) ; 7,32 (d, 1H) ; 5,15 (s, 2H) ; 3,25 (m, 4H) ; 1,88 (m, 4H).

7.2 *N*-[5-(Pyrrolidin-1-yl)pyrazin-2-yl]-5-trifluorométhyl-1-(3-fluorobenzyl)-1*H*-pyrrolo[2,3-*b*]pyridine-2-carboxamide (composé n°7 du tableau 2)

Le composé N° 7 a été préparé selon un procédé similaire à celui décrit à l'étape 1.4 en faisant réagir 0,126 g (0,77 mmole) de l'amine obtenue à l'étape 7.1 avec 0,2 g (0,59 mmole) de l'acide obtenu à l'étape 1.3. On obtient 96 mg du produit attendu sous la forme d'un solide jaune.
PF = 182 - 183 °C
R.M.N. $^1$H (DMSO $D_6$), $\delta$ (ppm): 10,9 (s, 1 H) ; 8,79 (m, 1 H) ; 8,67 (m, 1 H) ; 8,1 (m, 1H) ; 7,79 (s, 1 H) ; 7,65 (s, 1 H) ; 7,3 (m, 1 H) ; 7,1-6,85 (m, 3H) ; 5,98 (s, 2H) ; 3,41 (m, 4H) ; 1,95 (m, 4H).

**<u>Exemple 8</u> (Composé N°8 du tableau 2)**

*N*-[6-(*N*,*N*-Diméthylamino)pyridazin-3-yl]-5-trifluorométhyl-1-(3-fluorobenzyl)-1*H*-pyrrolo[2,3-*b*]pyridine-2-carboxamide

Le composé N° 8 a été préparé selon un procédé similaire à celui décrit à l'étape 1.4 en faisant réagir 0,098 g (0,71 mmole) de 3-amino-6-(*N*,*N*-diméthylamino)pyridazine (US1977805419) avec 0,2 g (0,59 mmole) de l'acide obtenu à l'étape 1.3. On obtient ainsi 73 mg du produit attendu sous la forme d'un solide.
PF = 224 - 226 °C
R.M.N. $^1$H (DMSO $D_6$), $\delta$ (ppm): 11,3 (s, 1H) ; 8,8 (m, 1H) ; 8,71 (m, 1H) ; 7,89 (d, 1H) ; 7,72 (s, 1H) ; 7,35-6,8 (m, 5H) ; 5,94 (s, 2H) ; 3,05 (s, 6H).

**<u>Exemple 9</u> (Composé N°9 du tableau 2)**

*N*-[6-(pyrrolidin-1-yl)pyridin-3-yl]-5-trifluorométhyl-1-[(4-pyridyl)methyl]-1*H*-pyrrolo[2,3-*b*]pyridine-2-carboxamide

9.1 5-Trifluorométhyl-1-[(4-pyridyl)methyl]-1*H*-pyrrolo[2,3-b]pyridine-2-carboxylate d'éthyle

A une solution de 1 g (3,87 mmoles) de produit obtenu à l'étape 1.1, dans 20 mL de tétrahydrofurane sec, maintenue sous atmosphère inerte, sont ajoutés successivement sous agitation, 0,63 g (5,81 mmoles) de (pyridin-4-yl) méthanol puis 1,52 g (5,81 mmoles) de triphénylphosphine. On ajoute ensuite, goutte à goutte, 1,01 g (5,81 mmoles) d'azodicarboxylate de diéthyle. Le mélange réactionnel est alors agité pendant 20 h à température ambiante puis concentré sous pression réduite. On purifie l'huile résultante par chromatographie sur colonne de gel de silice en éluant avec un mélange d'heptane et d'acétate d'éthyle. On isole 1,23 g du produit attendu sous la forme d'un solide blanc.
R.M.N. $^1$H (DMSO $D_6$), $\delta$ (ppm): 8,8 (d, 1 H) ; 8,7 (d, 1H) ; 8,45 (d, 2H) ; 7,55 (s, 1 H) ; 6,95 (d, 2H) ; 5,9 (s, 2H) ; 4,3 (q, 2H) ; 1,2 (t, 3H).

9.2 Acide 5-trifluorométhyl-1-[(4-pyridyl)methyl]-1*H*-pyrrolo[2,3-*b*]pyridine-2-carboxylique

Une solution, dans 30 mL d'éthanol, de 0,8 g (2,29 mmoles) de 5-trifluorométhyl-1-[(4-pyridyl)methyl]-1*H*-pyrrolo [2,3-*b*]pyridine-2-carboxylate d'éthyle, obtenu à l'étape 9.1, est ajoutée une solution de 0,39 g (6,87 mmoles) de potasse dans 2 mL d'eau. Le mélange est agité quatre heures à reflux. Après ce temps le mélange réactionnel est concentré sous pression réduite puis repris dans 40 mL d'eau. Le milieu est acidifié à pH 5 par ajouts successifs d'acide chlorhydrique concentré. On recueille par filtration le précipité , on le lave à l'eau puis on le sèche sous pression réduite. On isole ainsi

0,54 g du produit attendu, sous la forme d'une poudre blanche, qui est utilisé tel quel dans l'étape suivante.
PF = 302 - 303 °C

9.3 *N*-[6-(Pyrrolidin-1-yl)pyridin-3-yl]-]-5-trifluorométhyl-1-[(4-pyridyl)methyl]-1*H*-pyrrolo[2,3-*b*]pyridine-2-carboxamide (composé n°9 du tableau 2)

Le composé N° 9 a été préparé selon un procédé similaire à celui décrit à l'étape 5.6 en faisant réagir 0,3 g (0,93 mmole) de l'acide obtenu à l'étape 9.2 avec 0,286 g (1,21 mmole) du chlorhydrate de 3-amino-6-(pyrrolidin-1-yl) pyridine (WO200248152), préalablement solubilisé dans un mélange de 0,2 mL de triéthylamine et 5 mL de tétrahydrofurane avec. On isole ainsi 30 mg du produit attendu sous la forme d'un solide jaune.
PF = 244 - 245 °C
R.M.N. $^1$H (DMSO $D_6$), $\delta$ (ppm) : 10,39 (s, 1H) ; 8,71 (dxd, 2H) ; 8,41 (dxd, 2H) ; 8,28 (d, 1H) ; 7,73 (dxd, 1H) ; 7,59 (s, 1H) ; 7 (d, 2H) ; 6, 41 (d, 1H) ; 5,97 (s, 2H) ; 3,31 (m, 4H) ; 1,9 (m, 4H).

**<u>Exemple 10</u> (Composé N°16 du tableau 3)**

*N*-[6-(Pyrrolidin-1-yl)pyridin-3-yl]-5-trifluorométhyl-1-(3-fluorobenzyl)-1*H*-pyrrolo[3,2-*b*]pyridine-2-carboxamide

10.1 3-amino-2-iodo-6-(trifluorométhyl)pyridine

On ajoute, en plusieurs portions, 1,56 g (6,17 mmoles) d'iode à un mélange, agité sous argon à 20°C, de 1 g (6,17 mmoles) de 3-amino-6-trifluorométhylpyridine et de 1,25 g (6,17 mmoles) de sulfate d'argent dans 40 mL d'éthanol. L'agitation est maintenue pendant 18 heures. On élimine par filtration l'insoluble que l'on lave à l'éthanol, le filtrat est concentré sous pression réduite, le résidu est repris dans 100 mL de dichlorométhane. La phase organique est lavée successivement avec 20 mL d'une solution aqueuse de soude à 5%, 40 mL d'eau et 20 mL de solution aqueuse saturée de chlorure de sodium, séchée sur sulfate de sodium, concentrée sous pression réduite puis purifiée par chromatographie sur colonne de silice (éluants : heptane - acétate d'éthyle). On isole ainsi 1,17 g du produit attendu que l'on engage tel quel dans la suite de la synthèse.

10.2 Acide (5-trifluorométhyl)pyrrolo[3,2-*b*]pyridine-2-carboxylique

On introduit sous argon, dans un tube scellé, 0,5 g (1,74 mmole) de 3-amino-2-iodo-6-(trifluorométhyl)pyridine, obtenu à l'étape 10.1, 0,45 g (5,21 mmoles) d'acide pyruvique, 0,51 mL (5,21 mmoles) de 1,4-diazabicyclo[2.2.2]octane et 10 mL de diméthylformamide sec. La solution est dégazée quelques minutes puis on ajoute 0,19 g (0,87 mmole) d'acétate de palladium, on ferme le tube et on porte au reflux à 130°C durant 4 heures. La solution refroidie est ensuite concentrée sous pression réduite et le résidu est repris par 100 mL d'acétate d'éthyle. La phase organique est extraite successivement avec deux fois 50 mL d'une solution aqueuse de soude 2N. Les phases aqueuses basiques sont réunies, refroidies à 0°C, acidifiées par ajouts d'acide chlorhydrique puis extraites par 4 fois 50 mL d'acétate d'éthyle. Ces phases organiques sont rassemblées, lavées avec 20 mL d'une solution aqueuse saturée en chlorure de sodium, séchées sur sulfate de sodium puis concentrées sous pression réduite. On obtient 0,22 g de produit que l'on utilise tel quel dans l'étape suivante.

10.3 5-(Trifluorométhyl)pyrrolo[3,2-*b*]pyridine-2-carboxylate d'éthyle

On ajoute 1 mL (18,71 mmoles) d'acide sulfurique concentré à une solution de 0,2 g (0,87 mmole) d'acide 5-trifluorométhyl-pyrrolo[3,2-*b*]pyridine-2-carboxylique, obtenu à l'étape 10.2, dans 10 mL d'éthanol. On agite à reflux durant 20 heures puis on refroidit la solution quel l'on concentre sous pression réduite. Le résidu résultant est ensuite repris avec 50 mL de dichlorométhane puis lavé successivement avec 20 mL d'une solution aqueuse saturée d'hydrogénocarbonate de sodium, 40 mL d'eau et 20 mL de solution aqueuse saturée de chlorure de sodium, séchée sur sulfate de sodium puis concentrée sous pression réduite. On obtient 0,19 g de produit que l'on utilise tel quel dans l'étape suivante.

10.4 1-(3-Fluorobenzyl)-5-(trifluorométhyl)pyrrolo[3,2-*b*]pyridine-2-carboxylate d'éthyle

A une solution de 0,2 g (0,77 mL) de 5-(trifluorométhyl)pyrrolo[3,2-*b*]pyridine-2-carboxylate d'éthyle, obtenu à l'étape 10.3, dans 120 mL de tétrahydrofurane sec, maintenue à 0°C sous argon, sont ajoutés successivement 0,13 mL (1,16 mmole) d'alcool 3-fluorobenzylique, 0,3 g (1,16 mmole) de triphénylphosphine puis 0,2 g (1,16 mmole) d'azodicarboxylate de diéthyle. Le mélange réactionnel est agité durant 20 heures à 20°C puis concentré sous pression réduite. Le résidu résultant est purifié par chromatographie sur colonne de silice (éluants : heptane - acétate d'éthyle). On isole

ainsi 0,21 g du produit attendu sous la forme d'une huile jaune.

10.5 Acide 1-(3-fluorobenzyl)-5-trifluorométhyl-pyrrolo[3,2-*b*]pyridine-2-carboxylique

Le composé a été préparé selon un procédé similaire à celui décrit à l'étape 9.2 en faisant réagir 0,6 g (1,64 mmole) de l'ester obtenu à l'étape 10.4 avec 0,275 g (4.91 mmoles) de potasse. On obtient 0,47 g du produit attendu sous la forme d'un solide blanc.
PF = 254 - 255 °C
LCMS : $[MH]^+$ = 339 ; pureté 98%

10.6 *N*-[6-(Pyrrolidin-1-yl)pyridin-3-yl]-5-trifluorométhyl-1-(3-fluorobenzyl)-1*H*-pyrrolo[3,2-*b*]pyridine-2-carboxamide (composé n°16 du tableau 3)

Le composé N° 10 a été préparé selon un procédé similaire à celui décrit à l'étape 1.4 en faisant réagir 0,25 g (0,74 mmole) de l'acide obtenu à l'étape 10.5 avec 0,2 g (0,89 mmole) du chlorhydrate de 3-amino-6-(pyrrolidin-1-yl) pyridine (WO200248152), préalablement solubilisé dans un mélange de 0,13 mL de triéthylamine et 3 mL de diméthylformamide. On isole ainsi 40 mg du produit attendu sous la forme d'un solide jaune.
PF = 219-220 °C
R.M.N. $^1H$ (DMSO $D_6$), $\delta$ (ppm) : 10,4 (s, 1H) ; 8,3 (m, 2H) ; 7,74 (m, 2H) ; 7,56 (s, 1H) ; 7,3 (m, 1H) ; 7,1-6,82 (m, 3H) ; 6, 41 (d, 1 H) ; 5,91 (s, 2H) ; 3,37 (m, 4H) ; 1,91 (m, 4H).

**<u>Exemple 11</u> (Composé N°10 du tableau 2)**

*N*-[6-(Isopropylamino)pyridin-3-yl]-5-trifluorométhyl-1-(3-fluorobenzyl)-1*H*-pyrrolo[2,3-*b*]pyridine-2-carboxamide.

Selon un procédé similaire à celui décrit à l'étape 6.2, en faisant réagir 0,15 g (0,33 mmole) de *N*-(6-chloropyridin-3-yl)-5-trifluorométhyl-1-(3-fluorobenzyl)-1*H*-pyrrolo[2,3-*b*]pyridine-2-carboxamide, obtenu à l'étape 6.1, avec 0,29 mL (3,34 mmoles) d'isopropylamine, on obtient 50 mg du produit attendu sous la forme d'une poudre jaune clair.
PF = 210 - 212 °C
R.M.N. $^1H$ (DMSO $D_6$), $\delta$ (ppm): 10,32 (s, 1 H) ; 8,79 (d, 1 H) ; 8,71 (d, 1 H) ; 8,2 (m, 1 H) ; 7,61 (dxd, 1 H) ; 7,51 (s, 1H) ; 7,31 (m, 1H) ;7,12-6,86 (m, 3H) ; 6,42 (d, 1 H) ; 6,25 (d, 1 H) ; 5,95 (s, 2H) ; 3,91 (sext., 1 H) ; 1,12 (d, 6H).

**<u>Exemple 12</u> (Composé N°15 du tableau 2)**

Chlorhydrate (1 :1) du *N*-[6-(azétidin-1-yl)pyridin-3-yl]-5-trifluorométhyl-1-(3-fluorobenzyl)-1*H*-pyrrolo[2,3-*b*]pyridine-2-carboxamide.

12.1 2-(azétidin-1-yl)-5-nitropyridine

On ajoute, goutte à goutte, 4,7 g (80,76 mmoles) d'azétidine sur une suspension agitée à 20°C de 27,9 g (0,201 mole) de carbonate de potassium et de 11 g (67,3 mmoles) de 2-chloro-5-nitropyridine dans 100 mL de diméthylformamide. Le mélange est agité 5 minutes à 20°C puis 6 heures à 70°C. Après ce temps, la suspension est versée sur un mélange de 300 mL d'eau et 300 mL d'acétate d'éthyle. La phase aqueuse est séparée puis extraite avec 200 mL d'acétate d'éthyle. Les phases organiques sont réunies, lavées trois fois avec 250 mL d'eau puis séchées sur sulfate de sodium et concentrées sous pression réduite. On obtient ainsi 11,7 g du produit attendu sous la forme d'un solide.
PF=132-134 °C
R.M.N. $^1H$ ($CDCl_3$), $\delta$ (ppm) : 8,92 (d, 1 H) ; 8,09 (dxd, 1 H) ; 6,07 (d, 1 H) ; 4,12 (m, 4H) ; 2,46 (quint., 2H).

12.2 3-amino-6-(azétidin-1-yl)-pyridine (Amine Vc)

Une suspension de 11,5 g (64,18 mmoles) de 2-(azétidin-1-yl)-5-nitropyridine, préparée à l'étape précédente, et de 1 g de Pd/C à 10% dans 400 mL d'éthanol est agitée vigoureusement à 20°C sous 5 atmosphère d'hydrogène durant 4 heures. Après ce temps, le mélange est filtré sur un tampon de célite puis concentré sous pression réduite. On obtient ainsi 9,3 g du produit attendu qui est utilisé tel quel dans la suite de la synthèse.
R.M.N. $^1H$ (DMSO $D_6$), $\delta$ (ppm) : 7,57 (d, 1 H) ; 6,92 (dxd, 1 H) ; 6,2 (d, 1 H) ; 4,46 (pic élargi, 2H) ; 3,78 (m, 4H) ; 2,25 (quint., 2H).

12.3 *N*-[6-(azétidin-1-yl)pyridin-3-yl]-5-trifluorométhyl-1-(3-fluorobenzyl)-1*H*-pyrrolo[2,3-*b*]pyridine-2-carboxamide (Composé n°15 du tableau n°2).

On ajoute, goutte à goutte, 4,26 g (24,83 mmoles) de cyanophosphonate de diéthyle sur une solution, agitée à 20°C sous atmosphère d'azote, de 7 g (20,69 mmoles) d'acide 5-trifluorométhyl-1-[(3-fluorophényl)méthyl]-1*H*-pyrrolo [2,3-*b*]pyridine-2-carboxylique tel qu'obtenu à l'étape 1.3 et 3,7 g (24,83 mmoles) de 3-amino-6-(azétidin-1-yl)-pyridine préparée à l'étape précédente dans 140 mL de diméthylformamide. On ajoute ensuite, goutte à goutte, 4,6 mL (45,53 mmoles) de triéthylamine puis on agite le mélange réactionnel 70 heures à 20°C. Après ce temps le mélange est concentré sous pression réduite puis repris avec 700 mL d'acétate d'éthyle et 300 mL d'eau. La phase aqueuse est séparée puis extraite avec 200 mL d'acétate d'éthyle. Les phases organiques sont réunies, lavées trois fois avec 200 mL d'une solution saturée en hydrogénocarbonate de sodium puis séchées sur sulfate de sodium et concentrées sous pression réduite. Le produit ainsi obtenu est purifié sur colonnne de silice, en éluant avec un mélange de dichorométhane et d'acétate d'éthyle, puis recristallisé dans un mélange d'éthanol et de méthanol. On isole ainsi 5,7 g du produit attendu sous la forme de cristaux blancs
PF = 243 - 244 °C

12.4 Chlorhydrate (1 :1) du *N*-[6-(azétidin-1-yl)pyridin-3-yl]-5-trifluorométhyl-1-(3-fluorobenzyl)-1*H*-pyrrolo [2,3-*b*]pyridine-2-carboxamide (Composé n°15 du tableau n°2). On ajoute 2,6 mL d'une solution 2M d'acide chlorhydrique sur une solution de 1,2 g de *N*-[6-(azétidin-1-yl)pyridin-3-yl]-5-trifluorométhyl-1-(3-fluorobenzyl)-1*H*-pyrrolo[2,3-*b*]pyridine-2-carboxamide, préparé à l'étape précédente, dans 50 mL de dichlorométhane. La solution est concentrée à sec et le solide résultant est recristallisé dans l'éthanol. On isole ainsi 0,62 g du sel attendu.
PF = 230 - 235 °C
R.M.N. $^1$H (DMSO $D_6$), $\delta$ (ppm) : 10,98 (s, 1H) ; 8,87 (s, 1H) ; 8,79 (s, 1H) ; 8,47 (s, 1H) ; 8,2 (dxd, 1H) ; 7,71 (s, 1 H) ; 7,31 (m, 1 H) ; 7,08 (m, 1 H) ; 6,89 (m, 3H) ; 5,98 (s, 2H) ; 4,25 (m, 4H) ; 2,42 (quint., 2H).

**<u>Exemple 13</u> (Composé N°19 du tableau 2)**

*N*-[6-(3,3-difluoroazétidin-1-yl)pyridin-3-yl]-5-trifluorométhyl-1-(3-fluorobenzyl)-1*H*-pyrrolo[2,3-*b*]pyridine-2-carboxamide.

13.1 2-(3,3'-difluoroazétidin-1-yl)-5-nitropyridine

On chauffe la nuit à 100°C un mélange de 0,367 g (2,84 mmoles) de chlorhydrate de 3,3-difluoroazétidine, de 0,3 g (1,89 mmole) de 2-chloro-5-nitropyridine et de 0,41 mL (2,84 mmoles) de triéthylamine dans 10 mL de diméthylformamide. Après ce temps, la suspension est versée sur un mélange de 30 mL d'eau et 30 mL d'acétate d'éthyle. La phase aqueuse est séparée puis extraite avec 20 mL d'acétate d'éthyle. Les phases organiques sont réunies, lavées trois fois avec 25 mL d'eau puis séchées sur sulfate de sodium et concentrées sous pression réduite. Le produit résultant est purifié par chromatographie sur colonne de silice en éluant avec du dichlorométhane. On obtient ainsi 0,34 g du produit attendu sous la forme d'un solide jaune.
R.M.N. $^1$H (DMSO $D_6$), $\delta$ (ppm): 9 (d, 1 H) ; 8,32 (dxd, 1 H) ; 6,61 (d, 1 H) ; 4,6 (m, 4H).

13.2 3-amino-6-(3,3-difluoroazétidin -1-yl)pyridine (Amine Vh)

Une suspension de 0,34 g (1,58 mmole) de 2-(3,3-difluoroazétidin-1-yl)-5-nitropyridine, préparée à l'étape précédente, et de 8 mg de Pd/C à 10% dans 10 mL d'éthanol est agitée vigoureusement à 20°C sous 5 atmosphère d'hydrogène durant 4 heures. Après ce temps, le mélange est filtré sur un tampon de célite puis concentré sous pression réduite. Le produit résultant est purifié par chromatographie sur colonne de silice en éluant avec un mélange de dichlorométhane et de méthanol. On obtient ainsi 0,188 g du produit attendu sous la forme d'une poudre rouge utilisée telle quelle dans la suite de la synthèse.
R.M.N. $^1$H (DMSO $D_6$), $\delta$ (ppm) : 7,6 (s, 1H) ; 6,96 (dxd, 1 H) ; 6,4 (d, 1 H) ; 4,67 (s, 2H) ; 4,2 (t, 4H).

13.3 *N*-[6-(3,3-difluoroazétidin-1-yl)pyridin-3-yl]-5-trifluorométhyl-1-(3-fluorobenzyl)-1*H*-pyrrolo[2,3-*b*]pyridine-2-carboxamide (compose n° 19 du tableau n°2)

Le composé N° 19 a été préparé selon un procédé similaire à celui décrit à l'étape 1.4 en faisant réagir 0,188 g (0,02 mmole) de 3-amino-6-(3,3-difluoroazétidin-1-yl)pyridine, préparée à l'étape précédente, avec 0,23 g (0,68 mmole) de l'acide obtenu à l'étape 1.3. On obtient ainsi 140 mg du produit attendu sous la forme d'un solide.
PF = 222 - 224 °C
R.M.N. $^1$H (DMSO $D_6$), $\delta$ (ppm): 10,6 (s, 1 H) ; 8,84 (s, 1 H) ; 8,74 (s, 1 H) ; 8,44 (s, 1H) ; 7,98 (d, 1 H) ; 7,6 (s, 1 H) ;

7,32 (m, 1 H) ; 7,08 (txd, 1 H) ; 6,92 (m, 2H) ; 6,64 (d, 1 H) ; 6 (s, 2H) ; 4,39 (m, 4H).

**<u>Exemple 14</u> (Composé N°20 du tableau 2)**

*N*-[6-(3-hydroxyazétidin-1-yl)pyridin-3-yl]-5-trifluorométhyl-1-(3-fluorobenzyl)-1*H*-pyrrolo[2,3-*b*]pyridine-2-carboxamide.

14.1 2-(3-hydroxyazétidin-1-yl)-5-nitropyridine

On chauffe la nuit à 100°C un mélange de 0,311 g (2,84 mmoles) de chlorhydrate de 3-hydroxyazétidine, de 0,3 g (1,89 mmole) de 2-chloro-5-nitropyridine et de 0,41 mL (2,84 mmoles) de triéthylamine dans 10 mL de diméthylformamide. Après ce temps, la suspension est versée sur un mélange de 30 mL d'eau et 30 mL d'acétate d'éthyle. La phase aqueuse est séparée puis extraite avec 20 mL d'acétate d'éthyle. Les phases organiques sont réunies, lavées trois fois avec 25 mL d'eau puis séchées sur sulfate de sodium et concentrées sous pression réduite. On obtient ainsi 0,34 g du produit attendu sous la forme d'un solide qui sera utilisé tel quel dans la suite de la synthèse.
R.M.N. $^1$H (DMSO $D_6$), δ (ppm): 8,95 (s, 1 H) ; 8,21 (dxd, 1 H) ; 6,41 (d, 1 H) ; 5,85 (d, 1H) ; 4,62 (m, 1 H) ; 4,37 (m, 2H) ; 3,9 (m, 2H).

14.2 3-amino-6-(3-hydroxyazétidin-1-yl)pyridine (Amine Ve)

Une suspension de 0,34 g (1,74 mmole) de 2-(3-hydroxyazétidin-1-yl)-5-nitropyridine, préparée à l'étape précédente, et de 9 mg de Pd/C à 10% dans 10 mL d'éthanol est agitée vigoureusement à 20°C sous 5 atmosphère d'hydrogène durant 4 heures. Après ce temps, le mélange est filtré sur un tampon de célite puis concentré sous pression réduite. Le produit résultant est purifié par chromatographie sur colonne de silice en éluant avec un mélange de dichlorométhane et de méthanol. On obtient ainsi 0,1 g du produit attendu qui sera utilisé tel quel dans la suite de la synthèse.
R.M.N. $^1$H (DMSO $D_6$), δ (ppm) : 7,58 (s, 1H) ; 6,91 (d, 1H) ; 6,22 (d, 1H) ; 5,48 (pic élargi, 1H) ; 4,48 (pic élargi, 3H) ; 3,98 (m, 2H) ; 3,49 (m, 2H).

14.3 *N*-[6-(3-hydroxyazétidin-1-yl)pyridin-3-yl]-5-trifluorométhyl-1-(3-fluorobenzyl)-1*H*-pyrrolo[2,3-*b*]pyridine-2-carboxamide (compose n° 20 du tableau n°2)

Le composé N° 20 a été préparé selon un procédé similaire à celui décrit à l'étape 1.4 en faisant réagir 99 mg (0,6 mmole) de 3-amino-6-(3-hydroxyazétidin-1-yl)-pyridine, préparée à l'étape précédente, avec 0,135 g (0,4 mmole) de l'acide obtenu à l'étape 1.3. On obtient ainsi 103 mg du produit attendu sous la forme d'un solide.
PF = 221 - 223 °C
R.M.N. $^1$H (DMSO $D_6$), δ (ppm): 10,51 (s, 1H) ; 8,87 (s, 1H) ; 8,78 (s, 1H) ; 8,36 (s, 1H) ; 7,87 (d, 1 H) ; 7,59 (s, 1 H) ; 7,35 (m, 1 H) ; 7,09 (m, 1 H) ; 6,95 (m, 2H) ; 6,45 (d, 1 H) ; 6 (s, 2H) ; 5,61 (s, 1 H) ; 4,59 (m, 1 H) ; 4,15 (m, 2H) ; 3,68 (m, 2H).

**<u>Exemple 15</u> (Composé N°22 du tableau 2)**

*N*-[4-méthyl-6-(pyrrolidin-1-yl)pyridin-3-yl]-6-trifluorométhyl-1-(3-fluorobenzyl)-1*H*-pyrrolo[2,3-*b*]pyridine-2-carboxamide.

15.1 1 6-trifluorométhyl-1-[(3-fluorophényl)méthyl]-1*H*-pyrrolo[2,3-b]pyridine-2-carboxylate de méthyle

Selon un procédé similaire à celui décrit à l'étape 1.2, en faisant réagir 1,5 g (6,14 mmoles) de 6-trifluorométhyl-1*H*-pyrrolo[2,3-*b*]pyridine-2-carboxylate de méthyle et 1 mL (9,21 mmoles) d'alcool 3-fluorobenzylique, on obtient 2,1 g du composé attendu.
R.M.N. $^1$H ($CDCl_3$), δ (ppm): 8,3 (d, 1H) ; 7,69 (d, 1H) ; 7,48 (s, 1H) ; 7,4-7,19 (m, 2H) ; 7,08 (m, 2H) ; 6,11 (s, 2H) ; 4,05 (s, 3H).

15.2 Acide 6-trifluorométhyl-1-[(3-fluorophényl)méthyl]-1*H*-pyrrolo[2,3-*b*]pyridine-2-carboxylique

Selon un procédé similaire à celui décrit à l'étape 1.3, à partir de 2,1 g (5,96 mmoles) de 6-trifluorométhyl-1-[(3-fluorophényl)méthyl]-1*H*-pyrrolo[2,3-*b*]pyridine-2-carboxylate de méthyle, préparé à l'étape précédente, on obtient 2 g du composé attendu.
R.M.N. $^1$H (DMSO $D_6$), δ (ppm): 8,39 (d, 1 H) ; 7,64 (d, 1 H) ; 7,32 (m, 1 H) ; 7,24 (s, 1H) ; 7,05 (m, 3H) ; 6,02 (s, 2H).

15.3 *N*-[4-méthyl-6-(pyrrolidin-1-yl)pyridin-3-yl]-6-trifluorométhyl-1-(3-fluorobenzyl)-1*H*-pyrrolo[2,3-*b*]pyridine-2-carboxamide (compose n°22 du tableau n°2)

Le composé N° 22 a été préparé selon un procédé similaire à celui décrit à l'étape 1.4 en faisant réagir 0,314 g (1,77 mmole) de 3-amino-4-méthyl-6-(pyrrolidin-1-yl)pyridine (WO05/035526) avec 0,5 g (0,1,48 mmole) de l'acide obtenu à l'étape précédente. On obtient ainsi 0,24 g du produit attendu sous la forme d'un solide.
PF = 202 - 203 °C
R.M.N. $^1$H (DMSO $D_6$), δ (ppm): 10,09 (s, 1 H) ; 8,5 (d, 1 H) ; 7,87 (s, 1 H) ; 7,72 (d, 1H) ; 7,5 (s, 1 H) ; 7,32 (m, 1 H) ; 7,08 (m, 1 H) ; 6,95 (m, 2H) ; 6,38 (s, 1 H) ; 5,94 (s, 2H) ; 3,39 (m, 4H) ; 2,06 (s, 3H) ; 1,95 (m, 4H).

**<u>Exemple 16</u> (Composé N°33 du tableau 2)**

*N*-[6-(pyrrolidin-1-yl)pyrid in-3-yl]-6-trifluorométhyl-1-(3-fluorobenzyl)-1*H*-pyrrolo[2,3-*b*]pyridine-2-carboxamide.
16.1 *N*-[6-chloropyridin-3-yl]-6-trifluorométhyl-1-(3-fluorobenzyl)-1*H*-pyrrolo[2,3-*b*]pyridine-2-carboxamide.
Selon un procédé similaire à celui décrit à l'étape 6.1, en faisant réagir 0,5 g (1,48 mmole) d'acide 6-trifluorométhyl-1-[(3-fluorophényl)méthyl]-1*H*-pyrrolo[2,3-*b*]pyridine-2-carboxylique, décrit à l'étape 15.2 avec 0,288 (2,22 mmoles) de 3-amino-6-chloropyridine, on obtient 0,33 g du composé attendu sous la forme d'un solide blanc.
PF = 202 - 203 °C
R.M.N. $^1$H (DMSO $D_6$), δ (ppm): 10,95 (s, 1H) ; 8,75 (d, 1H) ; 8,58 (d, 1H) ; 8,22 (d, 1H) ; 7,76 (d, 1 H) ; 7,61 (s, 1 H) ; 7,53 (d, 1 H) ; 7,32 (m, 1 H) ; 7,05 (m, 1 H) ; 6,98 (m, 2H) ; 5,94 (s, 2H).

16.2 *N*-[6-(pyrrolidin-1-yl)pyridin-3-yl]-6-trifluorométhyl-1-(3-fluorobenzyl)-1*H*-pyrrolo[2,3-*b*]pyridine-2-carboxamide (Produit n°33 du tableau n°2)

Selon un procédé similaire à celui décrit à l'étape 6.2, en faisant réagir 0,3 g (0,67 mmole) de *N*-[6-chloropyridin-3-yl]-6-trifluorométhyl-1-(3-fluorobenzyl)-1*H*-pyrrolo[2,3-*b*]pyridine-2-carboxamide, décrit à l'étape précédente, avec 0,56 mL (6,68 mmoles) de pyrrolidine, on obtient 0,21 g du composé attendu.
PF = 204 - 205°C
R.M.N. $^1$H (DMSO $D_6$), δ (ppm): 10,5 (s, 1H) ; 8,58 (d, 1H) ; 8,39 (d, 1H) ; 7,87 (dxd, 1H) ; 7,79 (d, 1H) ; 7,59 (s, 1 H) ; 7,4 (m, 1 H) ; 7,12 (m, 1 H) ; 7,07 (m, 2H) ; 6,53 (d, 1 H) ; 6,01 (s, 2H) ; 3,46 (m, 4H) ; 2 (m, 4H).

**<u>Exemple 17</u> (Composé N°34 du tableau 2)**

*N*-[6-(pyrrolidin-1-yl)-4-trifluorométhylpyridin-3-yl]-5-trifluorométhyl-1-(3-fluorobenzyl)-1*H*-pyrrolo[2,3-*b*]pyridine-2-carboxamide.

17.1 6-(pyrrolidin-1-yl)-4-(trifluorométhyl)pyridine-3-carboxylate de méthyle

On chauffe à 100°C, pendant 3 heures, un mélange de 2,5 g (10,43 mmoles) de 6-chloro-4-trifluorométhyl-nicotinate de méthyle, de 2,88 g (20,87 mmoles) de carbonate de potassium et de 2,61 mL (31,3 mmoles) de pyrrolidine dans 90 mL de diméthylformamide. Le mélange réactionnel est ensuite concentré sous pression réduite puis repris dans 100 mL d'eau. On recueille, par filtration, un précipité que l'on lave avec 150 mL d'eau. On isole, après séchage sous pression réduite, 2,5 g du composé attendu. R.M.N. $^1$H ($CDCl_3$), δ (ppm): 2,11 (m, 4H) ; 3,61 (pic élargi, 4H) ; 3,93 (s, 3H) ; 6,69 (s, 1 H) ; 8,89 (s, 1 H).

17.2 Acide 6-(pyrrolidin-1-yl)-4-(trifluorométhyl)pyridine-3-carboxylique

On agite pendant 24 heures à 20°C un mélange de 2,5 g (9,12 mmoles) de 6-(pyrrolidin-1-yl)-4-(trifluorométhyl) pyridine-3-carboxylate de méthyle, obtenu à l'étape précédente, et de 0,76 g (13,67 mmoles) de potasse dans 50 mL de méthanol et 2 mL d'eau. Le mélange est ensuite concentré sous pression réduite. On ajoute ensuite 100 mL d'eau et la solution est lavée avec 100 mL de dichlorométhane puis acidifiée à pH 4 par ajouts d'acide chlorhydrique concentré. On recueille un précipité par filtration que l'on lave avec 50 mL d'eau. On isole, après séchage sous pression réduite, 2,2 g du composé attendu. R.M.N. $^1$H (DMSO $D_6$), δ (ppm): 1,99 (s, 4H) ; 3,51 (pic élargi, 4H) ; 6,71 (s, 1H) ; 8,72 (s, 1 H).

17.3 6-(pyrrolidin-1-yl)-4-trifluorométhyl-3-(terbutoxycarbonylamino)pyridine

On chauffe pendant 5 heures à 90°C un mélange de 2,2 g (8,45 mmoles) d'acide 6-(pyrrolidin-1-yl)-4-(trifluorométhyl)pyridine-3-carboxylique, obtenu à l'étape précédente, de 2,37 mL (10,99 mmoles) de diphénylphosphorylazide et de 2,95 mL (21,14 mmoles) de triéthylamine dans 25 mL de terbutanol. Le mélange réactionnel est ensuite concentré sous pression réduite, repris avec 50 mL d'eau puis extrait 3 fois avec 50 mL de dichlorométhane. Les phases organiques sont réunies, lavées avec 50 ml d'eau, séchées sur sulfate de sodium puis concentrées sous pression réduite. L'huile obtenue est purifiée par chromatographie sur colonne de silice en éluant avec un mélange de dichlorométhane et de méthanol. On isole ainsi 1,05 g du produit attendu.
R.M.N. $^1H$ ($CDCl_3$), $\delta$ (ppm): 1,53 (s, 9H) ; 2,09 (m, 4H) ; 3,51 (m, 4H) ; 6,2 (pic élargi, 1 H) ; 6,52 (s, 1 H) ; 8,39 (pic élargi, 1 H).

17.4 Chlorhydrate de 6-(pyrrolidin-1-yl)-4-trifluorométhyl-3-aminopyridine (Amine Vg)

On agite pendant 5 heures à reflux une solution de 1 g (3,02 mmoles) de 6-(pyrrolidin-1-yl)-4-trifluorométhyl-3-(terbutoxycarbonylamino)pyridine, préparée à l'étape précédente, dans 11 mL d'acide chlorhydrique 4N dans le dioxane. On ajoute ensuite 200 mL d'éther éthylique au mélange réactionnel refroidi. On recueille 0,8 g d'un précipité par filtration. Point de fusion : 207 - 209 °C ;
R.M.N. $^1H$ (DMSO $D_6$), $\delta$ (ppm): 2 (m, 4H) ; 3,52 (m, 4H) ; 7,1 (s, 1H) ; 7,49 (pic élargi, 2H) ; 7,92 (s, 1 H).

17.5 N-[6-(pyrrolidin-1-yl)-4-trifluorométhylpyridin-3-yl]-5-trifluorométhyl-1-(3-fluorobenzyl)-1*H*-pyrrolo[2,3-*b*]pyridine-2-carboxamide (composé n° 34 du tableau).

Le composé N° 34 a été préparé selon un procédé similaire à celui décrit à l'étape 1.4 en faisant réagir 0,411 g (1,54 mmole) de chlorhydrate de 6-(pyrrolidin-1-yl)-4-trifluorométhyl-3-aminopyridine, obtenu à l'étape précédente, avec 0,4 g (0,18 mmole) de l'acide 5-trifluorométhyl-1-[(3-fluorophényl)méthyl]-1*H*-pyrrolo[2,3-*b*]pyridine-2-carboxylique obtenu à l'étape 1.3, le tout en présence de 0,25 mL (1,77 mmole) de triéthylamine, 0,175 g (1,3 mmole) de 1-hydroxybenzotriazole monohydrate et de 0,25 g (1,3 mmole) de chlorhydrate de *N*-(3-diméthylaminopropyl)-*N*'-éthylcarbodümide. On obtient ainsi 0,15 g du produit attendu sous la forme d'un solide.
PF = 207 - 208 °C
R.M.N. $^1H$ (DMSO $D_6$), $\delta$ (ppm): 10,29 (s, 1 H) ; 8,81 (s, 1 H) ; 8,72 (s, 1H) ; 8,09 (s, 1H) ; 7,57 (s, 1 H) ; 7,32 (m, 1 H) ; 7,09 (m, 1H) ; 6,99 (d, 1 H) ; 6,91 (d, 1 H) ; 6,7 (s, 1 H) ; 5,95 (s, 2H) ; 3,5 (m, 4H) ; 1,99 (m, 4H).

**<u>Exemple 18</u> (Composé N°35 du tableau 2)**

*N*-[5-fluoro-6-(*N*,*N*-diméthylamino)pyridin-3-yl]-5-trifluorométhyl-1-(3-fluorobenzyl)-1*H*-pyrrolo[2,3-*b*]pyridine-2-carboxamide.

Le composé N° 35 a été préparé selon un procédé similaire à celui décrit à l'étape 1.4 en faisant réagir 0,4 g (1,18 mmole) de 5-fluoro-6-(*N*,*N*-diméthylamino)-3-aminopyridine (WO2004/110986), avec 0,4 g (0,18 mmole) de l'acide 5-trifluorométhyl-1-[(3-fluorophényl)méthyl]-1*H*-pyrrolo[2,3-*b*]pyridine-2-carboxylique obtenu à l'étape 1.3. On obtient ainsi 0,33 g du produit attendu sous la forme d'un solide.
PF = 199 - 200 °C
R.M.N. $^1H$ (DMSO $D_6$), $\delta$ (ppm): 10,7 (s, 1H); 8,85 (s, 1H) ; 8,77 (s, 1H) ; 8,29 (s, 1H) ; 7,89 (d, 1 H) ; 7,6 (s, 1 H) ; 7,32 (m, 1 H) ; 7,09 (m, 1 H) ; 6,98 (m, 2H) ; 6 (s, 2H) ; 3,02 (s, 6H).

**<u>Exemple 19</u> (Composé N°36 du tableau 2)**

*N*-[2-(*N*,*N*-diméthylamino)pyrimidin-5-yl]-5-trifluorométhyl-1-(3-fluorobenzyl)-1*H*-pyrrolo[2,3-*b*]pyridine-2-carboxamide.

19.1 *N*-(2-chloropyrimidin-5-yl)-5-trifluorométhyl-1-(3-fluorobenzyl)-1*H*-pyrrolo[2,3-*b*]pyridine-2-carboxamide.

Selon un procédé similaire à celui décrit à l'étape 1.4, en partant de 1,08 g (3,2 mmoles) d'acide 5-trifluorométhyl-1-[(3-fluorophényl)méthyl]-1*H*-pyrrolo[2,3-*b*]pyridine-2-carboxylique, décrit à l'étape 1.3, et de 0,46 g (3,52 mmoles) de 2-chloro-5-aminopyrimidine, on obtient 0,33 g du produit attendu.

19.2 *N*-[2-(*N*,*N*-diméthylamino)pyrimidin-5-yl]-5-trifluorométhyl-1-(3-fluorobenzyl)-1*H*-pyrrolo[2,3-*b*]pyridine-2-carboxamide (composé n° 36 du tableau n°2)

On chauffe, 20 h à 110°C dans un tube scellé, un mélange de 0,2 g (0,44 mmole) de N-(2-chloropyrimidin-5-yl)-5-trifluorométhyl-1-(3-fluorobenzyl)-1*H*-pyrrolo[2,3-b]pyridine-2-carboxamide, préparé à l'étape précédente, et de 1,3 mL (8,89 mmoles) d'une solution aqueuse de diméthylamine à 40%, le tout en solution dans 15 mL d'éthanol. Après ce temps, on laisse revenir le mélange réactionnel à température ambiante puis on recueille par filtration un précipité blanc que l'on lave à l'eau et séche sous pression réduite pour obtenir 0,14 g du produit attendu.
PF = 236 - 238 °C
R.M.N. $^1$H (DMSO $D_6$), $\delta$ (ppm): 10,51 (s, 1H) ; 8,82 (s, 1 H) ; 8,75 (s, 1 H) ; 8,6 (s, 2H) ; 7,56 (s, 1 H) ; 7,32 (m, 1 H) ; 7,08 (m, 1 H) ; 6,96 (m, 2H) ; 5,98 (s, 2H) ; 3,11 (s, 6H).

**<u>Exemple 20</u> (Composé N°37 du tableau 2)**

*N*-[6-(3-azabicyclo[3.2.0]hept-3-yl)pyridin-3-yl]-5-trifluorométhyl-1-(3-fluorobenzyl)-1*H*-pyrrolo[2,3-*b*]pyridine-2-carboxamide

20.1 2-(3-azabicyclo[3.2.0]hept-3-yl)-5-nitropyridine.

On chauffe durant 4 h à 65°C, un mélange de 0,1 g (0,63 mmole) de 2-chloro-5-nitropyridine, de 0,1 g (0,75 mmole) de 3-aza-bicyclo[3.2.0]heptane *(*J.Med.Chem. 1967, 10(4), 621) et de 0,26 mL (0,19 mmole) de triéthylamine dans 10 mL de dioxane. Le mélange réactionnel est ensuite versé dans 50 mL d'eau glacée. On agite le mélange jusqu'à fusion compléte de la glace puis on recueille par filtration un précipité jaune que l'on sèche sous pression réduite. On isole ainsi 0,121 g du produit attendu sous la forme d'une poudre jaune.
R.M.N. $^1$H (DMSO $D_6$), $\delta$ (ppm): 9 (d, 1 H) ; 8,23 (dxd, 1 H) ; 6,7 (d, 1 H) ; 3,81 (pic élargi, 2H) ; 3,51 (m, 2H) ; 3,09 (m, 2H) ; 2,27 (m, 2H) ; 1,71 (m, 2H).

20.2 6-(3-azabicyclo[3.2.0]hept-3-yl)-3-aminopyridine (Amine Va)

Selon un protocole similaire à celui décrit à l'étape 14.2, à partir de 0,12 g (0,55 mmole) de 2-(3-azabicyclo [3.2.0]hept-3-yl)-5-nitropyridine, préparé à l'étape précédente, et de 0,17 g de Palladium sur charbon à 10%, on obtient 0,096 g du composé attendu. R.M.N. $^1$H (DMSO $D_6$), $\delta$ (ppm): 7,62 (d, 1H) ; 6,95 (d, 1H) ; 6,49 (d, 1H) ; 4,42 (pic élargi, 2H) ; 3,52 (d, 2H) ; 2,98 (m, 4H) ; 2,21 (m, 2H) ; 1,72 (m, 2H).

20.3 *N*-[6-(3-azabicyclo[3.2.0]hept-3-yl)pyridin-3-yl]-5-trifluorométhyl-1-(3-fluorobenzyl)-1*H*-pyrrolo[2,3-*b*]pyridine-2-carboxamide (composé n° 37 du tableau n°2)

Selon un procédé similaire à celui décrit à l'étape 12.3, en partant de 0,12 g (0,35 mmole) d'acide 5-trifluorométhyl-1-[(3-fluorophényl)méthyl]-1*H*-pyrrolo[2,3-*b*]pyridine-2-carboxylique, décrit à l'étape 1.3, et de 0,075 g (0,38 mmole) de 6-(3-azabicyclo[3.2.0]hept-3-yl)-3-aminopyridine, préparée à l'étape précédente, on obtient 0,12 g du produit attendu.
PF = 172 - 174°C
R.M.N. $^1$H (DMSO $D_6$), $\delta$ (ppm): 10,45 (s, 1 H) ; 8,81 (s, 1 H) ; 8,71 (s, 1 H) ; 8,36 (s, 1H) ; 7,81 (d, 1 H) ;7,52 (s, 1 H) ; 7,31 (m, 1H) ; 7,08 (m, 1 H) ; 6,92 (m, 2H) ; 6,62 (d, 1H) ; 5,98 (s, 2H) ; 3,67 (d, 2H) ; 3,2 (m, 2H) ; 3,02 (m, 2H) ; 2,23 (m, 2H) ; 1,72 (m, 2H).

**<u>Exemple 21</u> (Composé N°38 du tableau 2)**

*N*-[6-(3-azabicyclo[3.1.0]hex-3-yl)pyridin-3-yl]-5-trifluorométhyl-1-(3-fluorobenzyl)-1*H*-pyrrolo[2,3-*b*]pyridine-2-carboxamide.

21.1 2-(3-azabicyclo[3.1.0]hex-3-yl)-5-nitropyridine

On chauffe durant 4 h à 65°C, un mélange de 0,5 g (3,15 mmoles) de 2-chloro-5-nitropyridine, de 0,452 g (3,78 mmoles) de 3-aza-bicyclo[3.2.0]heptane *(*J.Med.Chem. 1967, 10(4), 621) et de 1,32 mL (6,94 mmoles) de triéthylamine dans 10 mL de dioxane. Le mélange réactionnel est ensuite versé dans 50 mL d'eau glacée. On agite le mélange jusqu'à fusion compléte de la glace puis on recueille par filtration un précipité jaune que l'on sèche sous pression réduite. On isole ainsi 0,63 g du produit attendu sous la forme d'une poudre jaune.
R.M.N. $^1$H (DMSO $D_6$), $\delta$ (ppm): 8,91 (d, 1H) ; 8,15 (dxd, 1H) ; 6,53 (d, 1H) ; 3,95 - 3,45 (m, 4H) ; 1,75 (m, 2H) ; 0,77 (m, 1 H) ; 0,65 (m, 1 H).

21.2 6-(3-azabicyclo[3.1.0]hex-3-yl)-3-aminopyridine (amine Vf)

Selon un protocole similaire à celui décrit à l'étape 14.2, à partir de 0,6 g (2,92 mmoles) de 2-(3-azabicyclo [3.2.0]hex-3-yl)-5-nitropyridine, préparé à l'étape précédente, et de 0.9 g de Palladium sur charbon à 10%, on obtient 0,49 g du composé attendu.
R.M.N. $^1H$ (DMSO $D_6$), δ (ppm): 7,55 (s, 1H) ; 6,99 (d, 1 H) ; 6,25 (d, 1 H) ; 4,32 (pic élargi, 2H) ; 3,51 (d, 2H) ; 3,11 (m, 2H) ; 1,6 (m, 2H) ; 0,66 (m, 1H) ; 0,2 (m, 1H).

21.3 *N*-[6-(3-azabicyclo[3.1.0]hex-3-yl)pyridin-3-yl]-5-trifluorométhyl-1-(3-fluorobenzyl)-1*H*-pyrrolo[2,3-*b*]pyridine-2-carboxamide (composé n°38 du tableau n°2)

Selon un procédé similaire à celui décrit à l'étape 12.3, en partant de 0,5 g (1,48 mmole) d'acide 5-trifluorométhyl-1-[(3-fluorophényl)méthyl]-1*H*-pyrrolo[2,3-*b*]pyridine-2-carboxylique, décrit à l'étape 1.3, et de 0,3 g (1,71 mmole) de 6-(3-azabicyclo[3.1.0]hex-3-yl)-3-aminopyridine, préparée à l'étape précédente, on obtient 0,233 g du produit attendu.
PF = 212 - 214 °C
R.M.N. $^1H$ (DMSO $D_6$), δ (ppm): 10,42 (s, 1 H) ; 8,81 (s, 1 H) ; 8,72 (s, 1H) ; 8,31 (s, 1H) ; 7,79 (d, 1 H) ;7,52 (s, 1 H) ; 7,31 (m, 1 H) ; 7,08 (m, 1 H) ; 6,93 (m, 2H) ; 6,48 (d, 1 H) ; 5,97 (s, 2H) ; 3,63 (d, 2H) ; 3,31 (m, 2H) ; 1,69 (m, 2H) ; 0,74 (m, 1 H) ; 0,2 (m, 1 H).

**<u>Exemple 22</u> (Composé N°39 du tableau 2)**

*N*-[6-(3-azabicyclo[3.1.0]hex-3-yl)-pyridin-3-yl]-5-fluoro-1-(3-fluorobenzyl)-1*H*-pyrrolo[2,3-*b*]pyridine-2-carboxamide.

22.1 acide 5-fluoro-1-(3-fluorobenzyl)-1*H*-pyrrolo[2,3-*b*]pyridine-2-carboxylique

On chauffe durant 1 h 30 à reflux, un mélange de 6,9 g (20,58 mmoles) de 5-fluoro-1-(3-fluorobenzyl)-1*H*-pyrrolo [2,3-*b*]pyridine-2-carboxylate d'éthyle (WO071010138) et de 1,89 g (33,7 mmoles) de potasse dans un mélange de 80 mL d'éthanol et 10 mL d'eau. Après ce temps, le mélange réctionnel est concentré sous pression réduite puis repris dans 200 mL d'eau. La solution est acidifiée par ajouts d'acide chlorhydrique concentré puis extraite trois fois avec 50 mL de dichlorométhane. Les phases organiques sont réunies, séchées sur sulfate de sodium puis concentrées sous pression réduite. Le produit résultant est trituré dans 5 mL de dichlorométhane. On recueille un précipité par filtration qui est séché sous pression réduite. On isole ainsi 5,35 g du produit attendu qui est engagé tel quel dans la suite de la synthèse.

22.2 *N*-[6-(3-azabicyclo[3.1.0]hex-3-yl)-pyridin-3-yl]-5-fluoro-1-(3-fluorobenzyl)-1*H*-pyrrolo[2,3-*b*]pyridine-2-carboxamide (composé n° 39 du tableau n°2)

Selon un procédé similaire à celui décrit à l'étape 12.3, en partant de 0,45 g (1,56 mmole) d'acide 5-fluoro-1-[(3-fluorophényl)méthyl]-1*H*-pyrrolo[2,3-*b*]pyridine-2-carboxylique, décrit à l'étape précédente, et de 0,3 g (1,71 mmole) de 6-(3-azabicyclo[3.1.0]hex-3-yl)-3-aminopyridine, préparée à l'étape 21.2, on obtient 0,47 g du produit attendu.
PF = 222 - 224 °C
R.M.N. $^1H$ (DMSO $D_6$), δ (ppm): 10,32 (s, 1 H) ; 8,47 (s, 1 H) ; 8,31 (s, 1 H) ; 8,13 (d, 1H) ; 7,78 (d, 1H) ;7,39 (s, 1 H) ; 7,19 (m, 1 H) ; 7,02 (m, 1 H) ; 6,92 (m, 2H) ; 6,47 (d, 1 H) ; 5,9 (s, 2H) ; 3,62 (d, 2H) ; 3,31 (m, 2H) ; 1,69 (m, 2H) ; 0,75 (m, 1 H) ; 0,2 (m, 1 H).

**<u>Exemple 23</u> (Composé N°171 du tableau 2)**

3-[[5-[[[1-(3-fluorobenzyl)-5-trifluorométhyl-1*H*-pyrrolo[2,3-b]pyridin-2-yl]carbonyl]amino]pyridin-2-yl]amino]propionate d'éthyle

23.1 3-[(5-aminopyridin-2-yl)amino]propionate d'éthyle

On agite 7 heures, à 20°C sous 5 atmosphères d'hydrogène, une suspension de 2,3 g (9,61 mmoles) de 3-[(5-nitropyridin-2-yl)amino]propionate d'éthyle (JP07/051121) et de 0,5 g de Nickel de Raney dans 96 mL d'acide acétique. Après ce temps, le mélange réactionnel est filtré sur un tampon de célite puis concentré sous pression réduite. On utilise tel quel le produit obtenu dans la suite de la synthèse.

23.4 3-[[5-[[[1-(3-fluorobenzyl)-5-trifluorométhyl-1*H*-pyrrolo[2,3-*b*]pyridin-2-yl]carbonyl]amino]pyridin-2-yl]amino]propionate d'éthyle

Selon un procédé similaire à celui décrit à l'exemple 1.4, à partir de 0,558 g (2,67 mmoles) de 3-[(5-aminopyridin-2-yl)amino]propionate d'éthyle, obtenu à l'étape précédente, et de 0,3 g (0,89 mmole) d'acide 5-trifluorométhyl-1-[(3-fluorophényl)méthyl]-1*H*-pyrrolo[2,3-*b*]pyridine-2-carboxylique, obtenu selon l'étape 1.3, on obtient 0,26 g de produit attendu.
LCMS : $[MH]^+$ = 530
R.M.N. $^1$H (DMSO $D_6$), δ (ppm): 10,4 (s, 1H); 8,81 (s, 1H) ; 8,71 (s, 1H) ; 8,25 (s, 1H) ; 7,68 (d, 1 H) ;7,53 (s, 1 H) ; 7,31 (m, 1 H) ; 7,05 (m, 1 H) ; 6,92 (m, 2H) ; 6,59 (m, 1 H) ; 6,51 (d, 1 H) ; 5,96 (s, 2H) ; 4,1 (q, 2H) ; 3,5 (m, 2H) ; 2,56 (m, 2H) ; 1,2 (t, 3H).

**<u>Exemple 24</u> (Composé N°40 du tableau 2)**

Acide 3-[[5-[[[1-(3-fluorobenzyl)-5-trifluorométhyl-1*H*-pyrrolo[2,3-b]pyridin-2-yl]carbonyl]amino]pyridin-2-yl]amino]propionique

On agite 20 h à 20 °C une solution, dans 10 mL d'éthanol, de 0,26 g (0,49 mmole) du composé N°171 préparé dans l'exemple 23 et de 1 mL (1mmole) d'une solution molaire de soude. Après ce temps, le mélange est concentré sous pression réduite puis repris dans 100 mL d'eau et 100 mL d'acétate d'éthyle. Le pH de la phase aqueuse est acidifié par ajouts successifs d'une solution d'acide chlorhydrique 1 N. La phase organique est alors séparée, lavée une fois avec 100 mL d'eau, 1 fois avec 50 mL d'une solution saturée en chlorure de sodium puis séchée sur sulfate de magnésium et concentrée sous pression réduite. On isole ainsi 0,21 g du produit attendu.
PF = 196 -199 °C
R.M.N. $^1$H (DMSO $D_6$), δ (ppm): 12,1 (pic élargi, 1H) ; 10,39 (s, 1H) ; 8,8 (s, 1H) ; 8,71 (s, 1 H) ; 8,25 (s, 1 H) ; 7,69 (d, 1 H) ;7,52 (s, 1 H) ; 7,31 (m, 1 H) ; 7,07 (m, 1 H) ; 6,92 (m, 2H) ; 6,5 (m, 2H) ; 5,96 (s, 2H) ; 3,46 (m, 2H) ; 2,49 (m, 2H partiellement couverts par le pic du DMSO).

**<u>Exemple 25(Composé</u> N°41 du tableau 2)**

*N*-[6-(3-hydroxypropylamino)pyridin-3-yl]-5-trifluorométhyl-1-(3-fluorobenzyl)-1*H*-pyrrolo[2,3-*b*]pyridine-2-carboxamide

On agite 48 h à reflux un mélange, dans 7,5 mL de tétrahydrofurane, de 0,2 g (0,38 mmole) du composé N°171, préparé à l'étape 23, de 0,14 g (3,78 mmoles) de borohydrure de sodium et de 0,15 mL (3,78 mmoles) de méthanol. Après ce temps, le mélange réactionnel est versé dans 200 mL d'eau glacée. On ajoute 1 mL d'acide acétique puis le mélange est extrait 2 fois avec 100 mL d'acétate d'éthyle. Les phases organiques sont réunies puis lavées 2 fois avec 100 mL d'eau, 1 fois avec 50 mL d'une solution saturée en chlorure de sodium puis séchées sur sulfate de magnésium et concentrées sous pression réduite. Le résidu obtenu est purifié par chromatographie sur colonne de silice en éluant avec un mélange de dichlorométhane et de méthanol. On obtient ainsi 67 mg du composé attendu.
PF = 207 - 210 °C
R.M.N. $^1$H (DMSO $D_6$), δ (ppm): 10,22 (s, 1H) ; 8,68 (s, 1H) ; 8,59 (s, 1H) ; 8,09 (s, 1H) ; 7,52 (d, 1 H) ;7,4 (s, 1 H) ; 7,17 (m, 1 H) ; 6,91 (m, 1 H) ; 6,8 (m, 2H) ; 6,35 (d, 1 H) ; 6,3 (m, 1H) ; 5,83 (s, 2H) ; 4,33 (t, 1H) ; 3,37 (m, 2H) ; 2,49 (m, 2H partiellement couverts par le pic du DMSO) ; 1,55 (m, 2H).

**<u>Exemple 26</u>(Composé N°29 du tableau 2)**

*N*-[4-méthoxy-6-(pyrrolidin-1-yl)-pyridin-3-yl]-5-trifluorométhyl-1-[(4-pyridinyl)méthy)]-1*H*-pyrrolo[2,3-*b*]pyridine-2-carboxamide

26.1 4-méthoxy-5-nitro-2-(pyrrolidin-1-yl)pyridine

On procède suivant la méthode décrite à l'étape 12.1 à partir de 5,4 g (28,64 mmoles) de 2-chloro-4-méthoxy-5-nitropyridine (WO2003/080610) et de 4,53 g (63 mmoles) de pyrrolidine. Le produit obtenu est, dans ce cas, purifié par chromatographie sur colonne de silice en éluant avec un mélange d'heptane et d'acétate d'éthyle. On isole ainsi 3 g du produit attendu.
R.M.N. $^1$H (DMSO $D_6$), δ (ppm): 8,98 (s, 1H) ; 5,79 (s, 1H) ; 4,07 (s, 3H) ; 3,65 (m, 4H) ; 2,17 (m, 4H).

26.2 5-amino-4-méthoxy-2-(pyrrolidin-1-yl)pyridine (amine Vd)

On procède suivant la méthode décrite à l'étape 12.2 à partir de 1,5 g (6,72 mmoles) de 4-méthoxy-5-nitro-2-(pyrrolidin-1-yl)-pyridine, obtenu à l'étape 6.1, et de 0,15 g de palladium sur charbon à 10%. On obtient ainsi 1,25 g du produit attendu.
R.M.N. $^1$H (DMSO $D_6$), $\delta$ (ppm): 7,16 (s, 1 H) ; 6,61 (s, 1 H) ; 4,52 (pic élargi, 2H) ; 3,71 (s, 3H) ; 3,29 (m, 4H) ; 1,81 (m, 4H).

26.3 *N*-[4-méthoxy-6-(pyrrolidin-1-yl)pyridin-3-yl]-5-trifluorométhyl-1-[(4-pyridyl)méthyl]-1*H*-pyrrolo[2,3-*b*]pyridine-2-carboxamide (Composé n° 29 du tableau 2)

On procède suivant la méthode décrite dans l'exemple 12.3 à partir de 5-amino-4-méthoxy-2-(pyrrolidin-1-yl) pyridine, préparée à l'étape précédente et d'acide 5-trifluorométhyl-1-[(4-pyridyl)méthyl]-1*H*-pyrrolo[2,3-*b*]pyridine-2-carboxylique préparé à l'étape 9.2.
PF = 222 - 224 °C
R.M.N. $^1$H (DMSO $D_6$), $\delta$ (ppm): 9,86 (s, 1 H) ; 8,79 (s, 1 H) ; 8,71 (s, 1H) ; 8,45 (d, 2H) ; 7,85 (s, 1 H) ; 7,59 (s, 1 H) ; 7,02 (d, 2H) ; 6,01 (s, 1 H) ; 5,95 (s, 1 H) ; 4,38 (s, 3H) ; 3,39 (m, 4H) ; 1,93 (m, 4H)

__Exemple 27__ **(Composé N°124 du tableau 2)**

Trifluoroacétate (1 :1) du *N*-[6-(3-hydroxyazétidin-1-yl)-pyridin-3-yl]-1-(3-méthoxybenzyl)-1*H*-pyrrolo[2,3-*b*]pyridine-2-carboxamide

27.1 1-(3-méthoxybenzyl)-1*H*-pyrrolo[2,3-*b*]pyridine-2-carboxylate d'éthyle.

On ajoute, goutte à goutte sous argon à 20°C, 0,089 mL (0,45 mmole) d'azodicarboxylate de diisopropyle a un mélange de 260 mg (0,6 mmole) de triphénylphosphine supporté (Argonaut PS-$PPh_3$, charge 2,3 mmole/g), 57 mg (0,3 mmole) de 1H-pyrrolo[2,3-*b*]pyridine-2-carboxylate d'éthyle (WO07/010138) et 62 mg (0,45 mmole) d'alcool 3-méthoxybenzylique dans 2,5 mL de tétrahydrofuranne. Le réacteur est ensuite scellé et le mélange est agité 3 jours à 20°C. Après ce temps, le mélange réactionnel est filtré, le résidu solide est lavé avec 5 mL de tétrahydrofuranne et le filtrat est concentré sous pression réduite pour conduire au produit attendu qui est utilisé tel quel dans la suite de la synthèse.

27.2 Acide 1-(3-méthoxybenzyl)-1*H*_pyrrolo[2,3-*b*]pyridine-2-carboxylique.

On chauffe, 2 h à 70°C en tube scellé, un mélange du 1-(3-méthoxybenzyl)-1*H*-pyrrolo[2,3-*b*]pyridine-2-carboxylate d'éthyle, obtenu à l'étape précédente et 0,195 mL (0,39 mmole) de soude 2N dans 5 mL d'éthanol et 1 mL d'eau. Après ce temps, le mélange réactionnel est concentré sous pression réduite, repris dans 2 mL de diméthylformamide et 0,03 mL d'acide trifluoroacétique. La solution est filtrée puis chromatographiée sur colonne HPLC en phase inverse. On isole ainsi 33,4 mg du produit attendu.

27.3 Trifluoroacétate (1 :1) de *N*-[6-(3-hydroxyazétidin-1-yl)pyridin-3-yl]-1-(3-méthoxybenzyl)-1*H*-pyrrolo[2,3-*b*]pyridine-2-carboxamide (composé n° 124 du tableau n° 2)

Selon un procédé similaire à celui décrit à l'étape 12.3, en partant de 0,0167 g (0,59 mmole) d'acide 1-(3-méthoxybenzyl)-1*H*-pyrrolo[2,3-*b*]pyridine-2-carboxylique, décrit à l'étape précédente, et de 0,0149 g (0,09 mmole) de 3-amino-6-(3-hydroxyazétidin-1-yl)pyridine, préparée à l'étape 14.2, on obtient 0,013 g du produit attendu.
LCMS : $[MH]^+$ = 430
R.M.N. $^1$H (DMSO $D_6$), $\delta$ (ppm): 10,59 (s, 1H) ; 8,45 (d, 1H) ; 8,4 (s, 1H) ; 8,21 (d, 1H) ; 8,01 (d, 1H) ; 7,39 (s, 1H) ; 7,26 (m, 1H) ; 7,12 (dxd, 1H) ; 6,75 (m, 2H) ; 6,52 (m, 2H) ; 5,89 (s, 2H) ; 4,62 (m, 1H) ; 4,32 (m, 2H) ; 3,86 (m, 2H) ; 3,62 (s, 3H).

Selon un procédé similaire à celui décrit à l'exemple 27, 128 autres composés ont été préparés et caractérisés par leur pic de masse en LC-MS dans les tableaux 2, 3 et 4. A titre d'exemples, sont décrits ci-dessous, les spectres RMN $^1$H de certains de ces produits :

Trifluoroacétate (1 :1) du *N*-[6-(azétidin-1-yl)-pyridin-3-yl]-1-(2-fluorobenzyl)-5-trifluorométhyl-1*H*-pyrrolo[2,3-*b*]pyridine-2-carboxamide (composé n° 43 du tableau n° 2) LCMS : $[MH]^+$ = 470
R.M.N. $^1$H (DMSO $D_6$), $\delta$ (ppm): 10,7 (s, 1H); 8,79 (d, 2H) ; 8,34 (s, 1H) ; 7,99 (d, 1H) ; 7,59 (s, 1 H) ; 7,28 (m, 1 H) ; 7,19 (m, 1 H) ; 7,02 (t, 1 H) ; 6,7 (m, 2H) ; 6 (s, 2H) ; 4,12 (m, 4H) ; 2,4 (m, 2H).

Trifluoroacétate (1 :1) du *N*-[6-(azétidin-1-yl)pyridin-3-yl]-1-(4-fluorobenzyl)-5-trifluorométhyl-1*H*-pyrrolo[2,3-*b*]pyridine-2-carboxamide (composé n° 44 du tableau n° 2) LCMS : $[MH]^+$ = 470
R.M.N. $^1H$ (DMSO $D_6$), δ (ppm): 10,7 (s, 1H) ; 8,82 (s, 1H) ; 8,76 (s, 1H) ; 8,49 (s, 1H) ; 8,02 (s, 1H) ; 7,58 (s, 1H) ; 7,19 (m, 2H) ; 7,09 (m, 2H) ; 6,75 (d, 1H) ; 5,93 (s, 2H) ; 4,12 (m, 4H) ; 2,41 (m, 2H).

Trifluoroacétate (1 :1) du *N*-[6-(azétidin-1-yl)pyridin-3-yl]-1-(3-trifluorométhylbenzyl)-5-trifluorométhyt-1*H*-pyrrolo [2,3-*b*]pyridine-2-carboxamide (composé n° 45 du tableau n° 2) LCMS : $[MH]^+$ = 520
R.M.N. $^1H$ (DMSO $D_6$), δ (ppm): 10,7 (s, 1 H) ; 8,85 (s, 1H) ; 8,78 (s, 1H) ; 8,38 (s, 1H) ; 8 (pic élargi, 1 H) ; 7,6 (m, 3H) ; 7,51 (m, 1 H) ; 7,37 (m, 1H) ; 6,72 (pic élargi, 1H) ; 6,03 (s, 2H) ; 4,11 (m, 4H) ; 2,39 (m, 2H).

Trifluoroacétate (1 :1) du *N*-[6-(azétidin-1-yl)pyridin-3-yl]-1-(4-méthylbenzyl)-5-trifluorométhyl-1*H*-pyrrolo[2,3-*b*]pyridine-2-carboxamide (composé n° 46 du tableau n° 2) LCMS : $[MH]^+$ = 466
R.M.N. $^1H$ (DMSO $D_6$), δ (ppm): 10,61 (s, 1H) ; 8,82 (s, 1H) ; 8,73 (s, 1H) ; 8,39 (s, 1H) ; 8 (pic élargi, 1 H) ; 7,52 (s, 1 H) ; 7,04 (m, 4H) ; 6,71 (pic élargi, 1 H) ; 5,91 (s, 2H) ; 4,11 (m, 4H) ; 2,4 (m, 2H) ; 2,21 (s, 3H).

Trifluoroacétate (1 :1) du *N*-[6-(azétidin-1-yl)pyridin-3-yl]-1-(3-méthoxybenzyl)-5-trifluorométhyl-1*H*-pyrrolo[2,3-*b*] pyridine-2-carboxamide (composé n° 48 du tableau n° 2) LCMS : $[MH]^+$ = 482
R.M.N. $^1H$ (DMSO $D_6$), δ (ppm): 10,7 (s, 1H); 8,81 (s, 1H) ; 8,75 (s, 1H); 8,39 (s, 1H); 8 (pic élargi, 1 H) ; 7,55 (s, 1 H) ; 7,17 (t, 1 H) ; 6,79 (dxd, 1H) ; 6,75 (pic élargi, 1 H) ; 6,67 (s, 1 H) ; 6,63 (d, 1 H) ; 5,94 (s, 2H) ; 4,11 (m, 4H) ; 3,65 (s, 3H) ; 2,41 (m, 2H).

Trifluoroacétate (1 :1) du *N*-[6-(azétidin-1-yl)-pyridin-3-yl]-1-(3-chlorobenzyl)-5-trifluorométhyl-1*H*-pyrrolo[2,3-*b*]pyridine-2-carboxamide (composé n° 49 du tableau n° 2) LCMS : $[MH]^+$ = 486
R.M.N. $^1H$ (DMSO $D_6$), δ (ppm): 10,71 (s, 1H) ; 8,84 (s, 1H) ; 8,78 (s, 1H) ; 8,39 (s, 1H) ; 8 (pic élargi, 1 H) ; 7,6 (s, 1 H) ; 7,3 (m, 2H) ; 7,2 (d, 1 H) ; 7,08 (m, 1H) ; 6,72 (pic élargi, 1 H) ; 5,94 (s, 2H) ; 4,1 (m, 4H) ; 2,4 (m, 2H).

Trifluoroacétate (1 :1) du *N*-[6-(azétidin-1-yl)-pyridin-3-yl]-1-(3-trifluorométhoxybenzyl)-5-trifluorométhyl-1*H*-pyrrolo [2,3-*b*]pyridine-2-carboxamide (composé n° 50 du tableau n° 2) LCMS : $[MH]^+$ = 536
R.M.N. $^1H$ (DMSO $D_6$), δ (ppm): 10,71 (s, 1 H) ; 8,83 (s, 1 H) ; 8,78 (s, 1 H) ; 8,38 (s, 1 H) ; 8 (pic élargi, 1 H) ; 7,6 (s, 1 H) ; 7,4 (t, 1 H) ; 7,25 (d, 1 H) ; 7,16 (s, 1 H) ; 7,11 (d, 1 H) ; 6,73 (pic élargi, 1 H) ; 6 (s, 2H) ; 4,12 (m, 4H) ; 2,41 (m, 2H).

Trifluoroacétate (1 :1) du *N*-[6-(azétidin-1-yl)-pyridin-3-yl]-1-(4-méthoxybenzyl)-5-trifluorométhyl-1*H*-pyrrolo[2,3-*b*] pyridine-2-carboxamide (composé n° 51 du tableau n° 2) LCMS : $[MH]^+$ = 482
R.M.N. $^1H$ (DMSO $D_6$), δ (ppm): 10,6 (s, 1H); 8,82 (s, 1H) ; 8,71 (s, 1H) ; 8,39 (s, 1H) ; 8,01 (pic élargi, 1 H) ; 7,51 (s, 1 H) ; 7,11 (d, 2H) ; 6,82 (d, 1 H) ; 6,71 (pic élargi, 1 H) ; 5,9 (s, 2H) ; 4,12 (m, 4H) ; 3,67 (s, 3H) ; 2,41 (m, 2H).

Trifluoroacétate (1 :1) du *N*-[6-(azétidin-1-yl)-pyridin-3-yl]-1-(4-méthoxybenzyl)-5-fluoro-1*H*-pyrrolo[2,3-*b*]pyridine-2-carboxamide (composé n° 62 du tableau n° 2) LCMS : $[MH]^+$ = 432
R.M.N. $^1H$ (DMSO $D_6$), δ (ppm): 10,55 (s, 1 H) ; 8,49 (s, 1 H) ; 8,39 (s, 1 H) ; 8,14 (dxd, 1 H) ; 8,01 (pic élargi, 1 H) ; 7,35 (s, 1H) ; 7,1 (d, 2H) ; 6,8 (d, 2H) ; 6,72 (pic élargi, 1 H) ; 5,82 (s, 2H) ; 4,12 (m, 4H) ; 3,68 (s, 3H) ; 2,4 (m, 2H).

Trifluoroacétate (1 :1) du *N*-[6-(azétidin-1-yl)-pyridin-3-yl]-1-(3-trifluorométhylbenzyl)-6-trifluorométhyl-1*H*-pyrrolo [2,3-*b*]pyridine-2-carboxamide (composé n° 67 du tableau n° 2) LCMS : $[MH]^+$ = 520
R.M.N. $^1H$ (DMSO $D_6$), δ (ppm): 10,7 (s, 1H) ; 8,53 (d, 1H) ; 8,39 (s, 1H) ; 7,98 (pic élargi, 1 H) ; 7,77 (d, 1 H) ; 7,71 (s, 1 H) ; 7,61 (d, 1H) ; 7,55 (s, 1 H) ; 7,49 (m, 2H) ; 6,71 (pic élargi, 1 H) ; 5,98 (s, 2H) ; 4,12 (m, 4H) ; 2,39 (m, 2H).

Trifluoroacétate (1 :1) du *N*-[6-(azétidin-1-yl)-pyridin-3-yl]-1-(4-méthylbenzyl)-6-trifluorométhyl-1*H*-pyrrolo[2,3-*b*]pyridine-2-carboxamide (composé n° 68 du tableau n° 2) LCMS : $[MH]^+$ = 466
R.M.N. $^1H$ (DMSO $D_6$), δ (ppm): 10,68 (s, 1H) ; 8,51 (d, 1H) ; 8,39 (s, 1H) ; 7,98 (pic élargi, 1 H) ; 7,71 (d, 1 H) ; 7,5 (s, 1 H) ; 7,05 (m, 4H) ; 6,75 (pic élargi, 1 H) ; 5,87 (s, 2H) ; 4,12 (m, 4H) ; 2,39 (m, 2H) ; 2,2 (s, 3H).

Trifluoroacétate (1 :1) du *N*-[6-(azétidin-1-yl)-pyridin-3-yl]-1-(3-chlorobenzyl)-6-trifluorométhyl-1*H*-pyrrolo[2,3-*b*]pyridine-2-carboxamide (composé n° 71 du tableau n° 2) LCMS : $[MH]^+$ = 486
R.M.N. $^1H$ (DMSO $D_6$), δ (ppm): 10,7 (s, 1 H) ; 8,54 (d, 1 H) ; 8,36 (s, 1 H) ; 7,98 (pic élargi, 1 H) ; 7,73 (d, 1 H) ; 7,55 (s, 1 H) ; 7,29 (m, 3H) ; 7,09 (m, 1 H) ; 6,72 (pic élargi, 1H) ; 5,91 (s, 2H) ; 4,1 (m, 4H) ; 2,4 (m, 2H).

Trifluoroacétate (1 :1) du *N*-[6-(azétidin-1-yl)-pyridin-3-yl]-1-benzyl-1*H*-pyrrolo[2,3-*b*]pyridine-2-carboxamide (composé n° 75 du tableau n° 2)
LCMS : $[MH]^+$ = 384
R.M.N. $^1H$ (DMSO $D_6$), δ (ppm): 10,5 (s, 1H); 8,49 (d, 1H) ; 8,38 (s, 1H) ; 8,22 (d, 1H) ; 8,01 (pic élargi, 1 H) ; 7,41 (s, 1 H) ; 7,22 (m, 4H) ; 7,09 (m, 2H) ; 6,72 (pic élargi, 1H) ; 5,93 (s, 2H) ; 4,12 (m, 4H) ; 2,39 (m, 2H).

Trifluoroacétate (1 :1) du *N*-[6-(azétidin-1-yl)-pyridin-3-yl]-1-(3-trifluorométhylbenzyl)-1*H*-pyrrolo[2,3-*b*]pyridine-2-carboxamide (composé n° 78 du tableau n° 2)
LCMS : $[MH]^+$ = 452
R.M.N. $^1H$ (DMSO $D_6$), δ (ppm): 10,6 (s, 1H) ; 8,49 (d, 1 H) ; 8,39 (s, 1 H) ; 8,27 (d, 1H) ; 8,02 (pic élargi, 1 H) ; 7,59 (d, 1H) ; 7,53 (s, 1H) ; 7,48 (m, 2H) ; 7,39 (d, 1 H) ; 7,29 (m, 1 H) ; 6,77 (pic élargi, 1 H) ; 6 (s, 2H) ; 4,12 (m, 4H) ; 2,41 (m, 2H).

Trifluoroacétate (1 :1) du *N*-[6-(azétidin-1-yl)-pyridin-3-yl]-1-(3-méthoxybenzyl)-1*H*-pyrrolo[2,3-*b*]pyridine-2-carboxamide (composé n° 81 du tableau n° 2)
LCMS : $[MH]^+$ = 414
R.M.N. $^1H$ (DMSO $D_6$), δ (ppm): 10,5 (s, 1H); 8,47 (d, 1H) ; 8,39 (s, 1H) ; 8,21 (d, 1H) ; 7,98 (pic élargi, 1 H) ; 7,39 (s, 1 H) ; 7,27 (dxd, 1 H) ; 7,13 (m, 1 H) ; 6,78 (m, 1H) ; 6,7 (pic élargi, 1 H) ; 6,62 (m, 2H) ; 5,91 (s, 2H) ; 4,09 (m, 4H) ; 3,62 (s, 3H) ; 2,4 (m, 2H).

Trifluoroacétate (1 :1) du *N*-[6-(azétidin-1-yl)-pyridin-3-yl]-1-(4-méthoxylbenzyl)-1*H*-pyrrolo[2,3-*b*]pyridine-2-carboxamide (composé n° 84 du tableau n° 2)
LCMS : $[MH]^+$ = 414
R.M.N. $^1H$ (DMSO $D_6$), δ (ppm): 10,31 (s, 1H); 8,47 (d, 1H) ; 8,38 (s, 1H); 8,19 (d, 1H) ; 7,91 (pic élargi, 1 H) ; 7,32 (s, 1 H) ; 7,23 (dxd, 1 H) ; 7,1 (m, 2H) ; 6,78 (m, 2H) ; 6,57 (pic élargi, 1 H) ; 5,85 (s, 2H) ; 4,01 (m, 4H) ; 3,67 (s, 3H) ; 2,35 (m, 2H).

Trifluoroacétate (1 :1) du *N*-[6-(3-hydroxyazétidin-1-yl)pyridin-3-yl]-5-fluoro-1-(3-trifluorométhoxybenzyl)-1*H*-pyrrolo[2,3-*b*]pyridine-2-carboxamide (composé n° 115 du tableau n° 2)
LCMS : $[MH]^+$ = 502
R.M.N. $^1H$ (DMSO $D_6$), δ (ppm): 10,51 (s, 1 H) ; 8,48 (s, 1 H) ; 8,35 (s, 1 H) ; 8,19 (d, 1H) ; 7,92 (pic élargi, 1 H) ; 7,41 (m, 2H) ; 7,21 (d, 1 H) ; 7,12 (m, 2H) ; 6,63 (m, 1 H) ; 5,91 (s, 2H) ; 4,6 (m, 1 H) ; 4,23 (m, 2H) ; 3,78 (m, 2H).

Trifluoroacétate (1 :1) du *N*-[6-(3-hydroxyazétidin-1-yl)-pyridin-3-yl]-5-fluoro-1-(4-méthoxybenzyl)-1*H*-pyrrolo[2,3-*b*]pyridine-2-carboxamide (composé n° 116 du tableau n° 2)
LCMS : $[MH]^+$ = 448
R.M.N. $^1H$ (DMSO $D_6$), δ (ppm): 10,4 (s, 1H) ; 8,47 (s, 1 H) ; 8,36 (s, 1 H) ; 8,11 (dxd, 1H) ; 7,89 (pic élargi, 1H) ; 7,31 (s, 1H) ; 7,1 (d, 2H) ; 6,8 (d, 2H) ; 6,51 (m, 1H) ; 5,81 (s, 2H) ; 4,58 (m, 1 H) ; 4,2 (m, 2H) ; 3,7 (m, 2H) ; 3,67 (s, 3H).

Trifluoroacétate (1 :1) du *N*-[6-(3-hydroxyazétidin-1-yl)-pyridin-3-yl]-5-fluoro-1-(3-chloro-5-trifluorométhylbenzyl)-1*H*-pyrrolo[2,3-*b*]pyridine-2-carboxamide (composé n° 117 du tableau n° 2)
LCMS : $[MH]^+$ = 520
R.M.N. $^1H$ (DMSO $D_6$), δ (ppm): 10,55 (s, 1H) ; 8,49 (s, 1H) ; 8,34 (s, 1H) ; 8,2 (d, 1H) ; 7,9 (pic élargi, 1H) ; 7,73 (s, 1H) ; 7,51 (s, 1H) ; 7,45 (m, 2H) ; 6,61 (m, 1H) ; 5,91 (s, 2H) ; 4,6 (m, 1 H) ; 4,25 (m, 2H) ; 3,78 (m, 2H).

Trifluoroacétate (1 :1) du *N*-[6-(3-hydroxyazétidin-1-yl)-pyridin-3-yl]-1-(3-chlorobenzyl)-1*H*-pyrrolo[2,3-*b*]pyridine-2-carboxamide (composé n° 125 du tableau n° 2)
LCMS : $[MH]^+$ = 434
R.M.N. $^1H$ (DMSO $D_6$), δ (ppm): 10,41 (s, 1H) ; 8,47 (d, 1H) ; 8,35 (s, 1H) ; 8,22 (d, 1H) ; 7,92 (pic élargi, 1 H) ; 7,41 (s, 1 H) ; 7,29 (m, 3H) ; 7,12 (s, 1 H) ; 7,05 (d, 1 H) ; 6,62 (m, 1 H) ; 5,91 (s, 2H) ; 4,61 (m, 1 H) ; 4,25 (m, 2H) ; 3,78 (m, 2H).

Trifluoroacétate (1 :1) de *N*-[6-(azétidin-1-yl)-pyridin-3-yl]-1-benzyl-5-trifluorométhyl-1*H*-pyrrolo[3,2-*b*]pyridine-2-carboxamide (composé n° 129 du tableau n° 3)
LCMS : $[MH]^+$ = 452
R.M.N. $^1H$ (DMSO $D_6$), δ (ppm): 10,72 (s, 1H) ; 8,4 (s, 1H) ; 8,32 (d, 1H) ; 8,01 (pic élargi, 1 H) ; 7,78 (d, 1 H) ; 7,61 (s, 1 H) ; 7,26 (m, 3H) ; 7,11 (m, 2H) ; 6,72 (pic élargi, 1 H) ; 5,95 (s, 2H) ; 4,12 (m, 4H) ; 2,41 (m, 2H).

Trifluoroacétate (1:1) de *N*-[6-(azétidin-1-yl)-pyridin-3-yl]-1-(4-fluorobenzyl)-5-trifluorométhyl-1*H*-pyrrolo[3,2-*b*]pyridine-2-carboxamide (composé n° 131 du tableau n° 3)
LCMS : $[MH]^+$ = 470
R.M.N. $^1H$ (DMSO $D_6$), δ (ppm): 10,71 (s, 1H) ; 8,37 (m, 2H) ; 8 (pic élargi, 1H) ; 7,8 (d, 1 H) ; 7,61 (s, 1 H) ; 7,15 (m, 4H) ; 6,72 (pic élargi, 1 H) ; 5,91 (s, 2H) ; 4,12 (m, 4H) ; 2,41 (m, 2H).

Trifluoroacétate (1:1) du N-[6-(azétidin-1-yl)-pyridin-3-yl]-1-(3-méthylbenzyl)-5-trifluorométhyl-1*H*-pyrrolo[3,2-*b*]pyridine-2-carboxamide (composé n° 134 du tableau n° 3)
LCMS : $[MH]^+$ = 466
R.M.N. $^1H$ (DMSO $D_6$), δ (ppm): 10,75 (s, 1H) ; 8,39 (s, 1H) ; 8,31 (d, 1H) ; 8 (pic élargi, 1 H) ; 7,78 (d, 1 H) ; 7,59 (s, 1H) ; 7,15 (m, 1H) ; 7,04 (d, 1H) ; 6,96 (s, 1H) ; 6,84 (d, 1H) ; 6,72 (pic élargi, 1 H) ; 5,9 (s, 2H) ; 4,12 (m, 4H) ; 2,4 (m, 2H) ; 2,2 (s, 3H).

Trifluoroacétate (1 :1) du *N*-[6-(azétidin-1-yl)-pyridin-3-yl]-1-(3-méthoxybenzyl)-5-trifluorométhyl-1*H*-pyrrolo[3,2-*b*] pyridine-2-carboxamide (composé n° 135 du tableau n° 3)
LCMS : $[MH]^+$ = 482
R.M.N. $^1H$ (DMSO $D_6$), δ (ppm): 10,75 (s, 1H); 8,4 (s, 1H) ; 8,31 (d, 1H) ; 8 (pic élargi, 1H) ; 7,78 (d, 1 H) ; 7,6 (s, 1 H) ; 7,19 (m, 1 H) ; 6,8 (dxd, 1H) ; 6,72 (m, 2H) ; 6,61 (d, 1H) ; 5,91 (s, 2H) ; 4,12 (m, 4H) ; 3,65 (s, 3H) ; 2,41 (m, 2H).

Trifluoroacétate (1 :1) du N-[6-(azétidin-1-yl)-pyridin-3-yl]-1-(4-méthoxybenzyl)-5-trifluorométhyl-1*H*-pyrrolo[3,2-*b*] pyridine-2-carboxamide (composé n° 138 du tableau n° 3)
LCMS : $[MH]^+$ = 482
R.M.N. $^1H$ (DMSO $D_6$), δ (ppm): 10,7 (s, 1H) ; 8,4 (s, 1H) ; 8,35 (d, 1H) ; 8,02 (pic élargi, 1 H) ; 7,78 (d, 1 H) ; 7,58 (s, 1 H) ; 7,11 (d, 2H) ; 6,84 (d, 2H) ; 6,75 (pic élargi, 1 H) ; 5,87 (s, 2H) ; 4,12 (m, 4H) ; 3,69 (s, 3H) ; 2,39 (m, 2H).

Trifluoroacétate (1:1) du *N*-[6-(3-hydroxyazétidin-1-yl)-pyridin-3-yl]-1-(3-trifluorométhylbenzyl)-1*H*-pyrrolo[2,3-*c*]pyridine-2-carboxamide (composé n° 164 du tableau n° 4)
LCMS : $[MH]^+$ = 468
R.M.N. $^1H$ (DMSO $D_6$), δ (ppm): 10,8 (s, 1H) ; 9,51 (s, 1H) ; 8,92 (s, 1H) ; 8,88 (s, 1H) ; 8,44 (d, 1 H) ; 8,32 (m, 2H) ; 7,85 (pic élargi, 1 H) ; 7,63 (m, 2H) ; 7,53 (m, 1 H) ; 7,39 (d, 1 H) ; 6,51 (pic élargi, 1 H) ; 6,09 (s, 2H) ; 4,58 (m, 1 H) ; 4,2 (m, 2H) ; 3,71 (m, 2H).

Trifluoroacétate (1 :1) du N-[6-(3-hydroxyazétidin-1-yl)-pyridin-3-yl]-1-(4-méthylbenzyl)-1*H*-pyrrolo[2,3-*c*]pyridine-2-carboxamide (composé n° 165 du tableau n° 4)
LCMS : $[MH]^+$ = 414
R.M.N. $^1H$ (DMSO $D_6$), δ (ppm): 10,75 (s, 1H) ; 8,93 (s, 1H) ; 8,9 (s, 1H) ; 8,35 (m, 3H) ; 7,88 (pic élargi, 1H) ; 7,59 (s, 1 H) ; 7,09 (m, 4H) ; 6,51 (pic élargi, 1 H) ; 5,95 (s, 2H) ; 4,59 (m, 1 H) ; 4,2 (m, 2H) ; 3,72 (m, 2H) ; 2,22 (s, 3H).

Les tableaux 2, 3 et 4 qui suivent illustrent les structures chimiques et les propriétés physiques de quelques composés de formule générale (I) selon l'invention.

Dans ces tableaux :

- la colonne « PF (°C) ou $[MH]^+$ » renseigne respectivement, soit les points de fusion des produits en degrés Celsius (°C), soit leur pic de masse en LC-MS ;
- dans la colonne « Sel/base », « - » représente un composé sous forme de base libre, alors que « HCl » représente un composé sous forme de chlorhydrate et « TFA » représente un composé sous forme de trifluoroacétate ; le rapport entre parenthèses est le rapport (acide:base) ;
- « $CH_3$ » correspond à un groupe méthyle, « Et » correspond à un groupeméthyle, « $CF_3$ » correspond à un groupe trifluorométhyle, «$NC_4H_8$ » à un groupe pyrrolidin-1-yle.

Tableau 2

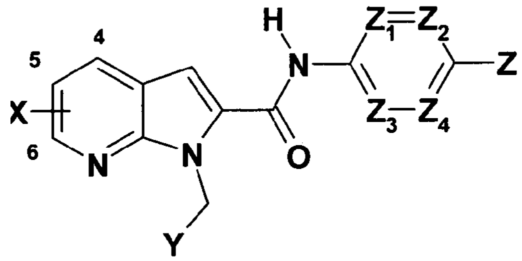

| N° | X | Y | $Z_1, Z_2, Z_3, Z_4$ | Z | sel | PF (°C) ou $[MH]^+$ |
|---|---|---|---|---|---|---|
| 1 | 5-$CF_3$ | 3-fluorophényl | CH,CH,CH,N | $NHCH_3$ | - | 252 - 254 |
| 2 | 5-$CF_3$ | 3-fluorophényl | CH,C($CH_3$),CH, N | $N(CH_3)_2$ | HCl 1:1. | 252 - 257 |
| 3 | 5-$CF_3$ | 3-fluorophényl | C($CH_3$),CH,CH, N | $N(CH_3)_2$ | HCl 1:1 | 242 - 247 |
| 4 | 5-$CF_3$ | 3-fluorophényl | CH,C($CF_3$),CH, N | $N(CH_3)_2$ | HCl 1:1 | 213 - 218 |
| 5 | 5-F | 3-fluorophényl | CH,CH,CH,N | $NC_4H_8$ | - | 239 - 240 |
| | | | | | HCl 2:3 | 241 - 242 |
| 6 | 5-$CF_3$ | 3-fluorophényl | CH,CH,CH,N | $NC_4H_8$ | - | 234 - 236 |
| 7 | 5-$CF_3$ | 3-fluorophényl | N,CH,CH,N | $NC_4H_8$ | - | 182 - 183 |
| 8 | 5-$CF_3$ | 3-fluorophényl | CH,CH,N,N | $N(CH_3)_2$ | - | 224 - 226 |
| 9 | 5-$CF_3$ | Pyridin-4-yl | CH,CH,CH,N | $NC_4H_8$ | - | 244 - 245 |
| 10 | 5-$CF_3$ | 3-fluorophényl | CH,CH,CH,N | $NHCH(CH_3)_2$ | - | 210 - 212 |
| 11 | 5-$CF_3$ | 3-fluorophényl | CH,CH,CH,N | 3-hydroxypyrrolidinyle | - | 233 - 235 |
| 12 | 5-$CF_3$ | 3-fluorophényl | CH,CH,CH,N | 3,3-difluoropyrrolidinyle | - | 211 - 213 |
| 13 | 5-$CF_3$ | 3-fluorophényl | CH,CH,CH,N | $N(CH_3)_2$ | - | 233 - 235 |
| 14 | 5-$CF_3$ | 3-fluorophényl | CH,CH,CH,N | cis-2,5-diméthylpyrrolidinyle | - | $[MH]^+$= 512 |
| 15 | 5-$CF_3$ | 3-fluorophényl | CH,CH,CH,N | azétidinyle | - | 243 - 244 |
| | | | | | HCl 1:1 | 230 - 235 |

(suite)

| N° | X | Y | $Z_1$, $Z_2$, $Z_3$, $Z_4$ | Z | sel | PF (°C) ou $[MH]^+$ |
|---|---|---|---|---|---|---|
| 17 | 5-$CF_3$ | 3-fluorophényl | CH,CH,CH,N | (S)-prolinyle | - | 235 - 237 |
| 18 | 5-$CF_3$ | 3-fluorophényl | CH,CH,CH,N | (*S*)-prolinyle ter-butyl ester | - | 106 - 108 |
| 19 | 5-$CF_3$ | 3-fluorophényl | CH,CH,CH,N | 3,3-difluoro azétidinyle | - | 222 - 224 |
| 20 | 5-$CF_3$ | 3-fluorophényl | CH,CH,CH,N | 3-hydroxy azétidinyle | - | 221 - 223 |
| 21 | 5-$CF_3$ | 3-fluorophényl | $C(CH_3)$,CH,CH, N | $N(CH_3)_2$ | - | 222 - 223 |
| 22 | 6-$CF_3$ | 3-fluorophényl | $C(CH_3)$,CH,CH, N | $NC_4H_8$ | - | 202 - 203 |
| 23 | 5-$CF_3$ | 3-fluorophényl | CH,CH,CH,N | 3-méthoxy pyrrolidinyle | - | 154 - 156 |
| 24 | 5-$CF_3$ | pyridin-4-yl | $C(CH_3)$,CH,CH, N | $N(CH_3)_2$ | - | 241 - 242 |
| 25 | 5-$CF_3$ | 3-fluorophényl | $C(CH_3)$,CH,CH, N | $NC_4H_8$ | - | 225 - 226 |
| 26 | 6-$CF_3$ | pyridin-4-yl | $C(CH_3)$,CH,CH, N | $NC_4H_8$ | - | 240 - 241 |
| 27 | 5-$CF_3$ | pyridin-4-yl | $C(CH_3)$,CH,CH, N | $NC_4H_8$ | - | 251 - 252 |
| 28 | 5-$CF_3$ | pyridin-4-yl | $C(CH_3)$,CH,CH, N | $NHCH_3$ | - | 230 - 231 |
| 29 | 5-$CF_3$ | pyridin-4-yl | $C(OCH_3)$,CH,CH , N | $NC_4H_8$ | - | 222 - 224 |
| 30 | 5-$CF_3$ | pyridin-4-yl | CH,CH,CH,N | azétidinyle | - | 233 - 235 |
| 31 | 5-$CF_3$ | 3-fluorophényl | CH,CH,CH,N | NHC(O) $CH_3$ | - | 282 - 284 |
| 32 | 5-$CF_3$ | 3-fluorophényl | CH,CH,CH,N | $NH_2$ | - | 230 - 232 |
| 33 | 6-$CF_3$ | 3-fluorophényl | CH,CH,CH,N | $NC_4H_8$ | - | 204 - 205 |
| 34 | 5-$CF_3$ | 3-fluorophényl | $C(CF_3)$,CH,CH, N | $NC_4H_8$ | - | 207 - 208 |
| 35 | 5-$CF_3$ | 3-fluorophényl | CH,C(F),CH,N | $N(CH_3)_2$ | - | 199 - 200 |
| 36 | 5-$CF_3$ | 3-fluorophényl | CH,N,CH,N | $N(CH_3)_2$ | - | 236 - 238 |
| 37 | 5-$CF_3$ | 3-fluorophényl | CH,CH,CH,N | 3-azabicyclo [3.2.0]heptyle | - | 172 - 174 |

(suite)

| N° | X | Y | $Z_1$, $Z_2$, $Z_3$, $Z_4$ | Z | sel | PF (°C) ou $[MH]^+$ |
|---|---|---|---|---|---|---|
| 38 | 5-$CF_3$ | 3-fluorophényl | CH,CH,CH,N | 3-azabicyclo [3.1.0]hexyle | - | 212 - 214 |
| 39 | 5-F | 3-fluorophényl | CH,CH,CH,N | 3-azabicyclo [3.1.0] hexyle | - | 222 - 224 |
| 40 | 5-$CF_3$ | 3-fluorophényl | CH,CH,CH,N | $NH(CH_2)_2CO_2H$ | - | 196 - 199 |
| 41 | 5-$CF_3$ | 3-fluorophényl | CH,CH,CH,N | $NH(CH_2)_3OH$ | - | 207 - 210 |
| 42 | 5-$CF_3$ | phényl | CH,CH,CH,N | azétidinyle | TFA 1:1 | $[MH]^+$ 452 |
| 43 | 5-$CF_3$ | 2-fluorophényl | CH,CH,CH,N | azétidinyle | TFA 1:1 | $[MH]^+$ 470 |
| 44 | 5-$CF_3$ | 4-fluorophényl | CH,CH,CH,N | azétidinyle | TFA 1:1 | $[M H]^+$ 470 |
| 45 | 5-$CF_3$ | 3-trifluoro méthylphényl | CH,CH,CH,N | azétidinyle | TFA 1:1 | [MH]+ 520 |
| 46 | 5-$CF_3$ | 4-méthylphényl | CH,CH,CH,N | azétidinyle | TFA 1:1 | $[MH]^+$ 466 |
| 47 | 5-$CF_3$ | 3-méthylphényl | CH,CH,CH,N | azétidinyle | TFA 1:1 | $[MH]^+$ 466 |
| 48 | 5-$CF_3$ | 3-méthoxy phényl | CH,CH,CH,N | azétidinyle | TFA 1:1 | $[MH]^+$ 482 |
| 49 | 5-$CF_3$ | 3-chlorophényl | CH,CH,CH,N | azétidinyle | TFA 1:1 | $[MH]^+$ 486 |
| 50 | 5-$CF_3$ | 3-trifluoro méthoxyphényl | CH,CH,CH,N | azétidinyle | TFA 1:1 | $[MH]^+$ 536 |
| 51 | 5-$CF_3$ | 4-méthoxyphényl | CH,CH,CH,N | azétidinyle | TFA 1:1 | $[MH]^+$ 482 |
| 52 | 5-$CF_3$ | 3-chloro-5-trifluoro méthylphényl | CH,CH,CH,N | azétidinyle | TFA 1:1 | $[MH]^+$ 554 |
| 53 | 5-F | phényl | CH,CH,CH,N | azétidinyle | TFA 1:1 | $[MH]^+$ 402 |
| 54 | 5-F | 2-fluorophényl | CH,CH,CH,N | azétidinyle | TFA 1:1 | $[MH]^+$ 420 |
| 55 | 5-F | 4-fluorophényl | CH,CH,CH,N | azétidinyle | TFA 1:1 | $[MH]^+$ 420 |
| 56 | 5-F | 3-trifluoro méthylphényl | CH,CH,CH,N | azétidinyle | TFA 1:1 | $[MH]^+$ 470 |
| 57 | 5-F | 4-Méthylphényl | CH,CH,CH,N | azétidinyle | TFA 1:1 | $[MH]^+$ 416 |
| 58 | 5-F | 3-méthylphényl | CH,CH,CH,N | azétidinyle | TFA 1:1 | $[MH]^+$ 416 |

(suite)

| N° | X | Y | $Z_1$, $Z_2$, $Z_3$, $Z_4$ | Z | sel | PF (°C) ou $[MH]^+$ |
|---|---|---|---|---|---|---|
| 59 | 5-F | 3-méthoxy phényl | CH,CH,CH,N | azétidinyle | TFA 1:1 | $[MH]^+$ 432 |
| 60 | 5-F | 3-chlorophényl | CH,CH,CH,N | azétidinyle | TFA 1:1 | $[MH]^+$ 436 |
| 61 | 5-F | 3-trifluoro méthoxyphényl | CH,CH,CH,N | azétidinyle | TFA 1:1 | $[MH]^+$ 486 |
| 62 | 5-F | 4-méthoxy phényl | CH,CH,CH,N | azétidinyle | TFA 1:1 | $[MH]^+$ 432 |
| 63 | 5-F | 3-chloro-5-trifluoro méthylphényl | CH,CH,CH,N | azétidinyle | TFA 1:1 | $[MH]^+$ 504 |
| 64 | 6- $CF_3$ | phényl | CH,CH,CH,N | azétidinyle | TFA 1:1 | $[MH]^+$ 452 |
| 65 | 6- $CF_3$ | 2-fluorophényl | CH,CH,CH,N | azétidinyle | TFA 1:1 | $[MH]^+$ 470 |
| 66 | 6- $CF_3$ | 4-fluorophényl | CH,CH,CH,N | azétidinyle | TFA 1:1 | $[MH]^+$ 470 |
| 67 | 6- $CF_3$ | 3-trifluoro méthylphényl | CH,CH,CH,N | azétidinyle | TFA 1:1 | $[MH]^+$ 520 |
| 68 | 6- $CF_3$ | 4-méthylphényl | CH,CH,CH,N | azétidinyle | TFA 1:1 | $[MH]^+$ 466 |
| 69 | 6- $CF_3$ | 3-méthylphényl | CH,CH,CH,N | azétidinyle | TFA 1:1 | $[MH]^+$ 466 |
| 70 | 6- $CF_3$ | 3-méthoxy phényl | CH,CH,CH,N | azétidinyle | TFA 1:1 | $[MH]^+$ 482 |
| 71 | 6- $CF_3$ | 3-chlorophényl | CH,CH,CH,N | azétidinyle | TFA 1:1 | $[MH]^+$ 486 |
| 72 | 6- $CF_3$ | 3-trifluoro méthoxyphényl | CH,CH,CH,N | azétidinyle | TFA 1:1 | $[MH]^+$ 536 |
| 73 | 6- $CF_3$ | 4-méthoxy phényl | CH,CH,CH,N | azétidinyle | TFA 1:1 | $[MH]^+$ 482 |
| 74 | 6- $CF_3$ | 3-chloro-5-trifluoro méthylphényl | CH,CH,CH,N | azétidinyle | TFA 1:1 | $[MH]^+$ 554 |
| 75 | H | phényl | CH,CH,CH,N | azétidinyle | TFA 1:1 | $[MH]^+$ 384 |
| 76 | H | 2-fluorophényl | CH,CH,CH,N | azétidinyle | TFA 1:1 | $[MH]^+$ 402 |
| 77 | H | 4-fluorophényl | CH,CH,CH,N | azétidinyle | TFA 1:1 | $[MH]^+$ 402 |
| 78 | H | 3-trifluoro méthylphényl | CH,CH,CH,N | azétidinyle | TFA 1:1 | $[MH]^+$ 452 |
| 79 | H | 4-Méthylphényl | CH,CH,CH,N | azétidinyle | TFA 1:1 | $[MH]^+$ 398 |

(suite)

| N° | X | Y | $Z_1$, $Z_2$, $Z_3$, $Z_4$ | Z | sel | PF (°C) ou $[MH]^+$ |
|---|---|---|---|---|---|---|
| 80 | H | 3-Méthylphényl | CH,CH,CH,N | azétidinyle | TFA 1:1 | $[MH]^+$ 398 |
| 81 | H | 3-méthoxy phényl | CH,CH,CH,N | azétidinyle | TFA 1:1 | $[MH]^+$ 414 |
| 82 | H | 3-chlorophényl | CH,CH,CH,N | azétidinyle | TFA 1:1 | $[MH]^+$ 418 |
| 83 | H | 3-trifluoro méthoxyphényl | CH,CH,CH,N | azétidinyle | TFA 1:1 | $[MH]^+$ 468 |
| 84 | H | 4-méthoxy phényl | CH,CH,CH,N | azétidinyle | TFA 1:1 | $[MH]^+$ 414 |
| 85 | H | 3-chloro-5-trifluoro méthylphényl | CH,CH,CH,N | azétidinyle | TFA 1:1 | $[MH]^+$ 486 |
| 86 | 5- $CF_3$ | 2-fluorophényl | CH,CH,CH,N | 3-hydroxy azétidinyle | TFA 1:1 | $[MH]^+$ 486 |
| 87 | 5- $CF_3$ | 4-fluorophényl | CH,CH,CH,N | 3-hydroxy azétidinyle | TFA 1:1 | $[MH]^+$ 486 |
| 88 | 5- $CF_3$ | 3-trifluoro méthylphényl | CH,CH,CH,N | 3-hydroxy azétidinyle | TFA 1:1 | $[MH]^+$ 536 |
| 89 | 5- $CF_3$ | 4-méthylphényl | CH,CH,CH,N | 3-hydroxy azétidinyle | TFA 1:1 | $[MH]^+$ 482 |
| 90 | 5- $CF_3$ | 3-méthylphényl | CH,CH,CH,N | 3-hydroxy azétidinyle | TFA 1:1 | $[MH]^+$ 482 |
| 91 | 5- $CF_3$ | 3-méthoxy phényl | CH,CH,CH,N | 3-hydroxy azétidinyle | TFA 1:1 | $[MH]^+$ 498 |
| 92 | 5- $CF_3$ | 3-chlorophényl | CH,CH,CH,N | 3-hydroxy azétidinyle | TFA 1:1 | $[MH]^+$ 502 |
| 93 | 5- $CF_3$ | 3-trifluoro méthoxyphényl | CH,CH,CH,N | 3-hydroxy azétidinyle | TFA 1:1 | $[MH]^+$ 552 |
| 94 | 5- $CF_3$ | 4-méthoxy phényl | CH,CH,CH,N | 3-hydroxy azétidinyle | TFA 1:1 | $[MH]^+$ 498 |
| 95 | 5- $CF_3$ | 3-chloro-5-trifluoro méthylphényl | CH,CH,CH,N | 3-hydroxy azétidinyle | TFA 1:1 | $[MH]^+$ 570 |
| 96 | 6- $CF_3$ | phényl | CH,CH,CH,N | 3-hydroxy azétidinyle | TFA 1:1 | $[MH]^+$ 468 |
| 97 | 6- $CF_3$ | 2-fluorophényl | CH,CH,CH,N | 3-hydroxy azétidinyle | TFA 1:1 | $[MH]^+$ 486 |
| 98 | 6- $CF_3$ | 4-fluorophényl | CH,CH,CH,N | 3-hydroxy azétidinyle | TFA 1:1 | $[MH]^+$ 486 |
| 99 | 6- $CF_3$ | 3-trifluoro méthylphényl | CH,CH,CH,N | 3-hydroxy azétidinyle | TFA 1:1 | $[MH]^+$ 536 |
| 100 | 6- $CF_3$ | 4-Méthylphényl | CH,CH,CH,N | 3-hydroxy azétidinyle | TFA 1:1 | $[MH]^+$ 482 |

(suite)

| N° | X | Y | $Z_1$, $Z_2$, $Z_3$, $Z_4$ | Z | sel | PF (°C) ou $[MH]^+$ |
|---|---|---|---|---|---|---|
| 101 | 6- $CF_3$ | 3-Méthylphényl | CH,CH,CH,N | 3-hydroxy azétidinyle | TFA 1:1 | $[MH]^+$ 482 |
| 102 | 6- $CF_3$ | 3-méthoxyphényl | CH,CH,CH,N | 3-hydroxy azétidinyle | TFA 1:1 | $[MH]^+$ 498 |
| 103 | 6- $CF_3$ | 3-chlorophényl | CH,CH,CH,N | 3-hydroxy azétidinyle | TFA 1:1 | $[MH]^+$ 502 |
| 104 | 6- $CF_3$ | 3-trifluoro méthoxyphényl | CH,CH,CH,N | 3-hydroxy azétidinyle | TFA 1:1 | $[MH]^+$ 552 |
| 105 | 6- $CF_3$ | 4-méthoxyphényl | CH,CH,CH,N | 3-hydroxy azétidinyle | TFA 1:1 | $[MH]^+$ 498 |
| 106 | 6- $CF_3$ | 3-chloro-5-trifluoro méthylphényl | CH,CH,CH,N | 3-hydroxy azétidinyle | TFA 1:1 | $[MH]^+$ 570 |
| 107 | 5-F | phényl | CH,CH,CH,N | 3-hydroxy azétidinyle | TFA 1:1 | $[MH]^+$ 418 |
| 108 | 5-F | 2-fluorophényl | CH,CH,CH,N | 3-hydroxy azétidinyle | TFA 1:1 | $[MH]^+$ 436 |
| 109 | 5-F | 4-fluorophényl | CH,CH,CH,N | 3-hydroxy azétidinyle | TFA 1:1 | $[MH]^+$ 436 |
| 110 | 5-F | 3-trifluoro méthylphényl | CH,CH,CH,N | 3-hydroxy azétidinyle | TFA 1:1 | $[MH]^+$ 486 |
| 111 | 5-F | 4-méthylphényl | CH,CH,CH,N | 3-hydroxyl azétidinyle | TFA 1:1 | $[MH]^+$ 432 |
| 112 | 5-F | 3-méthylphényl | CH,CH,CH,N | 3-hydroxy azétidinyle | TFA 1:1 | $[MH]^+$ 432 |
| 113 | 5-F | 3-méthoxy phényl | CH,CH,CH,N | 3-hydroxy azétidinyle | TFA 1:1 | $[MH]^+$ 448 |
| 114 | 5-F | 3-chlorophényl | CH,CH,CH,N | 3-hydroxy azétidinyle | TFA 1:1 | $[MH]^+$ 452 |
| 115 | 5-F | 3-trifluoro méthoxyphényl | CH,CH,CH,N | 3-hydroxy azétidinyle | TFA 1:1 | $[MH]^+$ 502 |
| 116 | 5-F | 4-méthoxy phényl | CH,CH,CH,N | 3-hydroxy azétidinyle | TFA 1:1 | $[MH]^+$ 448 |
| 117 | 5-F | 3-chloro-5-trifluoro méthylphényl | CH,CH,CH,N | 3-hydroxy azétidinyle | TFA 1:1 | $[MH]^+$ 520 |
| 118 | H | phényl | CH,CH,CH,N | 3-hydroxy azétidinyle | TFA 1:1 | $[MH]^+$ 400 |
| 119 | H | 2-fluorophényl | CH,CH,CH,N | 3-hydroxy azétidinyle | TFA 1:1 | $[MH]^+$ 418 |
| 120 | H | 4-fluorophényl | CH,CH,CH,N | 3-hydroxy azétidinyle | TFA 1:1 | $[MH]^+$ 418 |
| 121 | H | 3-trifluoro méthylphényl | CH,CH,CH,N | 3-hydroxy azétidinyle | TFA 1:1 | $[MH]^+$ 468 |

(suite)

| N° | X | Y | $Z_1$, $Z_2$, $Z_3$, $Z_4$ | Z | sel | PF (°C) ou $[MH]^+$ |
|---|---|---|---|---|---|---|
| 122 | H | 4-méthylphényl | CH,CH,CH,N | 3-hydroxyl azétidinyle | TFA 1:1 | $[MH]^+$ 414 |
| 123 | H | 3-méthylphényl | CH,CH,CH,N | 3-hydroxy azétidinyle | TFA 1:1 | $[MH]^+$ 414 |
| 124 | H | 3-méthoxy phényl | CH,CH,CH,N | 3-hydroxy azétidinyle | TFA 1:1 | $[MH]^+$ 430 |
| 125 | H | 3-chlorophényl | CH,CH,CH,N | 3-hydroxy azétidinyle | TFA 1:1 | $[MH]^+$ 434 |
| 126 | H | 3-trifluoro méthoxyphényl | CH,CH,CH,N | 3-hydroxy azétidinyle | TFA 1:1 | $[MH]^+$ 484 |
| 127 | H | 4-méthoxy phényl | CH,CH,CH,N | 3-hydroxy azétidinyle | TFA 1:1 | $[MH]^+$ 430 |
| 128 | H | 3-chloro-5-trifluoro méthylphényl | CH,CH,CH,N | 3-hydroxy azétidinyle | TFA 1:1 | $[MH]^+$ 502 |
| 171 | 5-$CF_3$ | 3-fluorophényl | CH,CH,CH,N | $NH(CH_2)_2CO_2Et$ | - | $[MH]^+$ 530 |
| 172 | 5-$CF_3$ | 3-fluorophényl | CH,CH,CH,N | 3-(méthoxy) azétidinyle | - | 185 - 187 |
| 173 | 5-$CF_3$ | 3-fluorophényl | CH,CH,CH,N | 3-(cyclopropyl méthyloxy) azétidinyle | - | 201 - 203 |

Tableau 3

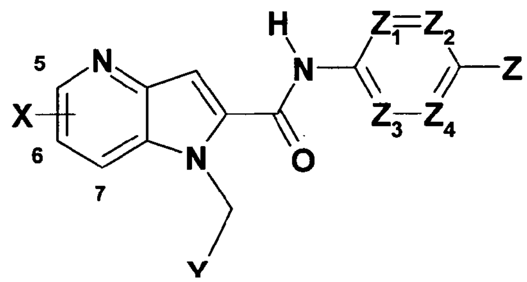

| N° | X | Y | $Z_1$, $Z_2$, $Z_3$, $Z_4$ | Z | sel | PF (°C) |
|---|---|---|---|---|---|---|
| 16 | 5-$CF_3$ | 3-fluorophényl | CH,CH,CH,N | $NC_4H_8$ | - | 219 - 220 |
| 129 | 5- $CF_3$ | phényl | CH,CH,CH,N | azétidinyle | TFA 1:1 | $[MH]^+$ 452 |
| 130 | 5- $CF_3$ | 2-fluorophényl | CH,CH,CH,N | azétidinyle | TFA 1:1 | $[MH]^+$ 470 |
| 131 | 5- $CF_3$ | 4-fluorophényl | CH,CH,CH,N | azétidinyle | TFA 1:1 | $[MH]^+$ 470 |
| 132 | 5- $CF_3$ | 3-trifluoro méthylphényl | CH,CH,CH,N | azétidinyle | TFA 1:1 | $[MH]^+$ 520 |
| 133 | 5- $CF_3$ | 4-méthylphényl | CH,CH,CH,N | azétidinyle | TFA 1:1 | $[MH]^+$ 466 |
| 134 | 5- $CF_3$ | 3-méthylphényl | CH,CH,CH,N | azétidinyle | TFA 1:1 | $[MH]^+$ 466 |
| 135 | 5- $CF_3$ | 3-méthoxyphényl | CH,CH,CH,N | azétidinyle | TFA 1:1 | $[MH]^+$ 482 |
| 136 | 5- $CF_3$ | 3-chlorophényl | CH,CH,CH,N | azétidinyle | TFA 1:1 | $[MH]^+$ 486 |

(suite)

| N° | X | Y | $Z_1$, $Z_2$, $Z_3$, $Z_4$ | Z | sel | PF (°C) |
|---|---|---|---|---|---|---|
| 137 | 5- $CF_3$ | 3-trifluoro méthoxyphényl | CH,CH,CH,N | azétidinyle | TFA 1:1 | $[MH]^+$ 536 |
| 138 | 5- $CF_3$ | 4-méthoxyphényl | CH,CH,CH,N | azétidinyle | TFA 1:1 | $[MH]^+$ 482 |
| 139 | 5- $CF_3$ | 3-chloro-5-trifluoro méthylphényl | CH,CH,CH,N | azétidinyle | TFA 1:1 | $[MH]^+$ 554 |
| 140 | 5- $CF_3$ | Phényl | CH,CH,CH,N | 3-hydroxy azétidinyle | TFA 1:1 | $[MH]^+$ 468 |
| 141 | 5- $CF_3$ | 2-fluorophényl | CH,CH,CH,N | 3-hydroxy azétidinyle | TFA 1:1 | $[MH]^+$ 486 |
| 142 | 5- $CF_3$ | 4-fluorophényl | CH,CH,CH,N | 3-hydroxy azétidinyle | TFA 1:1 | $[MH]^+$ 486 |
| 143 | 5- $CF_3$ | 3-trifluoro méthylphényl | CH,CH,CH,N | 3-hydroxy azétidinyle | TFA 1:1 | $[MH]^+$ 536 |
| 144 | 5- $CF_3$ | 4-méthylphényl | CH,CH,CH,N | 3-hydroxy azétidinyle | TFA 1:1 | $[MH]^+$ 482 |
| 145 | 5- $CF_3$ | 3-méthylphényl | CH,CH,CH,N | 3-hydroxy azétidinyle | TFA 1:1 | $[MH]^+$ 482 |
| 146 | 5- $CF_3$ | 3-méthoxyphényl | CH,CH,CH,N | 3-hydroxy azétidinyle | TFA 1:1 | $[MH]^+$ 498 |
| 147 | 5- $CF_3$ | 3-chlorophényl | CH,CH,CH,N | 3-hydroxy azétidinyle | TFA 1:1 | $[MH]^+$ 502 |
| 148 | 5- $CF_3$ | 3-trifluoro méthoxyphényl | CH,CH,CH,N | 3-hydroxy azétidinyle | TFA 1:1 | $[MH]^+$ 552 |
| 149 | 5- $CF_3$ | 4-méthoxyphényl | CH,CH,CH,N | 3-hydroxy azétidinyle | TFA 1:1 | $[MH]^+$ 498 |
| 150 | 5- $CF_3$ | 3-chloro-5-trifluoro méthylphényl | CH,CH,CH,N | 3-hydroxy azétidinyle | TFA 1:1 | $[MH]^+$ 570 |

Tableau 4

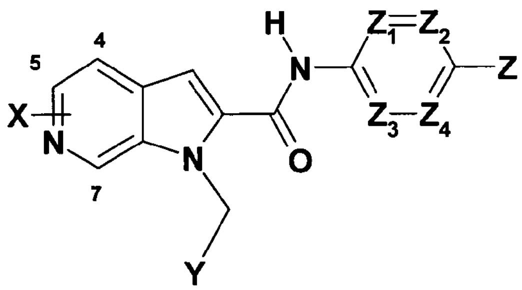

| N° | X | Y | $Z_1$, $Z_2$, $Z_3$, $Z_4$ | Z | sel | PF (°C) |
|---|---|---|---|---|---|---|
| 151 | H | phényl | CH,CH,CH,N | azétidinyle | TFA 1:1 | $[MH]^+$ 384 |
| 152 | H | 2-fluorophényl | CH,CH,CH,N | azétidinyle | TFA 1:1 | $[MH]^+$ 402 |
| 153 | H | 4-fluorophényl | CH,CH,CH,N | azétidinyle | TFA 1:1 | $[MH]^+$ 402 |
| 154 | H | 3-trifluoro méthylphényl | CH,CH,CH,N | azétidinyle | TFA 1:1 | $[MH]^+$ 452 |
| 155 | H | 4-méthylphényl | CH,CH,CH,N | azétidinyle | TFA 1:1 | $[MH]^+$ 398 |
| 156 | H | 3-méthylphényl | CH,CH,CH,N | azétidinyle | TFA 1:1 | $[MH]^+$ 398 |
| 157 | H | 3-méthoxy phényl | CH,CH,CH,N | azétidinyle | TFA 1:1 | $[MH]^+$ 414 |
| 158 | H | 3-chlorophényl | CH,CH,CH,N | azétidinyle | TFA 1:1 | $[MH]^+$ 418 |
| 159 | H | 3-trifluoro méthoxyphényl | CH,CH,CH,N | azétidinyle | TFA 1:1 | $[MH]^+$ 468 |
| 160 | H | 3-chloro-5-trifluoro méthylphényl | CH,CH,CH,N | azétidinyle | TFA 1:1 | $[MH]^+$ 486 |
| 161 | H | phényl | CH,CH,CH,N | 3-hydroxy azétidinyle | TFA 1:1 | $[MH]^+$ 400 |

(suite)

| N° | X | Y | $Z_1, Z_2, Z_3, Z_4$ | Z | sel | PF (°C) |
|---|---|---|---|---|---|---|
| 162 | H | 2-fluorophényl | CH,CH,CH,N | 3-hydroxy azétidinyle | TFA 1:1 | $[MH]^+$ 418 |
| 163 | H | 4-fluorophényl | CH,CH,CH,N | 3-hydroxy azétidinyle | TFA 1:1 | $[MH]^+$ 418 |
| 164 | H | 3-trifluoro méthylphényl | CH,CH,CH,N | 3-hydroxy azétidinyle | TFA 1:1 | $[MH]^+$ 468 |
| 165 | H | 4-méthylphényl | CH,CH,CH,N | 3-hydroxy azétidinyle | TFA 1:1 | $[MH]^+$ 414 |
| 166 | H | 3-méthylphényl | CH,CH,CH,N | 3-hydroxy azétidinyle | TFA 1:1 | $[MH]^+$ 414 |
| 167 | H | 3-méthoxy phényl | CH,CH,CH,N | 3-hydroxy azétidinyle | TFA 1:1 | $[MH]^+$ 430 |
| 168 | H | 3-chlorophényl | CH,CH,CH,N | 3-hydroxy azétidinyle | TFA 1:1 | $[MH]^+$ 434 |
| 169 | H | 3-trifluoro méthoxyphényl | CH,CH,CH,N | 3-hydroxy azétidinyle | TFA 1:1 | $[MH]^+$ 484 |
| 170 | H | 3-chloro-5-trifluoro méthylphényl | CH,CH,CH,N | 3-hydroxy azétidinyle | TFA 1:1 | $[MH]^+$ 502 |

Les composés de l'invention ont été soumis à des essais pharmacologiques *in vitro* et *in vivo* qui ont mis en évidence leur intérêt comme substances à activités thérapeutiques.

Les composés de l'invention présentent également des caractéristiques de solubilité dans l'eau qui favorise une bonne activité *in vivo.*

Test d'inhibition du courant induit par la capsaïcine sur les DRG de rat

- Culture primaire de cellules de ganglions de racine dorsale (DRG) de rat :

Les neurones du DRG expriment naturellement le récepteur TRPV1.

Les cultures primaires de DRG de rats nouveaux nés sont préparées à partir de ratons de 1 jour. Brièvement, après dissection, les ganglions sont trypsinés et les cellules dissociées mécaniquement par trituration ménagée. Les cellules sont re-suspendues dans un milieu de culture basal Eagle contenant 10 % de sérum de veau foetal, 25 mM KCl, 2 mM glutamine, 100 μg/ml gentamicine et 50 ng/ml de NGF, puis déposées sur des lamelles de verre recouvertes de laminine (0.25 x 106 cellules par lamelle) qui sont ensuite placées dans des boîtes 12 puits Corning. Les cellules sont incubées à 37°C en atmosphère humidifiée contenant 5% de $CO_2$ et 95% d'air. De la cytosine β-D-arabinoside (1 μM) est ajoutée 48 h après la mise en culture, pour prévenir le développement des cellules non neuronales. Les lamelles sont transférées dans les chambres expérimentales pour les études de patch-clamp après 7-10 jours de culture.

- Electrophysiologie :

Les chambres de mesure (volume 800 μl) contenant la préparation cellulaire sont placées sur la platine d'un microscope inversé (Olympus IMT2) équipé d'optiques Hoffman (Modulation Contrast, New York) et observées au grossissement de 400X. Les chambres sont continuellement perfusées par gravité (2,5 ml/min) à l'aide d'un distributeur de solutions acceptant 8 entrées et dont lasortie unique, constituée par un tube de polyéthylène (ouverture 500μm) est placée à moins de 3 mm de la cellule étudiée. La configuration "cellule entière" de la technique de patch-clamp à été utilisée.

Les pipettes en verre borosilicaté (résistance 5-10 MOhms) sont approchées de la cellule grâce à un micromanipulateur piézoélectrique 3D ( Burleigh, PC1000) . Les courants globaux (potentiel de membrane fixé à -60 mV) sont enregistrés avec un amplificateur Axopatch 1 D (Axon Instruments, Foster city, Californie), connecté à un PC piloté par les logiciels de Pclamp8 (Axon Instrument). Les traces de courant sont enregistrées sur papier et simultanément digitalisées (fréquence d'échantillonnage 15 à 25 Hz) et acquises sur le disque dur du PC.

L'application d'une solution micromolaire de capsaïcine , provoque sur les cellules de DRG (voltage fixé à -70 mV) un courant cationique entrant. Afin de minimiser la désensibilisation des récepteurs, l'intervalle d'une minute minimum entre deux applications de capsaïcine est respecté. Après une période contrôle (stabilisation de la réponse capsaïcine seule), les composés à tester sont appliqués seuls à une concentration donnée (concentration de 10 nM ou de 0,1 nM) pendant une durée de 4 à 5 minutes, au cours desquelles plusieurs tests capsaïcine + composé sont réalisés (obtention de l'inhibition maximale). Les résultats sont exprimés en % d'inhibition de la réponse capsaïcine contrôle.

Les pourcentages d'inhibition de la réponse capsaïcine (1 microM) sont compris entre 20% et 100% pour les

composés les plus actifs de l'invention testés à la concentration de 10 nM à 0,1 nM (voir exemple dans le tableau 5).

Les composés de l'invention sont donc des antagonistes efficaces in vitro des récepteurs de type TRPV1.

Tableau 5

| N°composé | % inhibition en patch DRG |
|---|---|
| 4 | 98% (1 nM) |
| 6 | 71% (1 nM) |
| 15 | 100% (10 nM) |

Test d'irritation cornéenne souris

Le caractère irritant de la capsaïcine est aisément apprécié au niveau de la cornée puisque cet organe est un des plus innervé par les fibres C. Dans ce contexte, d'après des expériences préliminaires, l'application d'une très faible quantité de capsaïcine (2 µl à une concentration de 160 µM) à la surface de la cornée d'un animal entraîne un certain nombre de comportements stéréotypés liés à l'irritation et qu'il est facile de répertorier. Parmi ceux-ci, on note : clignement de l'oeil, frottement de l'oeil instillé par la patte avant ipsilatérale, frottement de la face avec les deux pattes avant, grattement de la face ipsilatérale par la patte arrière. La durée de ces comportements ne dépasse pas les 2 minutes d'observation, et l'animal reprend alors son activité normale. Son aspect est par ailleurs également normal. La souris n'est pas recluse dans un coin avec les poils hérissés et ne développe aucun signe observable de souffrance. On peut en conclure que la durée d'action de la capsaïcine à ces doses est inférieure à 2 minutes.

Résumé de la méthodologie :

Le principe de la série d'expériences est de déterminer si les composés de l'invention peuvent influencer la réponse comportementale induite par une quantité donnée de capsaïcine. La capsaïcine est initialement diluée à 25 mM dans le DMSO et diluée, pour son utilisation finale, dans le sérum physiologique avec du Tween 80 à 10%. Il apparaît, à partir d'études contrôles que dans ces conditions, le solvant n'a aucun effet.

En pratique, le produit à tester, préparé à 25 mM dans le DMSO et dilué pour son utilisation finale dans le sérum physiologique avec 10% de Tween 80 à la plus forte concentration de 500 µM, est administré en application locale à la surface de la cornée sous un volume de 2 µl, 10 minutes avant l'application de la capsaicine. L'animal reçoit l'instillation oculaire de 2 µl d'une solution de capsaïcine à 160 µM préparée comme indiqué ci-dessus. Au cours d'une observation de 2 minutes suivant l'instillation, le nombre de frottements de l'oeil instillé par la patte antérieure ipsilatérale est compté pour chaque animal.

Pour un groupe donné, le pourcentage de protection est calculé comme suit :

P= 100 – ((nombre moyen de grattages du groupe traité par le composé / nombre moyen de grattages du groupe traité par le solvant) x 100)

Ce pourcentage de protection est moyenné pour chaque groupe d'animaux (n = nombre d'animaux testés avec le composé de l'invention).

Les pourcentages de protection évalués, dans ce modèle, pour les composés de l'invention les plus actifs, utilisés à la concentration de 500µM, sont compris entre 20% et 100% (voir exemple dans le tableau 6 :

Tableau 6

| n°composé | % P 500µM |
|---|---|
| 1 | 51% |
| 3 | 34% |
| 6 | 30% |
| 9 | 43% |
| 12 | 83% |

Les résultats de ces essais montrent que les composés les plus actifs de l'invention bloquent les effets induits par la stimulation des récepteurs TRPV1.

Les composés de l'invention peuvent donc être utilisés pour la préparation de médicaments, notamment pour la préparation d'un médicament destiné à prévenir ou à traiter les pathologies dans lesquelles les récepteurs de type TRPV1 sont impliqués.

Ainsi, selon un autre de ses aspects, l'invention a pour objet des médicaments qui comprennent un composé de formule (1) selon l'invention, ou un sel pharmaceutiquement acceptable, ou encore un hydrate ou un solvat dudit composé.

Ces médicaments trouvent leur emploi en thérapeutique, notamment dans la prévention et/ou le traitement de la douleur et de l'inflammation, de la douleur chronique, neuropathique (traumatique, diabétique, métabolique, infectieuse, toxique, induite par un traitement anticancéreux ou hiatrogène), (ostéo-) arthritique, rhumatismale, des fibromyalgies, de la douleur du dos, de la douleur liée au cancer, de la névralgie faciale, des céphalées, de la migraine, de la douleur dentaire, de la brûlure, du coup de soleil, de la morsure ou de la piqûre, de la nevralgie post-herpétique, de la douleur musculaire, de la compression nerveuse (centrale et/ou périphérique), des traumatismes de la moelle et/ou du cerveau, de l'ischémie (de la moelle et/ou du cerveau), de la neurodégénération, des accidents vasculaires hémorragiques (de la moelle et/ou du cerveau), de la douleur post-stroke.

Les composés de l'invention peuvent être utilisés pour la préparation d'un médicament destiné à prévenir et/ou à traiter les désordres urologiques tels que l'hyperactivité de la vessie, l'hyperéflexie vésicale, l'instabilité vésicale, l'incontinence, la miction d'urgence, l'incontinence urinaire, la cystite, la colique néphrétique, l'hypersensibilité pelvienne et la douleur pelvienne.

Les composés de l'invention peuvent être utilisés pour la préparation d'un médicament destiné à prévenir et/ou à traiter les désordres gynécologiques comme la vulvodynie, les douleurs liées aux salpingites, aux dysménorrhées.

On peut également utiliser ces produits pour la préparation d'un médicament destiné à prévenir et/ou à traiter les désordres gastrointestinaux tels que le désordre du réflexe gastroesophagique, l'ulcère de l'estomac, l'ulcère du duodénum, la dyspepsie fonctionnelle, la colite, l'IBS, la maladie de Crohn, la pancréatite, l'oesophagite, la colique hépatique.

Les composés de l'invention peuvent également être utilisés pour la préparation d'un médicament destiné à prévenir et/ou traiter les désordres métaboliques tels que le diabète.

De même, les produits de la présente invention peuvent être utiles dans la prévention et/ou le traitement des désordres respiratoires tels que l'asthme, la toux, la COPD, la bronchoconstriction et les désordres inflammatoires. Ces produits peuvent également être utilisés pour prévenir et/ou traiter le psoriasis, le pruritis, les irritations dermiques, des yeux ou des muqueuses, l'herpès, le zona.

Les composés de l'invention peuvent également être utilisés pour la préparation d'un médicament destiné à traiter la dépression.

Selon un autre de ses aspects, la présente invention concerne des compositions pharmaceutiques comprenant, en tant que principe actif, un composé selon l'invention.

Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable. Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) selon l'invention ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies citées ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |

(suite)

| | |
|---|---|
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,001 à 30 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

**1.** Composé répondant à la formule (I)

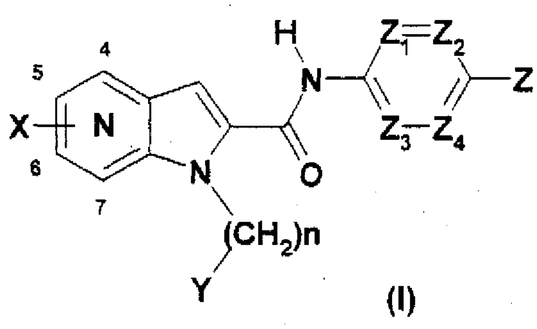

(I)

dans laquelle
le noyau pyrrolopyridine est un groupe pyrrolo[3,2-*b*]pyridine, un groupe pyrrolo[2,3-*c*]pyridine ou un groupe pyrrolo [2,3-*b*]pyridine ; le noyau pyridine étant éventuellement substitué en position 4, 5, 6 et/ou 7 par un ou plusieurs substituants X
le ou les substituents X, identiques ou différents l'un de l'autre, sont choisis parmi un atome d'hydrogène ou d'halogène ou un groupe $C_1$-$C_6$-fluoroalkyle ;
n est égal à 1 ;
Y représente un phènyle ou un pyridinyle, le phènyle ou le pyridinyle étant éventuellement substitué par un ou plusieurs groupes choisis parmi un atome d'halogène ou un groupe $C_1$-$C_6$-alkyle, $C_1$-$C_6$-fluoroalkyle, $C_1$-$C_6$-alcoxyle ou $C_1$-$C_6$-fluoroalcoxyle ;
$Z_1$, $Z_2$, $Z_3$, $Z_4$ représentent, indépendamment l'un de l'autre, un atome d'azote ou un groupe $C(R_6)$, l'un au moins correspondant à un atome d'azote et l'un au moins correspondant à un groupe $C(R_6)$ ;
$R_6$ représente un atome d'hydrogène ou d'halogène ou un groupe $C_1$-$C_6$-alkyle, $C_1$-$C_6$-fluoroalkyle ou $C_1$-$C_6$-alcoxyle ;
Z représente
soit une amine cyclique reliée par l'atome d'azote, de formule :

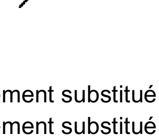

dans laquelle
A représente un groupe $C_1$-$C_7$-alkylène éventuellement substitué par un ou deux groupes $R_8$ ;
B représente un groupe $C_1$-$C_7$-alkylène éventuellement substitué par un ou deux groupes $R_9$ ;

L représente une liaison ;
les atomes de carbone de l'amine cyclique Z étant éventuellement substitués par un ou plusieurs groupes $R_{12}$ identiques ou différents l'un de l'autre ; $R_{12}$ représente un atome de fluor, un groupe $C_1$-$C_6$-alkyle, $C_1$-$C_6$-alcoxyle, $C_3$-$C_7$-cycloalkyle-$C_1$-$C_6$-alkylène-O-, $CO_2H$, C(O)O-$C_1$-$C_6$-alkyle ou hydroxyle ;
$R_8$ et $R_9$ sont définis tels que :
deux groupes $R_8$ peuvent former ensemble une liaison ou un groupe $C_1$-$C_6$-alkylène ; deux groupes $R_9$ peuvent former ensemble une liaison ou un groupe $C_1$-$C_6$-alkylène ; $R_8$ et $R_9$ peuvent former ensemble une liaison ou un groupe $C_1$-$C_6$-alkylène ;
soit une amine acylique, reliée par l'atome d'azote, de formule NRaRb dans laquelle Ra et Rb représentent, indépendamment l'un de l'autre, un atome d'hydrogène ou un groupe $C_1$-$C_8$-alkyle, $C_1$-$C_6$-alkyle-C(O)-, HO-C(O)-$C_1$-$C_6$-alkylène ou $C_1$-$C_6$-alkyle-O-C(O)-$C_1$-$C_6$-alkylène, Ra et Rb pouvant être éventuellement substitués par un groupe Rc où Rc représente un hydroxyle ;
à l'état de base ou de sel d'addition à un acide, ainsi qu'à l'état d'hydrate ou de solvat.

2. Composé de formule (I) selon la revendication 1, **caractérisé en ce que**
Z représente
soit une amine cyclique reliée par l'atome d'azote, de formule :

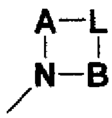

dans laquelle
A représente un groupe $C_1$-$C_7$-alkylène éventuellement substitué par un groupe $R_8$ ;
B représente un groupe $C_1$-$C_7$-alkylène éventuellement substitué par un groupe $R_9$ ;
L représente une liaison ;
les atomes de carbone de l'amine cyclique Z étant éventuellement substitués par un ou plusieurs groupes $R_{12}$ identiques ou différents l'un de l'autre ; $R_{12}$ représente un atome de fluor, un groupe $C_1$-$C_6$-alkyle, $C_1$-$C_6$-alcoxyle, $C_3$-$C_7$-cycloalkyle-$C_1$-$C_6$-alkylène-O-, $CO_2H$, C(O)O-$C_1$-$C_6$-alkyle ou hydroxyle ;
$R_8$ et $R_9$ sont définis tels que :
$R_8$ et $R_9$ peuvent former ensemble une liaison ;
soit une amine acrylique, reliée par l'atome d'azote, de formule NRaRb dans laquelle Ra et Rb représentent, indépendamment l'un de l'autre, un atome d'hydrogène ou un groupe $C_1$-$C_6$-alkyle, $C_1$-$C_6$-alkyle-C(O)-, HO-C(O)-$C_1$-$C_6$-alkylène ou $C_1$-$C_6$-alkyle-O-C(O)-$C_1$-$C_6$-alkylène, Ra et Rb pouvant être éventuellement substitués par un groupe Rc où Rc représente un hydroxyle ;
à l'état de base ou de sel d'addition à un acide, ainsi qu'à l'état d'hydrate ou de solvat.

3. Composé de formule (I) selon la revendication 2, **caractérisé en ce que**
Z représente
soit une amine cyclique reliée par l'atome d'azote et choisie parmi les groupes pyrrolidinyle, azétidinyle, azabicyclo[3.2.0]heptyle ou azabicyclo[3.1.0]hexyle, les atomes de carbone de l'amine cyclique étant éventuellement substitués par un ou plusieurs groupes $R_{12}$ identiques ou différents l'un de l'autre ; $R_{12}$ représente un atome de fluor, un groupe $C_1$-$C_6$-alkyle, $C_1$-$C_6$-alcoxyle, $C_3$-$C_7$-cycloalkyle-$C_1$-$C_6$-alkylène-O-, $CO_2H$, C(O)O$C_1$-$C_6$-alkyle ou hydroxyle ;
soit une amine acylique, reliée par l'atome d'azote, de formule NRaRb dans laquelle Ra et Rb représentent, indépendamment l'un de l'autre, un atome d'hydrogène ou un groupe $C_1$-$C_6$-alkyle, $C_1$-$C_6$-alkyle-C(O)-, HO-C(O)-$C_1$-$C_6$-alkylène ou $C_1$-$C_6$-alkyle-O-C(O)-$C_1$-$C_6$-alkylène, Ra et Rb pouvant être éventuellement substitués par un groupe Rc où Rc représente un hydroxyle ;
à l'état de base ou de sel d'addition à un acide, ainsi qu'à l'état d'hydrate ou de solvat.

4. Procédé de préparation d'un composé de formule (I) selon l'une quelconque des revendications 1 à 3, **caractérisé en ce que** l'on fait réagir un composé de formule générale (IV)

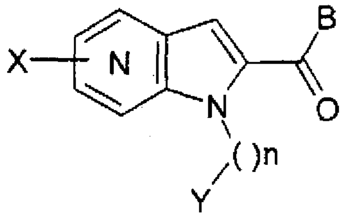

(IV)

dans laquelle X, Y et n sont tels que définis dans la formule générale (I) selon la revendication 1 et B représente un atome de chlore,
avec une amine de formule générale (V)

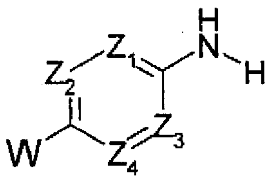

dans laquelle W = Z et Z, $Z_1$, $Z_2$, $Z_3$ et $Z_4$ sont tels que définis dans la formule générale (I) selon la revendication 1, dans un solvant.

5. Procédé de préparation d'un composé de formule (I) selon l'une quelconque des revendications 1 à 3, **caractérisé en ce que** l'on fait réagir un composé de formule générale (IV)

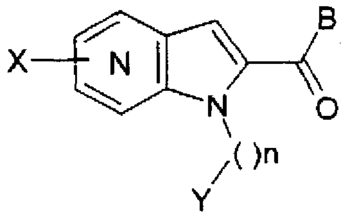

(IV)

dans laquelle X, Y et n sont tels que définis dans la formule générale (I) selon la revendication 1 et B représente un groupe hydroxyle,
avec une amine de formule générale (V)

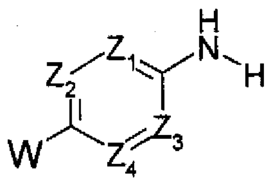

dans laquelle W = Z et Z, $Z_1$, $Z_2$, $Z_3$ et $Z_4$ sont tels que définis dans la formule générale (1) selon la revendication 1, en présence d'un agent de couplage, et éventuellement d'une base, dans un solvant.

6. Procédé de préparation d'un composé de formule (I) selon l'une quelconque des revendications 1 à 3, **caractérisé en ce que** l'on fait réagir un composé de formule générale (VI)

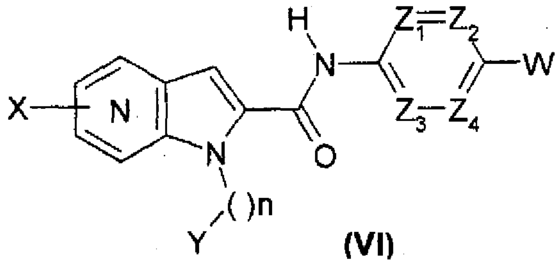

(VI)

dans laquelle X, Y, n, $Z_1$, $Z_2$, $Z_3$ et $Z_4$ sont tels que définis dans la formule générale (I) selon la revendication 1 et W représente un atome d'halogène,
avec amine de formule Z-H, dans laquelle Z est tel que défini dans la formule générale (I).

7. Composés de formules (Va), (Vb), (Vc), (Vd), (Ve), (Vf), (Vg), (Vh) :

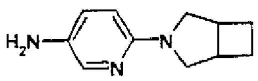

(Va)

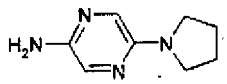

(Vb)

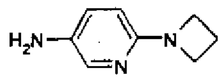

(Vc)

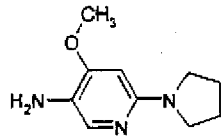

(Vd)

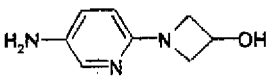

(Ve)

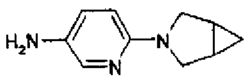

(Vf)

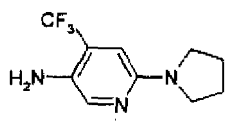

(Vg)

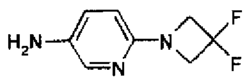

(Vh)

8. Médicament, **caractérisé en ce qu'**il comprend un composé de formule (I), selon l'une quelconque des revendications 1 à 3, ou un sel pharmaceutiquement acceptable, ou encore un hydrate ou un solvat du composé de formule (I).

9. Composition pharmaceutique, **caractérisée en ce qu'**elle comprend un composé de formule (1), selon l'une quelconque des revendications 1 à 3, ou un sel pharmaceutiquement acceptable, un hydrate ou un solvat de ce composé, ainsi qu'au moins un excipient pharmaceutiquement acceptable.

10. Utilisation d'un composé de formule (1), selon l'une quelconque des revendications 1 à 3, pour la préparation d'un médicament destiné à prévenir ou à traiter les pathologies dans lesquelles les récepteurs de type TRPV1 sont impliqués, ces pathologies etant choisies parmi la douleur, l'inflammation, les désordres urologiques, les désordres gynécologiques, les désordres gastrointestinaux, des désordres respiratoires, le psoriasis, le pruritis, les irritations dermiques, des yeux ou des muqueuses, l'herpès, le zona, ou à traiter la dépression ou les désordres métaboliques.

## Patentansprüche

1. Verbindung der Formel (I)

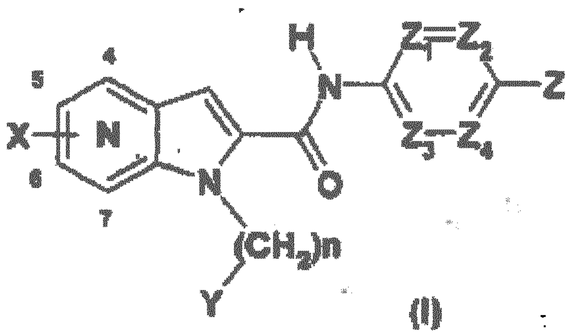

(I)

worin

der Pyrrolopyridinkern eine Pyrrolo[3,2-*b*]pyridingruppe, eine Pyrrolo[2,3-c]pyridingruppe oder eine Pyrrolo[2,3-*b*]pyridingruppe ist, wobei der Pyrrolopyridinkern gegebenenfalls in Position 4, 5, 6 und/oder 7 durch einen oder mehrere Substituenten X substituiert ist,

der oder die Substitutenten X gleich oder voneinander verschieden sind und unter einem Wasserstoff- oder Halogenatom oder einer $C_1$-$C_6$-Fluoralkylgruppe ausgewählt sind;

n gleich 1 ist;

Y für ein Phenyl oder ein Pyridinyl steht, wobei das Phenyl oder Pyridinyl gegebenenfalls durch eine oder mehrere aus einem Halogenatom oder einer $C_1$-$C_6$-Alkyl-, $C_1$-$C_6$-Fluoralkyl-, $C_1$-$C_6$-Alkoxy- oder $C_1$-$C_6$-Fluoralkylgruppe ausgewählte Gruppen substituiert sind;

$Z_1$, $Z_2$, $Z_3$ und $Z_4$ unabhängig voneinander für ein Stickstoffatom oder eine Gruppe $C(R_6)$ stehen, wobei mindestens eine dieser Gruppen einem Stickstoffatom entspricht und mindestens eine dieser Gruppen einer Gruppe $C(R_6)$ entspricht;

$R_6$ für ein Wasserstoff- oder Halogenatom oder eine $C_1$-$C_6$-Alkyl-, $C_1$-$C_6$-Fluoralkyl- oder $C_1$-$C_6$-Alkoxygruppe steht;

Z für

ein über das Stickstoffatom gebundenes cyclisches Amin der Formel:

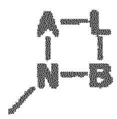

worin

A für eine $C_1$-$C_7$-Alkylengruppe, die gegebenenfalls durch eine oder zwei Gruppen $R_8$ substituiert ist, steht;

B für eine $C_1$-$C_7$-Alkylengruppe, die gegebenenfalls durch eine oder zwei Gruppen $R_9$ substituiert ist, steht;

L für eine Bindung steht;

wobei die Kohlenstoffatome des cyclischen Amins Z gegebenenfalls durch eine oder mehrere gleiche oder voneinander verschiedene Gruppen $R_{12}$ substituiert sind; $R_{12}$ für ein Fluoratom oder eine $C_1$-$C_6$-Alkyl-, $C_1$-$C_6$-Alkoxy-, $C_3$-$C_7$-Cycloalkyl-$C_1$-$C_6$-alkylen-O-, $CO_2H$-, C(O)O-$C_1$-$C_6$-Alkyl- oder Hydroxylgruppe steht;

$R_8$ und $R_9$ so definiert sind, dass:

zwei Gruppen $R_8$ zusammen eine Bindung oder eine $C_1$-$C_6$-Alkylengruppe bilden können;

zwei Gruppen $R_9$ zusammen eine Bindung oder eine $C_1$-$C_6$-Alkylengruppe bilden können;

$R_8$ und $R_9$ zusammen eine Bindung oder eine $C_1$-$C_6$-Alkylengruppe bilden können;

oder ein über das Stickstoffatom gebundenes acyclisches Amin der Formel NRaRb, worin Ra und Rb unabhängig voneinander für ein Wasserstoffatom oder eine $C_1$-$C_6$-Alkyl-, $C_1$-$C_6$-Alkyl-C(O)-, HO-C(O)-$C_1$-$C_6$-Alkylen- oder $C_1$-$C_6$-Alkyl-O-C(O)-$C_1$-$C_6$-alkylengruppe stehen, wobei Ra und Rb gegebenenfalls durch eine Gruppe Rc substituiert sein können, wobei Rc für ein Hydroxyl steht;

steht;

in Basen- oder Säureadditionssalzform sowie in Hydrat- oder Solvatform.

**2.** Verbindung der Formel (I) nach Anspruch 1, **dadurch gekennzeichnet, dass**

Z für

ein über das Stickstoffatom gebundenes cyclisches Amin der Formel:

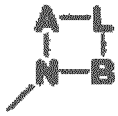

worin
A für eine $C_1$-$C_7$-Alkylengruppe, die gegebenenfalls durch eine Gruppe $R_8$ substituiert ist, steht;
B für eine $C_1$-$C_7$-Alkylengruppe, die gegebenenfalls durch eine Gruppe $R_9$ substituiert ist, steht;
L für eine Bindung steht;
wobei die Kohlenstoffatome des cyclischen Amins Z gegebenenfalls durch eine oder mehrere gleiche oder voreinander verschiedene Gruppen $R_{12}$ substituiert sind; $R_{12}$ für ein Fluoratom oder eine $C_1$-$C_6$-Alkyl-, $C_1$-$C_6$-Alkoxy-, $C_3$-$C_7$-Cycloalkyl-$C_1$-$C_6$-alkylen-O-, $CO_2H$-, C(O)O-$C_1$-$C_6$-Alkyl- oder Hydroxylgruppe steht;
$R_8$ und $R_9$ so definiert sind, dass:
$R_8$ und $R_9$ zusammen eine Bindung bilden können;
oder ein über das Stickstoffatom gebundenes acyclisches Amin der Formel NRaRb, worin Ra und Rb unabhängig voneinander für ein Wasserstoffatom oder eine $C_1$-$C_6$-Alkyl-, $C_1$-$C_6$-Alkyl-C(O)-, HO-C(O)-$C_1$-$C_6$-Alkylen- oder $C_1$-$C_6$-Alkyl-O-C(O)-$C_1$-$C_6$-alkylengruppe stehen, wobei Ra und Rb gegebenenfalls durch eine Gruppe Rc substituiert sein können, wobei Rc für ein Hydroxyl steht;
steht;
in Basen- oder Säureadditionssalzform sowie in Hydrat- oder Solvatform.

3. Verbindung der Formel (I) nach Anspruch 2, **dadurch gekennzeichnet, dass**
Z für
ein über das Stickstoffatom gebundenes cyclisches Amin, das aus Pyrrolidinyl-, Azetidinyl-, Azabicyclo[3.2.0]heptyl- oder Azabicyclo[3.1.0]-hexylgruppen ausgewählt ist, wobei die Kohlenstoffatome des cyclischen Amins gegebenenfalls durch eine oder mehrere gleiche oder voneinander verschiedene Gruppen $R_{12}$ substituiert sind; $R_{12}$ für ein Fluoratom oder eine $C_1$-$C_6$-Alkyl-, $C_1$-$C_6$-Alkoxy-, $C_3$-$C_7$-Cycloalkyl-$C_1$-$C_6$-alkylen-O-, $CO_2H$-, C(O)O-$C_1$-$C_6$-Alkyl- oder Hydroxylgruppe steht;
oder ein über das Stickstoffatom gebundenes acyclisches Amin der Formel NRaRb, worin Ra und Rb unabhängig voneinander für ein Wasserstoffatom oder eine $C_1$-$C_6$-Alkyl-, $C_1$-$C_6$-Alkyl-C(O)-, HO-C(O)-$C_1$-$C_6$-Alkylen- oder $C_1$-$C_6$-Alkyl-O-C(O)-$C_1$-$C_6$-alkylengruppe stehen, wobei Ra und Rb gegebenenfalls durch eine Gruppe Rc substituiert sein können, wobei Rc für ein Hydroxyl steht;
steht;
in Basen- oder Säureadditionssalzform sowie in Hydrat- oder Solvatform.

4. Verfahren zur Herstellung einer Verbindung der Formel (I) gemäß einem der Ansprüche 1 bis 3, **dadurch gekennzeichnet, dass** man eine Verbindung der allgemeinen Formel (IV)

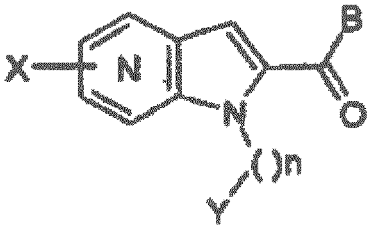

(IV)

worin X, Y und n wie in der allgemeinen Formel (I) gemäß Anspruch 1 definiert sind und B für ein Chloratom steht, in einem Lösungsmittel mit einem Amin der allgemeinen Formel (V)

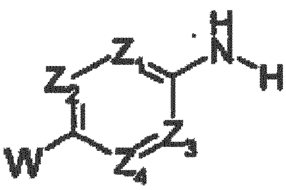

worin W = Z und Z, $Z_1$, $Z_2$, $Z_3$ und $Z_4$ wie in der allgemeinen Formel (I) gemäß Anspruch 1 definiert sind, umsetzt.

**5.** Verfahren zur Herstellung einer Verbindung der Formel (I) gemäß einem der Ansprüche 1 bis 3, **dadurch gekennzeichnet, dass** man eine Verbidung der allgemeinen Formel (IV)

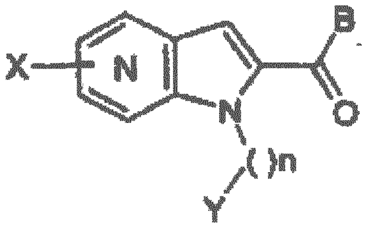

(IV)

worin X, Y und n wie in der allgemeinen Formel (I) gemäß Anspruch 1 definiert sind und B für eine Hydroxylgruppe steht,
in Gegenwart eines Kupplungsmittels und gegebenenfalls einer Base in einem Lösungsmittel mit einem Amin der allgemeinen Formel (V)

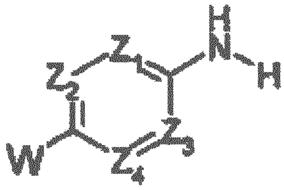

worin W = Z und Z, $Z_1$, $Z_2$, $Z_3$ und $Z_4$ wie in der allgemeinen Formel (I) gemäß Anspruch 1 definiert sind, umsetzt.

**6.** Verfahren zur Herstellung einher Verbindung der Formel (I) gemäß einen der Ansprüche 1 bis 3, **dadurch gekennzeichnet, dass** man eine Verbindung der allgemeinen Formel (VI)

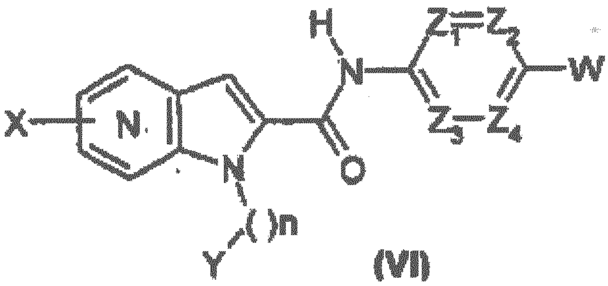

(VI)

worin X, Y, n, $Z_1$, $Z_2$, $Z_3$ und $Z_4$ wie in der allgemeinen Formel (I) gemäß Anspruch 1 definiert sind und W für ein Halogenatom steht,
mit einem Amin der allgemeinen Formel Z-H, worin Z wie in der allgemeinen Formel (I) definiert ist, umsetzt.

**7.** Verbindungen der Formeln (Va), (Vb), (Vc), (Vd), (Ve), (Vf), (Vg), (Vh):

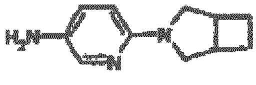

(Va)

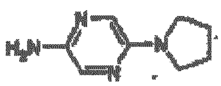

(Vb)

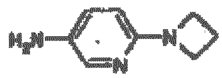

(Vc)

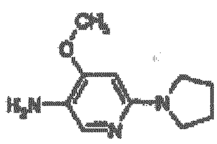

(Vd)

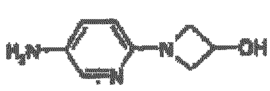
(Ve)

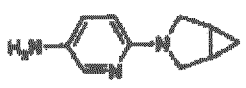
(Vf)

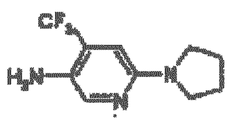
(Vg)

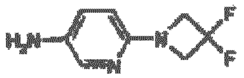
(Vh)

8. Arzneimittel, **dadurch gekennzeichnet, dass** es eine Verbindung der Formel (I) gemäß einem der Ansprüche 1 bis 3 oder ein pharmazeutisch unbedenkliches Salz oder auch ein Hydrat oder ein Solvat der Verbindung der Formel (I) umfasst.

9. Pharmazeutische Zusammensetzung, **dadurch gekennzeichnet, dass** sie eine Verbindung der Formel (I) gemäß einem der Ansprüche 1 bis 3 oder ein pharmazeutisch unbedenkliches Salz, ein Hydrat oder ein Solvat dieser Verbindung sowie mindestens einen pharmazeutisch unbedenklichen Hilfsstoff umfasst.

10. Verwendung einer Verbindung der Formel (I) gemäß einem der Ansprüche 1 bis 3 zur Herstellung eines Arzneimittels zur Prävention oder Behandlung von Pathologien, an denen Rezepturen vom TRPV1-Typ beteiligt sind, wobei diese Pathologien aus Schmerzen, Entzündungen, urologischen Störungen, gynäkologischen Störungen, gastrointestinalen Störungen, respiratorischen Störungen, Psoriasis, Pruritis, Haut-, Augen- oder Schleimhautreizungen, Herpes, Zona, ausgewählt sind, oder zur Behandlung von Depression oder Stoffwechselstörungen.

## Claims

1. Compound corresponding to formula (I)

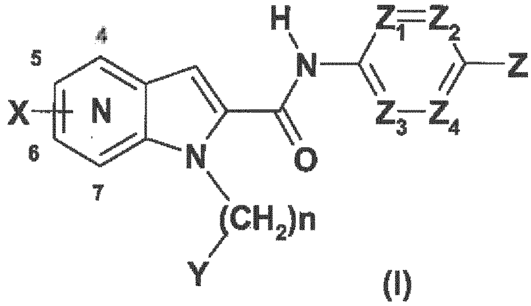
(I)

in which
the pyrrolopyridine nucleus is a pyrrolo[3,2-*b*]pyridine group, a pyrrolo[2,3-*c*]pyridine group or a pyrrolo[2,3-*b*]pyridine group; the pyridine nucleus being optionally substituted in position 4, 5, 6 and/or 7 with one or more substituents X, the substituent or substituents X, which may be identical to or different from one another, are selected from a hydrogen or halogen atom or a $C_1$-$C_6$-fluoroalkyl group;
n is equal to 1;
Y represents a phenyl or a pyridinyl, the phenyl or the pyridinyl being optionally substituted with one or more groups selected from a halogen atom or a $C_1$-$C_6$-alkyl, $C_1$-$C_6$-fluoroalkyl, $C_1$-$C_6$-alkoxyl or $C_1$-$C_6$-fluoroalkoxyl group;
$Z_1$, $Z_2$ $2_3$, $Z_4$ represent, independently of one another, a nitrogen atom or a group $C(R_6)$, at least one corresponding to a nitrogen atom and at least one corresponding to a group $C(R_6)$;
$R_6$ represents a hydrogen or halogen atom or a $C_1$-$C_6$-alkyl, $C_1$-$C_6$-fluoroalkyl or $C_1$-$C_6$-alkoxyl group;
Z represents
either a cyclic amine attached by the nitrogen atom, of formula:

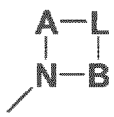

in which

A represents a $C_1$-$C_7$-alkylene group optionally substituted with one or two groups $R_8$;
B represents a $C_1$-$C_7$-alkylene group optionally substituted with one or two groups $R_9$;
L represents a bond;
the carbon atoms of the cyclic amine z being optionally substituted with one or more groups $R_{12}$ which may be identical to or different from one another; $R_{12}$ represents a fluorine atom, a $C_1$-$C_6$-alkyl, $C_1$-$C_6$-alkoxyl, $C_3$-$C_7$-cycloalkyl-$C_1$-$C_6$-alkylene-O-, $CO_2H$, C (O)O-$C_1$-$C_6$-alkyl or hydroxyl group;
$R_8$ and $R_9$ are defined such that:
two groups $R_8$ can together form a bond or a $C_1$-$C_6$-alkylene group;
two groups $R_9$ can together form a bond or a $C_1$-$C_6$-alkylene group;
$R_8$ and $R_9$ can together form a bond or a $C_1$-$C_6$-alkylene group;
or an acyl amine, attached by the nitrogen atom, of formula NRaRb in which Ra and Rb represent, independently of one another, a hydrogen atom or a $C_1$-$C_6$-alkyl, $C_1$-$C_6$-alkyl-C(O)-, HO-C(O)-$C_1$-$C_6$-alkylene or $C_1$-$C_6$-alkyl-O-C (O)-$C_1$-$C_6$-alkylene, Ra and Rb being optionally substituted with a group Rc where Rc represents a hydroxyl;
as a base or an addition salt to an acid, as well as in the form of hydrate or solvate.

**2.** Compound of formula (I) according to Claim 1, **characterized in that**
Z represents
either a cyclic amine attached by the nitrogen atom, of formula:

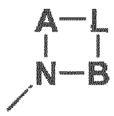

in which
A represents a $C_1$-$C_7$-alkylene group optionally substituted with a group $R_8$;
B represents a $C_1$-$C_7$-alkylene group optionally substituted with a group $R_9$;
L represents a bond;
the carbon atoms of the cyclic amine Z being optionally substituted with one or more groups $R_{12}$ which may be identical to or different from one another; $R_{12}$ represents a fluorine atom, a $C_1$-$C_6$-alkyl, $C_1$-$C_6$-alkoxyl, $C_3$-$C_7$-cycloalkyl-$C_1$-$C_6$-alkylene-O-, $CO_2H$, C(O)O-$C_1$-$C_6$-alkyl or hydroxyl group;
$R_8$ and $R_9$ are defined such that:
$R_8$ and $R_9$ can together form a bond;
or an acyl amine, attached by the nitrogen atom, of formula NRaRb in which Ra and Rb represent, independently of one another, a hydrogen atom or a $C_1$-$C_6$-alkyl, $C_1$-$C_6$-alkyl-C(O) -, HO-C(O)-$C_1$-$C_6$-alkylene or $C_1$-$C_6$-alkyl-O-C(O)-$C_1$-$C_6$-alkylene group, and Ra and Rb can optionally be substituted with a group Rc where Rc represents a hydroxyl;
as a base or an addition salt to an acid, as well as in the form of hydrate or solvate.

**3.** Compound of formula (I) according to Claim 2, **characterized in that**
Z represents
either a cyclic amine attached by the nitrogen atom and selected from the pyrrolidinyl, azetidinyl, azabicyclo[3.2.0]heptyl or azabicyclo[3.1.0]hexyl groups, the carbon atoms of the cyclic amine being optionally substituted with one or more groups $R_{12}$ which may be identical to or different from one another; $R_{12}$ represents a fluorine atom, a $C_1$-$C_6$-alkyl, $C_1$-$C_6$-alkoxyl, $C_3$-$C_7$-cycloalkyl-$C_1$-$C_6$-alkylene-O-, $CO_2H$, C(O)O-$C_1$-$C_6$-alkyl or hydroxyl group;
or an acyl amine, attached by the nitrogen atom, of formula NRaRb in which Ra and Rb represent, independently of one another, a hydrogen atom or a $C_1$-$C_6$-alkyl, $C_1$-$C_6$-alkyl-C(O)-, HO-C(O)-$C_1$-$C_6$-alkylene or $C_1$-$C_6$-alkyl-O-C (O)-$C_1$-$C_6$-alkylene group, Ra and Rb being optionally substituted with a group Rc where Rc represents a hydroxyl;
as a base or an addition salt to an acid, as well as in the form of hydrate or solvate.

**4.** Method of preparation of a compound of formula (I) according to any one of Claims 1 to 3, **characterized in that** a compound of general formula (IV)

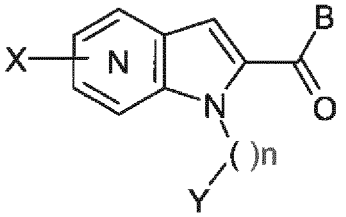

(IV)

in whiCh X, Y and n are as defined in general formula (I) according to Claim 1 and B represents a chlorine atom, is reacted with an amine of general formula (V)

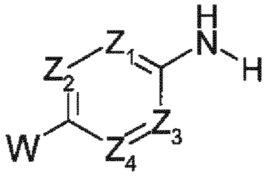

in which W = Z and Z, $Z_1$, $Z_2$, $Z_3$ and $Z_4$ are as defined in general formula (I) according to Claim 1, in a solvent.

**5.** Method of preparation of a compound of formula (I) according to any one of Claims 1 to 3, **characterized in that** a compound of general formula (IV)

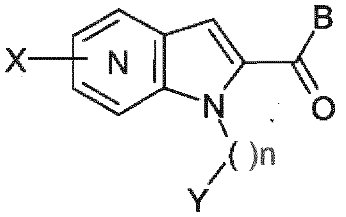

(IV)

in which X, Y and n are as defined in general formula (I) according to Claim 1 and B represents a hydroxyl group, is reacted with an amine of general formula (V)

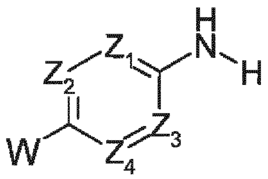

in which W = Z and Z, $Z_1$, $Z_2$, $Z_3$ and $Z_4$ are as defined in general formula (I) according to Claim 1, in the presence of a coupling agent, and optionally of a base, in a solvent.

**6.** Method of preparation of a compound of formula (I) according to any one of Claims 1 to 3, **characterized in that** a compound of general formula (VI)

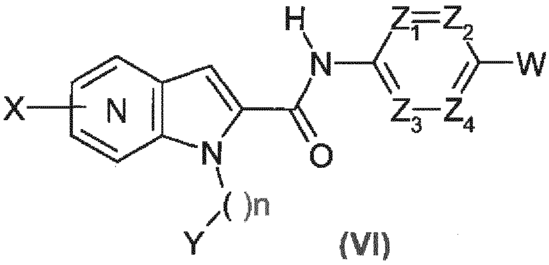

(VI)

in which X, Y, n, $Z_1$, $Z_2$, $Z_3$ and $Z_4$ are as defined in general formula (I) according to Claim 1 and W represents a halogen atom,
is reacted with an amine of formula Z-H, in which Z is as defined in general formula (I).

**7.** Compounds of formulae (Va), (Vb), (Vc), (Vd), (Ve), (Vf), (Vg), (Vh):

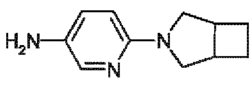
(Va)

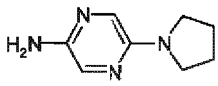
(Vb)

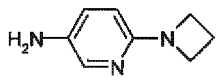
(Vc)

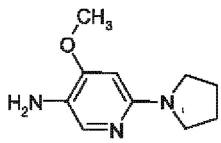
(Vd)

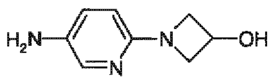
(Ve)

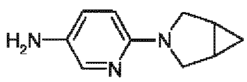
(Vf)

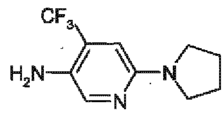
(Vg)

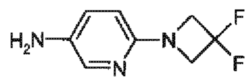
(Vh)

**8.** Medicinal product, **characterized in that** it comprises a compound of formula (I), according to any one of Claims 1 to 3, or a pharmaceutically acceptable salt, or alternatively a hydrate or a solvate of the compound of formula (I).

**9.** Pharmaceutical composition, **characterized in that** it comprises a compound of formula (I), according to any one of Claims 1 to 3, or a pharmaceutically acceptable salt, a hydrate or a solvate of said compound, as well as at least one pharmaceutically acceptable excipients

**10.** Use of a compound of formula (I) according to any one of Claims 1 to 3, for the preparation of a medicinal product intended for the prevention or treatment of pathologies that involve the type TRPV1 receptors, these pathologies being chosen from pain, inflammation, urological disorders, gynaecological disorders, gastrointestinal disorders, respiratory disorders, psoriasis, pruritus, irritations of the skin, eyes or mucosae, herpes simplex, herpes zoster, or for treating depression or metabolic disorders.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 200502845 A
- WO 2002048152 A
- WO 2006040522 A
- WO 2004052869 A
- WO 2004062665 A
- WO 2005035526 A
- WO 2004110986 A
- WO 200248152 A
- US 1977805419 B
- WO 05035526 A
- WO 071010138 A
- JP 7051121 A
- WO 2003080610 A
- WO 07010138 A


### Littérature non-brevet citée dans la description


- **J. MARCH.** Advances in Organic Chemistry. Wiley Interscience, 2001
- **P. ROY et al.** *Synthesis,* 2005, vol. 16, 2751-2757
- **N. LAHANCE et al.** *Synthesis,* 2005, vol. 15, 2571-2577
- **C. BARBERIS et al.** *Tetrahedron Left,* 2005, vol. 46 (51), 8877-8880
- **T. LOMBERGET.** *Synlett,* 2005, vol. 13, 2080-2082
- **M. NAZARE et al.** *Angew Chem Int Ed,* 2004, vol. 43 (34), 4526-4528
- **P.M. FRESNEDA et al.** *Tetrahedron Lett,* 2000, vol. 41 (24), 4777-4780
- **KOLASA T.** *Bioorg.Med.Chem.,* 1997, vol. 5 (3), 507
- **ABRAMOVITCH R.** *Synth. Commun.,* 1995, vol. 25 (1), 1
- **O. MITSONOBU.** *Synthesis,* 1981, 1-28
- **S. L. BUCHWALD et al.** *J. Am. Chem. Soc.,* 2001, vol. 123, 7727
- *J. AM. CHEM. SOC.,* 2002, vol. 124, 11684
- **HARTWIG et al.** *Angewandte Chemie,* 2005, vol. 44, 1371-1375
- *Pharmazie,* 1990, vol. 45, 346
- **GREENE, WUTS.** Protective groups in organic synthesis. Wiley-Interscience
- **GREENE, WUTS.** Protective groups in organic synthesis. iley-Interscience
- **CARLING R.W. et al.** *J.Med.Chem.,* 2004, 1807-1822
- **RUSSEL M.G.N. et al.** *J.Med.Chem.,* 2005, 1367-1383
- *Heterocycles,* 1977, vol. 6 (12), 1999-2004
- *Angew Chem Int Ed,* 2004, vol. 43 (34), 4526-4528
- *J.Med.Chem.,* 1994, 18-25
- *J.Med.Chem.,* 1967, vol. 10 (4), 621